# EUROPEAN PATENT SPECIFICATION

(11) **EP 3 342 868 B1**
(45) Date of publication and mention of the grant of the patent: **25.12.2019**
(21) Application number: 17209751.1
(22) Date of filing: 21.12.2017
(51) Int. Cl.: C12N 15/63, C12N 15/10

(54) **CONSTRUCTS AND SCREENING METHODS**
KONSTRUKTE UND SCREENING-VERFAHREN
CONSTRUCTIONS ET PROCÉDÉS DE CRIBLAGE

(30) Priority: 30.12.2016 WO PCT/EP2016/082936
(43) Date of publication of application: 04.07.2018
(73) Proprietor: Systasy Bioscience GmbH, 80797 München (DE)
(72) Inventor: Herholt, Alexander, 80801 München (DE); Rossner, Moritz, 68723 Schwetzingen (DE)
(74) Representative: Graf von Stosch Patentanwaltsgesellschaft mbH

(56) References cited:
- WO-A1-2010/034773
- US-A1- 2009 217 404
- DAVID SIMS ET AL: "High-throughput RNA interference screening using pooled shRNA libraries and next generation sequencing", GENOME BIOLOGY, BIOMED CENTRAL LTD., LONDON, GB, vol. 12, no. 10, 21 October 2011 (2011-10-21), page R104, XP021112698, ISSN: 1465-6906, DOI: 10.1186/GB-2011-12-10-R104
- NGO ET AL: "A Loss-Of-Function RNA Interference Screen For Molecular Targets In Cancer", NATURE, MACMILLAN JOURNALS LTD., ETC, GB , vol. 441, no. 7089 4 May 2006 (2006-05-04), pages 106-110, XP009500539, ISSN: 0028-0836, DOI: 10.1038/NATURE04687 Retrieved from the Internet: URL:http://epo.summon.serialssolutions.com /2.0.0/link/0/eLvHCXMwjV1LT8MwDI4ACYkLsIFg G6AcdoBDxFrn0R2riQ0htMM0DpyqNmm5wIba7cC_J2 4y9hBC3CLVsqw4duza_kJIN5eFNJIbBogzxHVhWF8Z YKkWkEdKSVOjfb4O-HSkJiP-vG6i2a3gQ3TvAC5tGh fhzDgAoPlNxtN1I0eAEYwvHHDeU5vAy34kb4fR1iXk XfE2kuYvkWZ94wxP_inrKTn2ISWN3RlokL181iSH7p HJL7uqmzx1
- KIMBERLY BROWN DAHLMAN ET AL: "Modulators of Prostate Cancer Cell Proliferation and Viability Identified by Short-Hairpin RNA Library Screening", PLOS ONE, vol. 7, no. 4, 11 April 2012 (2012-04-11), page e34414, XP055458958, DOI: 10.1371/journal.pone.0034414
- JASPER MULLENDERS ET AL: "A Large Scale shRNA Barcode Screen Identifies the Circadian Clock Component ARNTL as Putative Regulator of the p53 Tumor Suppressor Pathway", PLOS ONE, vol. 4, no. 3, 11 March 2009 (2009-03-11), page e4798, XP055458970, DOI: 10.1371/journal.pone.0004798
- PARNAS OREN ET AL: "A Genome-wide CRISPR Screen in Primary Immune Cells to Dissect Regulatory Networks", CELL, CELL PRESS, vol. 162, no. 3, 16 July 2015 (2015-07-16) , pages 675-686, XP029248090, ISSN: 0092-8674, DOI: 10.1016/J.CELL.2015.06.059
- Alexander Herholt: "Development of a multiplexed rnai-coupled sensor assay to study neuronal function on the large-scale", , 1 January 2017 (2017-01-01), XP055461545, Retrieved from the Internet: URL:http://ediss.uni-goettingen.de/bitstre am/handle/11858/00-1735-0000-0023-3EB1-0/P hD%20Thesis%20-%20Alexander%20Herholt%20-% 20ePub_SUB.pdf?sequence=1

### SEQUENCE LISTING

<110> Systasy Bioscience GmbH, Munich, Germany
<120> NOVEL CONSTRUCTS AND SCREENING METHODS
<130> SB07P001EP
<160> 37
<170> PatentIn version 3.5
<210> 1
   <211> 13
   <212> DNA
   <213> artificial sequence
<220>
   <223> UIS
<400> 1
   taggtgacac tat 13
<210> 2
   <211> 15
   <212> DNA
   <213> artificial
<220>
   <223> UIS
<400> 2
   cctatagtga gtcgt 15
<210> 3
   <211> 20
   <212> DNA
   <213> artificial
<220>
   <223> OligodT
<400> 3
   tttttttttt tttttttttt 20
<210> 4
   <211> 19
   <212> DNA
   <213> artificial
<220>
   <223> Tcf4 fwd (Mm)
<400> 4
   ctggagcagc aagttcgag 19
<210> 5
   <211> 22
   <212> DNA
   <213> artificial
<220>
   <223> Tcf4 rev (Mm)
<400> 5
   ttctcttcct cccttctttt ca 22
<210> 6
   <211> 18
   <212> DNA
   <213> artificial
<220>
   <223> Arc fwd (Mm)
<400> 6
   aggggctgag tcctcaca 18
<210> 7
   <211> 22
   <212> DNA
   <213> artificial
<220>
   <223> Arc rev (Mm)
<400> 7
   gacttctcag cagccttgag ac 22
<210> 8
   <211> 20
   <212> DNA
   <213> artificial
<220>
   <223> Rpl13a fwd (Mm)
<400> 8
   atccctccac cctatgacaa 20
<210> 9
   <211> 19
   <212> DNA
   <213> artificial
<220>
   <223> Rpl13a rev (Mm)
<400> 9
   gccccaggta agcaaactt 19
<210> 10
   <211> 20
   <212> DNA
   <213> artificial
<220>
   <223> WPRE fwd
<400> 10
   actgtgtttg ctgacgcaac 20
<210> 11
   <211> 18
   <212> DNA
   <213> artificial
<220>
   <223> WPRE rev
<400> 11
   agtcccggaa aggagctg 18
<210> 12
   <211> 28
   <212> DNA
   <213> artificial
<220>
   <223> hU6p fwd
<400> 12
   tttcaagtta cggtaagcat atgatagt 28
<210> 13
   <211> 27
   <212> DNA
   <213> artificial
<220>
   <223> hU6p rev
<400> 13
   caaggctgtt agagagataa ttggaat 27
<210> 14
   <211> 25
   <212> DNA
   <213> artificial
<220>
   <223> qDecl.2 fwd
<400> 14
   ccgagtagaa ttaaccctca ctaaa 25
<210> 15
   <211> 22
   <212> DNA
   <213> artificial
<220>
   <223> qDec2.2. rev
<400> 15
   cgcgtctact aatacgactc ac 22
<210> 16
   <211> 26
   <212> DNA
   <213> artificial
<220>
   <223> Dec fwd
<400> 16
   agctagttgc taagtctgcc gagtag 26
<210> 17
   <211> 26
   <212> DNA
   <213> artificial
<220>
   <223> Dec rev
<400> 17
   tcgtacatgc attgactcgc gtctac 26
<210> 18
   <211> 66
   <212> DNA
   <213> artificial
<220>
   <223> PGM_A_IXcodeX_AFA_s
<220>
   <221> misc_feature
   <222> (31)..(42)
   <223> n is a, c, g, or t
<400> 18
   ccatctcatc cctgcgtgtc tccgactcag nnnnnnnnnn nntcctcact aaagggtagg 60
   tgacac 66
<210> 19
   <211> 26
   <212> DNA
   <213> artificial
<220>
   <223> hU6 fwd
<400> 19
   tctcagagag agacagagac agatcc 26
<210> 20
   <211> 26
   <212> DNA
   <213> artificial
<220>
   <223> Dec rev
<400> 20
   gtagacgcga gtcaatgcat gtacga 26
<210> 21
   <211> 31
   <212> DNA
   <213> artificial
<220>
   <223> BC35 rev
<400> 21
   gagacttaag atatcggatc cagctagttg c 31
<210> 22
   <211> 112
   <212> DNA
   <213> artificial
<220>
   <223> SpA
<400> 22
   gtagacgcga gtcaatgcat gtacgatcta gacaataaaa gatctttatt ttcattagat 60
   ctgtgtgttg gttttttgtg tgactagtct cgtatgccgt cttttgcttg cc 112
<210> 23
   <211> 163
   <212> DNA
   <213> artificial
<220>
   <223> BC35
<220>
   <221> misc_feature
   <222> (93)..(94)
   <223> n is a, c, g, or t
<400> 23
   cttaagatat cggatccagc tagttgctaa gtctgccgag tagaattaac cctcactaaa 60
   gggtaggtga cactathhhg hhhghhhghh hgnntvvvtv vvtvvvtvvv tcctatagtg 120
   agtcgtatta gtagacgcga gtcaatgcat gtacgatcta gac 163
<210> 24
   <211> 45
   <212> DNA
   <213> artificial
<220>
   <223> PGM_trP1_hU6_as
<400> 24
   cctctctatg ggcagtcggt gatcttgtgg aaaggacgaa acacc 45
<210> 25
   <211> 22
   <212> DNA
   <213> artificial
<220>
   <223> Tcf4 (target sequence)
<400> 25
   ttctaattac cggatattga at 22
<210> 26
   <211> 67
   <212> DNA
   <213> artificial
<220>
   <223> Adcy3
<400> 26
   ccgggccatc tttctcaggt tatttgttaa tattcatagc aaatgacctg ggaaagatgg 60
   ctttttt                                                               67
<210> 27
   <211> 67
   <212> DNA
   <213> artificial
<220>
   <223> Il2rb
<400> 27
   ccggccctcc aaacttaatt atccagttaa tattcatagc tggatgatta agtttggagg 60
   gtttttt 67
<210> 28
   <211> 67
   <212> DNA
   <213> artificial
<220>
   <223> Camk2d
<400> 28
   ccgggacggg atgttctatg caaatgttaa tattcatagc gtttgcatgg aacatcccgt 60
   ctttttt                                                               67
<210> 29
   <211> 67
   <212> DNA
   <213> artificial
<220>
   <223> Cacnalf
<400> 29
   ccggccctca tctactgctt aataagttaa tattcatagc ttattgagca gtagatgagg 60
   gtttttt 67
<210> 30
   <211> 67
   <212> DNA
   <213> artificial
<220>
   <223> Bhlhe40
<400> 30
   ccgggtcagc acaattaagt aagaagttaa tattcatagc ttcttgctta attgtgctga 60
   ctttttt 67
<210> 31
   <211> 67
   <212> DNA
   <213> artificial
<220>
   <223> Calm1
<400> 31
   ccgggccgct atacttcttt attatgttaa tattcatagc ataataaaga agtatagcgg 60
   ctttttt 67
<210> 32
   <211> 67
   <212> DNA
   <213> artificial
<220>
   <223> Tacr2
<400> 32
   ccggccacag gcaatgttga tataagttaa tattcatagc ttgtatcagc attgcctgtg 60
   gtttttt 67
<210> 33
   <211> 67
   <212> DNA
   <213> artificial
<220>
   <223> Gphn
<400> 33
   ccgggcatac aagatagtac tagatgttaa tattcatagc atctggtact atcttgtatg 60
   ctttttt                                                                  67
<210> 34
   <211> 67
   <212> DNA
   <213> artificial
<220>
   <223> Disc1
<400> 34
   ccgggactgg cttatttgag agaaagttaa tattcatagc tttctctcga ataagccagt 60
   ctttttt                                                                  67
<210> 35
   <211> 67
   <212> DNA
   <213> artificial
<220>
   <223> Cacnalh
<400> 35
   ccgggctaga atgtagtgag gataagttaa tattcatagc ttatcctcgc tgcattctag 60
   ctttttt                                                                  67
<210> 36
   <211> 20
   <212> DNA
   <213> artificial
<220>
   <223> SV40-promoter
<400> 36
   gaatagctca gaggccgagg 20
<210> 37
   <211> 20
   <212> DNA
   <213> artificial
<220>
   <223> Arc-promoter
<400> 37
   cctactcgct cccctcccgt 20

## Description

The present invention generally relates the field of molecular biology and more specifically to the field of functional genomics. Functional genomics is a research field that refers to the impact of the genotype on molecular or cellular phenotypes at genome-scale. Such an approach involves for example the study of dynamic changes in the transcriptome, the proteome, and the epigenome as well as large-scale loss- and gain-of-function studies. Recent advances in genome sequencing techniques have led to the generation of a wealth of data linking genes to certain cellular states, responses or diseases. The exact function of genes now known to be associated with certain conditions however often remains obscure -although such information is essential for unravelling complicated cellular signaling networks and specifically targeting their key players as potential drug targets.

Understanding gene function is thus one major quest in molecular biology. Early after the discovery of the post-transcriptional mRNA abundance control mechanism called RNAi in the nematode *Caenorhabditis elegans* in 1998 and later in mammals, RNAi became the prime method for loss-of-function studies (Jinek M, Doudna JA. Nature. 2009 Jan 22;457(7228):405-12 and Fire A et al. Nature. 1998 Feb 19;391(6669):806-11). The use of RNAi for loss-of-function studies is easy and fast. Usually, short double-stranded RNA molecules are transfected into cells where they are processed into small interfering RNAs (siRNAs) of about 22 nucleotides in length. These siRNAs silence gene expression in a sequence specific manner. A second strategy is based on the expression of shRNAs, an endogenous-like early intermediate RNA of the RNAi pathway. shRNAs can be expressed from RNA polymerase II promoters or RNA polymerase III promoters (e.g. human U6 promoter) (Li L et al. RNA. 2007 Oct;13(10):1765-74 and Mohr SE et al. Nat Rev Mol Cell Biol. 2014 Sep;15(9):591-600), which allows viral delivery and stable shRNA expression over long cultivation periods. In 2011, the groundbreaking publication by Charpentier and colleagues described how the prokaryote *Streptococcus pyogenes* uses clustered regularly interspaced short palindromic repeats (CRISPR) as a defense mechanism against invading prophage DNA (Deltcheva E et al. Nature. 2011 Mar 31;471(7340):602-7). That mechanism has evolved into a new tool for genome editing and gene expression regulation (Jinek M et al. Elife. 2013 Jan 29;2).

The discovery of RNAi and the CRISPR/Cas9 system (as well as other powerful tools) paved the way for modifying gene expression or function in a highly specific manner. Pooled genetic screens employing viral libraries in principle allow high throughput assays for loss-of-function studies at genome-wide scale. However, due to the lack of a specific readout, the observed endpoints in most pooled genetic screens -even using the most elaborate genetic manipulation approaches- are still limited to observing cellular proliferation and viability. FACS-based genetic interference theoretically opens up the possibility to study phenotypes other than cell viability or proliferation. Therefore, the cell pool is treated with a stimulus and the cellular response is detected by an endogenous marker or a fluorescent reporter. Currently the most comprehensive FACS-based screen has been published by Parnas and colleagues (Parnas O et al. Cell. 2015 Jul 30;162(3):675-86) who used a genome-wide CRISPR-Cas9 knockout library in order to study the response of primary mouse dendritic cells to bacterial LPS. Using flow cytometry, the cell pool was sorted based on Tnf expression, which is induced downstream of the LPS/Tlr4 pathway. Parnas et al. then recovered cells that failed to fully induce Tnf or that exhibited an increased Tnf expression and determined sgRNA abundance by deep sequencing. Thereby, regulators of the Tlr4-to-Tnf pathway were confirmed and identified. This approach has several disadvantages. Here, the sgRNAs themselves are used as a reporter, making physical segregation of the cells according to their phenotype indispensable. The additional steps of staining and sorting the cells by flow cytometry renders the method susceptible to errors. The approach is moreover not applicable to many post-mitotic cell types (such as neurons) that would be of interest for identifying novel drug targets. In addition, the FACS-based approach does not allow for a truly quantitative readout but provides rather a semi-quantitative approach.

There is still a need in the art for novel tools and techniques which allow for improved pooled genetic screenings. Particularly, there is a need for novel approaches which obviate the need for an additional step of cell sorting based on the induced phenotype and which provide a quantitative readout. It is the object of the present invention to comply with this need. Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 10%.

The present inventors have developed novel tools and techniques that improve high throughput genetic screenings in a remarkable manner. Such pooled genetic screenings are particularly useful for investigating gene function and identifying novel drug targets. Specifically, the present invention features polynucleotides which are intelligently designed so as to allow for genetic screens with a specific and quantitative readout that obviates the need for cell sorting based on the induced phenotype. In contrast to other genetic screening methods that have been described in the art, the polynucleotides provided herein feature a unique "2 in 1" design coupling an expression cassette encoding a gene targeting effector with a sensor controlling the transcription of a unique barcode sequence. These unique barcode sequences allow for (1) a quantitative readout of the sensor response in the presence of the employed effector and (2) immediate identification of the effector and, thus, the targeted gene which obviates the need for sorting out of single cells exhibiting a desired phenotype. Advantageously, the readout can be accomplished in one single step: namely, by sequencing the barcodes which are expressed as RNA oligonucleotides. The inventive approach is thus applicable to a variety of cell types (including post-mitotic, primary cells with complex morphology). Moreover, the inventive approach is remarkably versatile as it allows for the implementation of any sensor of interest. It may thus aid in elucidating the effects of a multitude of genes on an endpoint of choice which is not restricted to cell proliferation or survival.

In a first aspect, the present invention provides a polynucleotide comprising (a) a sensor expression cassette, said sensor expression cassette comprising a sensor transcription control element operably linked to a unique identifier sequence encoding an RNA oligonucleotide; and (b) an effector expression cassette comprising a sequence encoding an effector expression product; wherein said unique identifier sequence specifically identifies said polynucleotide. The terms "polynucleotide", "nucleic acid", "nucleic acid molecule", "polynucleotide molecule", "nucleic acid fragment", "polynucleotide fragment", "nucleic acid sequence or segment" or polynucleotide sequence or segment" are used interchangeably herein to refer to deoxyribonucleotides or ribonucleotides and polymers thereof in either single- or double-stranded form, composed of monomers (nucleotides) containing a sugar, phosphate and a nitrogenous base that is either a purine or pyrimidine. A nucleoside comprises a nitrogenous base linked to a sugar molecule. The term "polynucleotide" thus generally includes without limitation probes, oligonucleotides, constructs, genomic DNA, antisense DNA, antisense RNA, cDNA, PCR products, restriction fragments, messenger RNA (mRNA), transfer-messenger-RNA (tmRNA), transfer RNA (tRNA), micro RNA (miRNA), ribosomal RNA (rRNA), small interfering RNA (siRNA), small hairpin RNA (shRNA), PNA, single-stranded RNA (ssRNA), double-stranded RNA (dsRNA), single-stranded DNA (ssDNA), double-stranded DNA (dsDNA), DNA:RNA hybrid molecules, ribozymes, aptamers, mini-genes, gene fragments and combinations thereof; all of the aforementioned with or without regulatory elements, untranslated regions or combinations thereof. As is well-known, deoxyribonucleic acid (DNA) in the majority of organisms is the genetic material while ribonucleic acid (RNA) is typically involved in the transfer of information contained within DNA into proteins. In the context of the present invention, polynucleotides preferably comprise single stranded, double stranded or partially double stranded nucleic acids which may be DNA or RNA. In particular, polynucleotides according to the invention are envisaged to be DNA, cDNA and RNA (in particular mRNA) or combinations thereof.

The length of a polynucleotide is not limited in any respect. Linkages between nucleotides can be phosphodiester linkages, or any other type of linkage such as phosphorothioate and 5'-N-phosphoramidite linkages. A polynucleotide can be produced by biological means (e.g., enzymatically), either *in vivo* (in a cell) or *in vitro* (in a cell-free system). A polynucleotide can be chemically synthesized using enzyme-free systems.

The term "polynucleotide" is not limited to naturally occurring polynucleotide structures, naturally occurring nucleotides, naturally occurring backbones or naturally occurring internucleotide linkages. One familiar with the art knows well the wide variety of polynucleotide analogues, unnatural nucleotides, non-natural phosphodiester bond linkages and internucleotide analogs that find use with the invention. Polynucleotides may thus include natural nucleosides (e.g., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), and/or nucleosides comprising chemically or biologically modified bases, (e.g., methylated bases), intercalated bases, and/or modified sugars (e.g., 2'-fluororibose, ribose, 2'-deoxyribose, arabinose, and hexose). Polynucleotides need not be uniformly modified along the entire length of the molecule. A polynucleotide comprising non-naturally occurring polynucleotide structures, sequences, backbones or internucleotide linkages is referred to as a "modified polynucleotide" herein. For example, different nucleotide modifications, different backbone structures, etc., may exist at various positions in the polynucleotide or oligonucleotide. Any of the polynucleotides described herein may utilize these modifications.

By convention, polynucleotides that are formed by 3'-5' phosphodiester linkages (including naturally occurring polynucleotides) are said to have 5'-ends and 3'-ends because the nucleotide monomers that are incorporated into the polymer are joined in such a manner that the 5' phosphate of one mononucleotide pentose ring is attached to the 3' oxygen (hydroxyl) of its neighbor in one direction via the phosphodiester linkage. Thus, the 5'-end of a polynucleotide molecule generally has a free phosphate group at the 5' position of the pentose ring of the nucleotide, while the 3' end of the polynucleotide molecule has a free hydroxyl group at the 3' position of the pentose ring. Within a polynucleotide molecule, a position that is oriented 5' relative to another position is said to be located "upstream," while a position that is 3' to another position is said to be "downstream." This terminology reflects the fact that polymerases proceed and extend a polynucleotide chain in a 5' to 3' fashion along the template strand. Unless denoted otherwise, whenever a polynucleotide sequence is represented, it will be understood that the nucleotides are in 5' to 3' orientation from left to right.

### Expression cassettes

The polynucleotide of the invention comprises a sensor expression cassette and an effector expression cassette. Both the sensor and effector expression cassette are thus located (or disposed) within the same nucleic acid molecule. This novel design advantageously enables the direct correlation (and quantification) of the effect mediated by the effector on the sensor comprised within the sensor expression cassette as described in greater detail below.

The term "expression cassette" refers a polynucleotide sequence capable of directing expression of one or more sequence(s) of interest in a suitable host cell. Expression cassettes typically comprise one or more sequence(s) providing an effector and regulatory elements operably linked thereto. As used herein, the terms "providing" means "encoding" or (being capable of) "yielding" a transcription product (e.g. a protein, (poly-)peptide or nucleic acid). The expressions "operably linked" "in operable combination," "in operable order," "operatively linked," "operatively joined" and similar phrases, when used in reference to polynucleotides, refer to the operational linkage of polynucleotide sequences placed in functional relationships with each other. For example, the transcription of a nucleic acid sequence is directed by an operably linked promoter sequence; post-transcriptional processing of a nucleic acid is directed by an operably linked processing sequence; the translation of a nucleic acid sequence is directed by an operably linked translational regulatory sequence; the transport or localization of a nucleic acid or polypeptide is directed by an operably linked transport or localization sequence; and the post-translational processing of a polypeptide is directed by an operably linked processing sequence. For example, promoters, enhancers, 5' and 3' UTR, and terminators are capable of regulating (i.e. controlling, regulating or modulating) the expression of an operably linked nucleic acid sequence of interest. Such control elements need not be contiguous with the coding sequence, so long as they function to direct its expression. Thus, for example, a promoter or terminator is "operably linked" to a coding sequence if it affects the transcription of the coding sequence. In some aspects, operably linked elements result in the transcription of an open reading frame and ultimately the production of a polypeptide (i.e., expression of the open reading frame). In some aspects, operably linked elements result in the transcription of a ribonucleotide encoding sequence and ultimately the production of a ribonucleotide. Regulatory elements are thus capable of regulating (i.e., controlling, directing or modulating) the expression of operably linked polynucleotide sequence(s) of interest. "Expression" of a polynucleotide sequence of interest as used herein generally refers to the processing of said sequence into a functional product encoded by said sequence. Expression of protein-encoding DNA sequences usually includes transcription of the encoded polynucleotide sequence into a transcript (especially an RNA transcript such as mRNA), and translation of said transcript into the encoded amino acid chain (polypeptide product). Expression may further include processing of the resulting transcript (e.g. by mRNA splicing) and/or post-translational processing of the resulting polypeptide. Expression of non-protein encoding DNA sequences typically involves transcription of complementary polynucleotide sequences into functional RNA products (e.g. shRNA, miRNA or precursors thereof).

Expression cassettes of the inventive polynucleotides typically comprise a transcription initiation site (usually comprising at least one promoter), a sequence providing the transcript interest, and a transcription termination site. In effector expression cassettes, said sequence providing the transcript of interest may be comprised in an open reading frame (ORF) providing (or encoding) the effector expression product. In sensor expression cassettes, said sequence providing the transcript of interest is typically the unique identifier sequence (UIS). Further, in sensor expression cassettes, the transcription initiation site typically comprises or corresponds to the sensor.

Expression cassettes may further comprise a 3' untranslated region (UTR) and/or a 5' untranslated region (UTR). The particular design of the sensor and effector expression cassette is within the skill and knowledge of the person skilled in the art and depends, *inter alia,* on the choice of the specific sensors and effectors and the host cell intended for expression.

As indicated previously, the sensor and effector expression cassette are located (or disposed) within the same nucleic acid molecule. However, it is to be understood that both expression cassettes are preferably not operably linked to each other in order to avoid a bias of the sensor response due to "cross-interaction" (or "interference") with regulatory elements controlling effector expression. As demonstrated in the appended examples, it is indeed possible to design polynucleotides wherein the sensor and effector expression cassette are located in relatively close proximity but without interfering with each other. In the polynucleotides according to the invention, the process of expressing the effector expression cassette thus preferably does not interfere with (i.e. alter, change, modify, influence or affect, e.g. induce, promote, terminate or prevent) the expression of the sensor expression cassette per se, and *vice versa.* Any undesired interference can be readily detected using the methods described in the appended examples.

### Sensor expression cassette

The sensor expression cassette comprises a sensor transcription control element of interest operably linked to a unique identifier sequence (UIS) encoding an RNA oligonucleotide. Expression of the UIS thus reflects the sensor response depending on the particular effector encoded by the same polynucleotide. The term "sensor response" refers to the activity of said sensor in terms of UIS expression. The sensor response is thus directly quantifiable by way of isolating, quantifying and identifying the RNA oligonucleotides expressed from the UIS. By way of example, if UIS expression is high, the sensor operably linked to the UIS may be considered "active", whereas if UIS expression is low, said sensor may be considered "inactive". Because the expression level of each RNA oligonucleotide transcribed from a UIS reflects the sensor response (active vs. inactive) and the identity of each RNA oligonucleotide reflects the identity of the gene-targeting effector provided by the same polynucleotide, the inventive polynucleotide allows for a "2 in 1" readout immediately correlating a particular effector to a specific sensor response in a pool of RNA oligonucleotides and does not have to rely on analyzing single cells.

### Sensor transcription control element

The term "transcription control element" refers to a polynucleotide sequence capable of regulating the transcription of operably linked polynucleotide sequences. The terms "transcription" and transcribing" in all their grammatical forms refer to the process of producing an RNA transcript using a DNA molecule as a template. The term generally includes transcription in a cell or *in vitro* transcription, wherein RNA, in particular mRNA, is in vitro synthesized in a cell-free system, preferably using appropriate cell extracts. It will however be readily acknowledged that in the screening assays of the present invention, transcription preferably takes places within a host cell. The "sensor transcription control element" (or "sensor") comprised by the sensor expression cassette of the inventive polynucleotide regulates the transcription of the UIS operably linked thereto. By way of example, in case the provided effector interferes with a positive regulator of the sensor (i.e. which induces or increases the sensor response), expression of the operably linked UIS will be reduced or inhibited; in case the provided effector interferes with a negative regulator of the sensor (i.e. which reduces or abolishes the sensor response), expression of the operably linked UIS is expected to be induced or increased; and in case the provided effector interferes with an entity that does not control the sensor response, expression of the operably linked UIS is expected to remain unchanged.

The sensor is typically capable of being regulated (i.e. controlled) by a target gene. The target gene is typically implicated in a biological event of interest. In the context of the present invention, the term "biological event" generally includes effects, interactions, processes, responses or pathways mediated by the (inter-)action of biological (macro-)molecules, in particular proteins, (poly-)peptides and nucleic acids. The term may include the presence of such effect, interactions, responses or pathways, or the absence of the same. The term "biological event" thus *inter alia* encompasses signaling pathways or processes triggered by or activated in response to intracellular or extracellular stimuli or events or the presence of genetic mutations, cellular states (activation, differentiation, different cell-cycle states such as interphase, mitosis, and cytokinesis), transcription factor activities, or promoter/enhancer activities. However, other biological events such as -in the simplest case- binding of a transcription factor to a regulatory sequence and activation of transcription of a gene operably linked thereto, are also encompassed by the term. Said biological event of interest preferably (directly or indirectly) induces activation or inactivation of the sensor (as preferably reflected by the expression levels of the UIS operably linked to said sensor). The sensor thus preferably represents an endpoint of a biological event of interest. The sensor response may be used as a surrogate readout for the occurrence or non-occurrence of the biological event of interest.

The choice of a suitable sensor depends *inter alia* on the biological event of interest to be elucidated. For instance, the present inventors utilized a modified SARE enhancer (enhanced SARE, E-SARE) as a sensor transcription control element in order to elucidate genes implicated in signaling upon neuronal activation. SARE (synaptic activity-responsive element) which has been described i.a. in Kawashima T el al. Proc Natl Acad Sci U S A. 2009 Jan 6;106(1):316-21 and Kawashima T et al. Nat Methods. 2013 Sep;10(9):889-95. The present inventors generated a modified SARE sensor with improved characteristics. This E-SARE sensor was chosen as a sensor based on its wide dynamic range and high signal-to-noise ratio upon synaptic silencing vs. synaptic activation.

For the identification of a suitable sensor, a pool of polynucleotides -each comprising a candidate sensor operably linked to a suitable reporter- can be introduced into the desired host cells intended for further screening (cf. the appended examples). Alternatively, reporter genes known in the art and exemplified elsewhere herein may be used as reporters. Thus, each of the candidate sensors is operably linked to a sequence encoding a reporter of choice. Subsequently, the response of all candidate sensors to the biological event of interest is quantified and compared to a control wherein the biological event is suppressed or not induced. Preferred sensors exhibit a wide dynamic range, i.e. a large ratio between the largest and smallest levels of expression of the employed reporter under different conditions (e.g. occurrence of biological event of interest vs. no occurrence of biological event of interest), and a high signal-to-noise ratio. Suitable sensors are typically robust, i.e. produce reliable and reproducible sensor responses under different conditions (e.g. occurrence of biological event of interest vs. no occurrence of biological event of interest). These characteristics facilitate the identification of true "hits" (i.e. target genes regulating the sensor of interest) in later screenings (see section captioned "Screening Methods" below). Further features of interest may include cell-type specificity, responsiveness to triggers and low selectivity.

By way of example, biological events and sensors of interest in the context of psychiatric and neurodegenerative diseases would be antioxidant response (using the ARE sensor), hypoxia (using the HRE sensor), hippo signaling (using the TEAD sensor), Wnt signaling (using the TCF-LEF sensor), unfolded protein response (using the ERSE sensor), and apoptosis (using the TP53INP1 sensor).

The present invention further features a library of polynucleotides which is described in further detail below. In some libraries of the invention, each polynucleotide comprises the same sensor, and provides a different effector. Such libraries can advantageously be used to simultaneously evaluate the effect of a multitude of effectors on a pathway controlling the response of a sensor of interest. In other libraries of the invention, each polynucleotide provides the same effector, but comprises a different sensor. Such libraries can advantageously be used to simultaneously evaluate the effect of a target gene on a multitude of different sensors.

The sensor is preferably selected from a promoter or an enhancer or any or any other control element that is capable of regulating transcription of a polynucleotide sequence operably linked thereto.

"Promoters" or "promoter sequences" are polynucleotide sequences located at the transcription initiation site (typically upstream or 5' of the site of transcription initiation) and initiate or induce transcription of an operably linked polynucleotide sequence. "Enhancers" are cis-acting polynucleotide sequences, which enhance the transcription from an operably linked promoter sequence. Enhancers function in an orientation and position-independent manner, i.e. an enhancer can function in any location, either upstream or downstream relative to the transcription initiation site. Thus, in case an enhancer is used as a sensor, it will typically be operably linked to a suitable promoter.

Transcription control elements such as promoters or enhancers may be selected from native (i.e. endogenous) or exogenous (i.e. foreign or synthetic) elements or functional variants thereof.

The term "functional variant" generally refers to a sequence (such as a polynucleotide or a polypeptide sequence) derived from or related to a reference (parent) sequence. The functional variant comprises an altered sequence as compared to said reference sequence, whilst retaining the biological function of said reference sequence. Functional variants also include fragments (portions or subsequences) of reference sequences. A functional variant can have 100% sequence identity with the reference sequence, or alternatively, can have less than 100% sequence identity with the reference sequence. In particular, a functional variant is envisaged to comprise at least one nucleotide deletion, substitution or insertion as compared to the reference sequence. As a result of the alterations, the functional derivative may comprise or consist a sequence which is at least 75%, preferably at least 80%, preferably at least 85%, preferably at least 90%, more preferably at least 95%, more preferably at least 96%, more preferably at least 97%, more preferably at least 98%, most preferably at least 99% or most preferably 100% identical to the reference sequence. Functional variants include sequences comprising the entire reference sequence, and further comprising additional sequences.

A functional variant of a sensor transcription control element is thus derived from or related to a reference (parent) sensor transcription control element and comprises an altered polynucleotide sequence as compared thereto, while retaining the capability of controlling the transcription of an operably linked UIS.

### Unique identifier sequence

The sensor expression cassette further comprises a unique identifier sequence (UIS) encoding an RNA oligonucleotide. The UIS is operably linked to the sensor, which thus controls expression of the RNA oligonucleotide transcribed from the UIS ("RNA barcode"). Preferably, the UIS comprises a specific part, and at least one primer binding site. The primer binding site can be located upstream or 5' of the specific part (referred to as a "5' primer binding site") or downstream or 3' of the specific part ("3' primer binding site"). The at least one primer binding site(s) allow(s) for the binding of specific primers for amplification of the UIS by PCR. The primer binding sites are, preferably, identical for all UIS of one library.

The UIS is also referred to as "unique expressed tag" or "EXT".

The specific part of a UIS comprises between 10 and 100 nucleotides, preferably between 40 and 60 nucleotides, and more preferably between 20 and 50 nucleotides The specific part is located between the 5' primer binding sites and the 3' primer binding site. It is unique, i.e. different from all other specific parts present in one library or employed in one pooled screening method. Thus, the sequence of one UIS preferably differs from the sequence of all of the other UIS in a polynucleotide library by at least 1, preferably at least 2, or more preferably more than 2 nucleotides. The different nucleotides are preferably located in the specific part of each UIS. Each UIS and the respective RNA oligonucleotide transcribed therefrom is thus, by way of the sequence of its specific part, capable of specifically identifying (1) the polynucleotide comprising the UIS and (2) all other elements provided or comprised by said polynucleotide (including the effector or the sensor). As used herein, the term "specifically identifying" thus means characterizing, distinguishing or differentiating one element from another, i.e. indicating the presence of a particular element (such as the polynucleotide and/or effector provided by or the sensor harbored in said polynucleotide) more readily or more likely than the presence another element different therefrom.

Each UIS is placed operably linked to the sensor. The sensor thus controls the transcription of the (unique) RNA oligonucleotide transcribed from the UIS. Said RNA oligonucleotide is preferably not translated into a peptide or protein. Each UIS is unique and assigned to a distinct polynucleotide providing either a distinct effector or harboring a distinct sensor. Advantageously, UIS expression levels as determined by amplification and identification of the expressed RNA oligonucleotides, directly reflect the response of the operably linked sensor.

The specific part of each UIS comprises between 10 and 100 nucleotides, preferably between 40 and 60 nucleotides and more preferably between 20 and 50 nucleotides. Preferably, the specific part comprises nine nucleotides flanked on both sides by five "words", a design optimized to allow unbiased amplification and optimal performance for microarray analysis.

Preferably, said "words" are assembled as taught in WO 00/20639, US 7,393,665 or (Brenner et al., 2000).

It is particularly preferred that the design of the UIS employed in the inventive polynucleotide corresponds to the design of the so-called "(unique) expressed tags" or "EXTs" disclosed in WO 2010/034773 A1. Specifically, "unique expressed tag" (EXT) according to WO 2010/034773 A1 is a nucleic acid sequence that comprises three sequence parts. Defined primer binding sites are located at the 5 '-end and at the 3 '-end of the EXT and correspond to the UIS primer binding sites. The "specific part" of the EXT is located between the primer binding sites and corresponds to the specific part of the UIS.

Each UIS specific part comprises or consists of a variable region comprising 49 bases and invariable 5' and 3' sequences (primer binding sites). The variable region may consist of several words (W) flanking a core region. Eight different 4-nucleotide words are typically used, each comprised of 3 Adenosine (A) / Thymidine (T) residues and one Cytosine (C) residue (5' CTTT 3', 5' CAAA 3', 5' ACAT 3', 5' TCTA 3', 5' TACT 3', 5' ATCA, 3' 5' TTAC 3', 5' AATC 3') (Brenner S et al. Proc Natl Acad Sci U S A. 2000 Feb 15;97(4):1665-70). The core region comprises nine bases of alternating A,T (W) or G,C (S) residues with three central G,C (S) residues. The invariable 5' region operably linked to the specific part represent short sequence stretches of similar melting temperature (e.g. 5' TAGGTGACACTAT 3 ' SEQ ID NO: 1) and 3' (e.g. 5' CCTATAGTGAGTCGT 3' SEQ ID NO: 2).

UIS oligonucleotide libraries can be generated with standard deoxy-nucleic-acid (DNA) oligonucleotide synthesis chemistry. Usually, the synthesis proceeds from the 3' to the 5' end. The synthesis of a UIS library can be initiated with eight reactions and the invariable 3' region attached to one of the eight different words. After the first synthesis cycle, the resins carrying the nucleotides can be mixed and subsequently divided into eight equal portions to add the next eight words. The fifth cycle can be extended with the core sequence followed by another five word cycles and the invariable 5' region. UIS libraries can then be cloned into appropriate polynucleotides or vectors and operably linked to the sensor of interest, as described into the appended examples.

### Reporter gene

The sensor expression cassette may further comprise a reporter gene operably linked to the sensor. Said "reporter gene" preferably encodes a detectable reporter or marker. Under appropriate physiological conditions, the reporter gene will be expressed to yield said detectable markers, which presence can be readily observed. Detectable markers thus serve to indicate, preferably visualize, sensor responses. Suitable detectable markers include, without limitation, fluorescent proteins, chromoproteins, luciferase proteins and enzymes that produce colored substrates. Chromoproteins can be detected with the naked eye or using a microscope. Exemplary chromoproteins include amilCP proteins, cjBlue proteins, meffBlue proteins, tsPurple proteins, amajLime proteins, fwYellow proteins, eforRed proteins, amilGFP proteins, aeBlue proteins, Cre-Dronpa Fusion proteins, gfasPurple proteins, spisPink proteins. It will be readily acknowledged the definitions of chromoproteins and fluorescent proteins may have a certain overlap. Fluorescent proteins can be readily detected using a microscope, plate reader or flow cytometer equipped to excite the fluorescent protein with the appropriate wavelength of light. Exemplary fluorescent proteins include green fluorescent protein (GFP) and its derivatives (such as EYFP, ECFP, GFPmut3b), yellow fluorescent protein (YFP) and its derivatives, and Red fluorescent proteins (RFP) and their derivatives (such as mRFP1, mOrange, mCherry). Luciferases can be readily detected using a plate reader or luminescence counter. Exemplary luciferases include Firefly luciferase, Gaussia luciferase and Renilla luciferase. Enzymes that produce colored substrates can be detected with the naked eye or under a microscope and can be quantified using spectrophotometers or other instruments that can take absorbance measurements including plate readers. Examples of such enzymes include beta-galactosidase encoded by the lacZ gene, which is detected using an enzymatic assay with X-gal, beta-lactamase and secreted alkaline phosphatase, which can be detected by an enzymatic assay using a substrate such as BM Purple AP Substrate (Boehringer Mannheim).

### Effector expression cassette

The polynucleotide of the invention further comprises an effector expression cassette encoding an effector expression product ("effector"). Said effector is envisaged to be expressed and capable of exerting its desired biological activity under appropriate physiological conditions (i.e. in a suitable host cell). The polynucleotide according to the invention combines a specific effector expression cassette (and its provided effector) with the sensor of interest and a UIS. Expression levels of the UIS reflect the sensor response in the presence of a particular effector. Thus, an effector capable of influencing the response of a certain sensor can directly be identified via quantification and identification of the RNA oligonucleotides transcribed from the UIS, without the need of analyzing single cells.

### Effector expression products

The polynucleotide of the invention is particularly useful to elucidate gene function in a pooled screening. To this end, polynucleotides according to the invention are introduced into suitable host cells. Said polynucleotides provide various effectors that are preferably evaluated with respect to their effect on the biological activity of the utilized sensor of interest. Effectors are particularly envisaged to exert their effect on the sensor response via interfering with target genes. In this context, "interfering with" or "targeting" a target gene means modifying its presence, sequence, structure or biological function of a target gene or its provided functional product (e.g. a nucleic acid or a peptide or protein). The effector is thus preferably capable of specifically modifying a target gene or the expression of said target gene.

"Modifying a target gene" includes modifying the presence or absence or sequence of a target gene in the genomic DNA. Thus, the present invention envisages effectors capable of mediating gene knock-out, or knock-in or the introduction of modified sequences. Such effectors may rely on the introduction of double-strand breaks (DSBs) into the genomic DNA and subsequent repair by non-homologous end-joining (NHEJ) -introducing random mutations or deletions- or the incorporation of a template by homology-directed repair. Components of the CRISPR/Cas system can be employed for such purposes.

The biological function a gene includes its expression to yield a functional product. Thus, effectors in accordance with the invention interfere with the biological function of a target gene by modifying its expression. "Modifying the expression of a target gene" includes altering (i.e. inducing, enhancing, reducing, interfering with or inhibiting) gene expression at a variety of levels, including via modification of chromatin domains, transcription, post-transcriptional modification, RNA transport, translation, or mRNA degradation. RNAi-inducing agents such as siRNAs or shRNAs can interfere with transcription and/or translation of an mRNA transcribed from a (protein-encoding) target gene. Modified CRISPR/Cas components can be employed for modifying gene expression in processes called CRISPRa and CRISPRi.

In any case, effectors are employed to elucidate or detect gene function in biological events which ultimately regulate the response of the sensor of interest.

The effectors provided by the inventive polynucleotide can be selected based on their known interference with particular target genes or can be selected randomly. Advantageously, the present invention enables the screening of a multitude of effector expression products having the ability of interfering with target genes.

Effectors may be selected from proteins, peptides, aptamers or nucleic acids, and functional variants and derivatives thereof, preferably capable of interfering with a target gene. Effector proteins include meganucleases, zinc finger nucleases and TALENs. Suitable effectors also include components of the CRISPR/Cas system. As described in greater detail below, the use of the CRISPR/Cas system relies on the presence of two molecules in each host cell -a CRISPR-associated (Cas) nuclease and a guide RNA (gRNA). In cases where the CRISPR/Cas system is employed, it is envisaged that (target gene specific) sgRNAs are used as effectors. In other words, the inventive polynucleotide may comprise an effector expression cassette providing a gRNA as an effector. In some cases, it may be desirable to a polynucleotide which provides not only the gRNA, but also the Cas nuclease. Suitable Cas nucleases and variants and derivatives thereof are described in greater detail below. In other cases (e.g. when the Cas gene is provided in an extra expression vector and/or the host cells are modified to stably express a suitable Cas nuclease), the inventive polynucleotide does not need to encode a Cas nuclease and may only provide the gRNA. In both cases, when using the CRISPR/Cas system, the (g)RNA (optionally in combination with the Cas nuclease) qualifies as the "effector" within the meaning of the present invention

The term "aptamer" refers to (RNA or DNA) oligonucleotides or (oligo-)peptides capable of specifically binding to a molecular target (such as a protein). Nucleic acids of interest include DNA and RNA encoding proteins or peptides capable of interfering with target genes. Nucleic acids of interest further include non-protein encoding nucleic acids, in particular non-coding RNA (ncRNA) which is also known as non-protein-coding RNA (npcRNA), non-messenger RNA (nmRNA), or functional RNA (fRNA). The term "ncRNA" refers to natural or synthetic RNA sequences (or RNA molecules comprising those sequences) which are not translated into an amino acid sequence and generally includes transfer RNAs (tRNAs), ribosomal RNAs (rRNAs), small nucleolar RNAs (snoRNAs), microRNAs (miRNAs), decoy RNAs, small interfering RNAs (siRNAs), small hairpin RNAs (shRNAs), small nuclear RNAs (snRNAs), extracellular RNAs (exRNAs), piwi-interacting RNAs (piRNAs), Small Cajal body RNAs (scaRNAs), single guide RNAs (sgRNAs), long ncRNAs or ncRNA inhibitors. Some effectors may act as gene editing agents. Other effectors may act as RNAi inducing agents. Effectors may be naturally occurring or synthetically generated.

Sequences encoding the effectors can be isolated from genomic or cDNA libraries using standard techniques well known in the art. Such libraries are commercially available. In the appended examples, a pooled lentiviral shRNA library commercially available from Cellecta was used. However, other libraries may also be used. Alternatively, sequences encoding effectors can be synthesized chemically.

In the following, some useful effectors are discussed in the context of their supposed mechanism of action (e.g. RNAi or gene editing). However, disclosure of such mechanism of action of (potential) effectors is not intended to be binding or limiting. Thus, in case one of the suggested effectors is found to be interfering via a different mechanism of action, its use in the polynucleotide of the invention is nevertheless envisaged.

### RNAi-inducing agents

"RNA interference or "RNAi" is a biological process in which RNA molecules inhibit gene expression, typically by causing the degradation and/or inhibiting the translation of a target transcript (usually an mRNA) (cf. Ozcan G et al. Adv Drug Deliv Rev. 2015 Jun 29; 87: 108-119 for review). Briefly, double-stranded RNA (dsRNA) is recognized by Dicer, an RNase type III enzyme, which cleaves the dsRNA into small fragments of 21-23 base pairs in length. The dsRNA comprises sequences forming a sense (passenger) strand and an antisense (guide) strand with respect to the target transcript. The dsRNA fragment binds to the RISC (RNA-induced silencing complex) protein complex, and the passenger strand of dsRNA is cleaved and discarded while the guide strand directs RISC to a complementary sequence in the target transcript. This complementary sequence is often located in the 3'-untranslated region (UTR) of the target transcript. RNAi can be induced via the siRNA pathway or the miRNA pathway.

Endogenously expressed siRNAs have not been found in mammals, but they can be produced from a dsRNA and a small hairpin RNA (shRNA) by Dicer cleavage or produced by RNase III nuclease activity. In either case, siRNA associates in the cytoplasm with a protein complex called the RNA induced silencing complex (RISC), whereupon one of the two RNA strands (passenger strand) is degraded and the other "guide" strand guides the RISC to mediate sequence-specific degradation of the corresponding mRNA. Dicer delivers the siRNA to a group of proteins called RNA-inducing silencing complex (RISC), where the catalytic component argonaute (Ago) is capable of degrading the siRNA into a single strand to bind to the corresponding mRNA and further degrade the mRNA, resulting in gene silencing.

The miRNA-mediated RNAi pathway is induced by miRNAs, naturally occurring small non-coding dsRNAs that are initially transcribed as pri-mRNAs (i.e. complex stem-loop or short hairpin structures). Pri-mRNAs are pre-processed by a nuclear RNase III (Drosha) into pre-mRNAs before being exported into the cytoplasm by exportin 5. Dicer cleaves the pre-miRNAs into shorter double-stranded miRNAs with imperfect complementarity. These short fragments are recognized by Argonaute 2 (AGO2) and RNA-induced silencing complex (RISC). As described above, one of the strands is degraded and the other strand guides the AGO2-RISC complex to bind and block translation of target mRNAs having partial complementary sites typically located in the 3'-UTR. Owing to imperfect matching with 3' UTRs, miRNAs in some instances do not lead to the cleavage of mRNA with the RISC but instead result in translational suppression.

The siRNA-mediated RNAi pathway can be induced by the introduction of chemically or enzymatically synthesized double-stranded small interfering (si) RNA or by intracellular generation of siRNAs from long dsRNAs or precursor small hairpin (sh) RNAs. Chemically or enzymatically synthesized double stranded siRNAs usually mimic the structure of Dicer-processed products and thus bypass the Dicer cleavage step. siRNAs are incorporated into the RISC and target the AGO2-RISC complex to complementary target transcripts. Long dsRNAs or shRNAs can be introduced into the host cell or be transcribed from inserted (or native) polynucleotide sequences within the host cell. By way of example, oligonucleotides containing the siRNA sequence followed by a ∼9 nt loop and a reverse complement of the siRNA sequence may be cloned in plasmid or viral vectors to endogenously express shRNA which is exported out of the nucleus by exportin 5, and is subsequently processed in the cytoplasm by Dicer into siRNA in association with dsRNA binding proteins like TRBP and PACT 16. ShRNA are typically transcribed under the control of RNA Pol-II or Pol-III promoters Because shRNA can be produced continually within the cell, the gene silencing effect is long lasting (weeks to months). Long dsRNAs introduced into or present within the host cell are processed Dicer into shorter siRNA fragments with two nucleotide (2-nt) 3' overhangs and 5' phosphate groups., and fold into a structure resembling a siRNA duplex which is then processed by Dicer into siRNAs. shRNAs and long dsRNAs are therefore considered to be siRNA precursors. Again, siRNAs are incorporated into the RISC and target the AGO2-RISC complex to complementary target transcripts. siRNAs are typically designed to bind target sequences with perfect or nearly perfect complementarity and usually cause cleavage of targets rather than of translational suppression.

As used herein, the term "RNA interference or "RNAi" includes siRNA-mediated and shRNA-mediated RNAi as well as miRNA-mediated RNAi resulting in a reduced expression of target transcripts (in particular mRNAs), optionally by transcript cleavage and/or translational repression. RNAi thus typically leads to silencing (i.e. reduced expression) of the gene encoding the targeted transcript.

As used herein, the term RNAi-inducing agent refers to an agent, typically an RNA molecule, which is capable of inducing RNAi in a host cell. In this context, the term "induce" is not intended to indicate that the agent activates or upregulates RNAi in general but rather to indicate that the agent activates or upregulates RNAi with regard to its specific targeted transcript, i.e. that the presence of the agent within a cell results in RNAi-mediated reduction in expression of a transcript to which the agent is targeted in a target-specific manner. Preferably, an "RNAi inducing agent" reduces the expression of a targeted transcript (typically an mRNA) or is processed within a cell into an agent that reduces the expression of a targeted transcript (typically an mRNA). Specifically, the RNAi-inducing agent may be an interfering RNA. Interfering RNAs may be designed as antisense oligonucleotides that block expression of a DNA or RNA target by complementary binding to the target sequence and halting expression at the level of transcription, translation, or splicing.

The interfering RNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure); the antisense strand comprises nucleotide sequence that is complementary to a nucleotide sequence in a target nucleic acid molecule or a portion thereof (i.e., a target gene) and the sense strand comprises nucleotide sequence corresponding to the target nucleic acid sequence or a portion thereof. Alternatively, interfering RNA is assembled from a single oligonucleotide, where the self-complementary sense and antisense regions are linked by means of nucleic acid based or non-nucleic acid-based linker(s). The interfering RNA can be a polynucleotide with a duplex, asymmetric duplex, hairpin or asymmetric hairpin secondary structure, having self-complementary sense and antisense regions, wherein the antisense region comprises a nucleotide sequence that is complementary to a nucleotide sequence in a separate target nucleic acid molecule or a portion thereof and the sense region having nucleotide sequence corresponding to the target nucleic acid sequence or a portion thereof. The interfering RNA can be a circular single-stranded polynucleotide having two or more loop structures and a stem comprising self- complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in a target cell to generate an active interfering RNA capable of mediating RNA interference.

Particularly envisaged RNAi inducing agents include miRNAs, siRNAs, shRNAs, or precursors or functional variants or derivatives of any of the aforementioned RNAi inducing agents. The term "precursor" refers to molecules that are capable of being processed by the host cell into or that self-assembles into functional siRNAs, shRNAs or miRNAs, i.e. siRNAs, shRNAs or miRNAs capable of inducing RNAi.

### siRNA

As used herein, the term "siRNA" or "small interfering RNA" refers to small (∼12-35 nucleotide) non-coding RNA molecules capable of inducing RNAi. siRNAs comprise an RNA duplex (double-stranded region) formed by complement base pairing with phosphorylated 5'-ends and hydroxylated 3'-ends, optionally with one or two single-stranded overhanging nucleotides. The duplex portion typically comprises between 17 and 29 nucleotides. siRNA may be generated from two RNA molecules that hybridize together or may alternatively be generated from a single RNA molecule that includes a self-hybridizing portion (shRNA). The duplex portion of an siRNA may, but typically does not, include one or more bulges containing one or more unpaired and/or mismatched nucleotides in one or both strands of the duplex or may contain one or more non-complementary nucleotide pairs. One strand of a siRNA (referred to as the antisense strand) includes a portion that hybridizes with a target transcript (e.g. a target mRNA). The antisense strand may be precisely complementary with a complementary region of the target transcript (i.e. the siRNA antisense strand may hybridize to the target transcript without a single mismatch) or one or more mismatches between the siRNA antisense strand and the complementary region of the target transcript may exist.

As used herein, the term "siRNA" includes naturally occurring siRNAs as well as synthetic siRNAs or precursors or functional derivatives thereof. Hence, the effector expression cassette of the inventive polynucleotide may comprise a sequence encoding a siRNA or a precursor or functional derivative thereof. Particularly useful precursors include long dsRNA molecules and small hairpin RNAs (shRNAs) which are processed by the RNAi machinery to generate siRNAs.

### shRNA

The term "short hairpin RNA" or "shRNA" refers to single-strand RNA molecules comprising at least two complementary portions hybridized or capable of hybridizing to form a double-stranded (duplex) structure sufficiently long to mediate RNAi. These complementary portions are generally between 17∼29 nucleotides in length, typically at least 19 base pairs in length. shRNAs further comprise at least one single-stranded portion, typically between 1∼10 nucleotides in length that forms a loop connecting the complementary strands forming the duplex portion. The duplex portion may, but typically does not, contain one or more bulges consisting of one or more unpaired nucleotides. As described above, shRNAs are thought to be processed into siRNAs (see above) by the RNAi machinery. shRNAs are therefore siRNA precursors and are thought to induce gene silencing via the siRNA-mediated RNAi pathway.

The effector expression cassette according to the invention may therefore provide a shRNA or a precursor or functional derivative thereof as an effector. The shRNA or precursor or functional derivative thereof is preferably capable of being processed into siRNA and of inducing the siRNA-mediated RNAi pathway in an appropriate host cell wherein it is expressed.

### MicroRNAs

The term "microRNA" or "miRNA" refers to small (∼20-24 nucleotide) non-coding double-stranded RNAs (dsRNAs) capable of recruiting the AGO-2 RISC complex to a complementary target transcript, thereby preferably inducing the miRNA-mediated RNAi pathway. The term "microRNA" includes miRNAs, mature single stranded miRNAs, precursor miRNAs (pre-miRNA), primary miRNA transcripts (pri-miRNA), duplex miRNAs and variants thereof, which may be naturally occurring or synthetic.

The effector expression cassette according to the invention may therefore provide a miRNA or a precursor or functional derivative thereof as an effector. The miRNA precursor or functional derivative thereof is preferably capable of inducing the miRNA-mediated RNAi pathway in an appropriate host cell wherein it is expressed.

### NcRNA inhibitors

Effectors provided by the polynucleotide according to the invention may also be ncRNA inhibitors. By way of example, miRNA inhibitors may reduce or abolish the inhibitory effect exerted by the targeted miRNAs on the expression of target genes of interest. Thus, miRNA inhibitors such as Decoy RNAs or circRNAs may be used to induce or increase the expression of target genes of interest that is otherwise hampered by the action of miRNAs.

### Decoy RNA

The term "Decoy RNA" as used herein refers to synthetic non-coding RNAs acting as inhibitors of other non-coding RNAs (such as miRNAs). Decoy RNAs (including miRNA inhibitors previously referred to as anti-miRs, antagomiRs, AMOs [Anti-miRNA antisense inhibitors] and RNA sponges) are typically antisense molecules that bind and sequester complementary ncRNAs (in particular miRNAs) from their natural targets in a sequence-specific manner (cf. Bak RO et al. Mol Ther. 2013 Aug; 21(8): 1478-1485 for review). Based on their mechanism of action, decoy RNAs are thus also classified as competing endogenous RNAs (ceRNAs). The term "Decoy RNA" encompasses short antisense RNAs without additional sequences as well as structured scaffolds or sponges encompassing multiple (i.e. at least two) ncRNA binding sites.

### circRNA

Recently, circular RNAs (circRNAs) have emerged as a new, highly prevalent, and conserved class of RNAs, which are derived from head-to-tail splicing of exons. Many of these circRNAs contain putative miRNA target sites and may therefore function as ceRNAs. circRNAs may function in a similar manner as Decoy RNAs and are therefore also envisaged as effectors according to the present invention.

### Gene editing agents

The effector used for targeting target genes in a host cell of interest may also be a gene (or genome) editing agent. Gene editing (or genome editing) refers to the insertion, deletion or substitution of polynucleotide sequences in the genome of an organism or cell. The gene editing agent is preferably targeted to a specific recognition site of interest, i.e. capable of specifically inserting, deleting or substituting single nucleotides, polynucleotide sequences or chromosomal segments into or from a specific recognition site in the host cell's genome. Preferably, by introducing said insertions, deletions or substitution introduced by said gene editing agents interferes (as described elsewhere herein) with at least one target gene of interest. By way of example, the action of the gene editing agent can introduce modifications that result either in loss-of-function (i.e. removal, inactivation or (partial) inhibition of functional parts, such as genes or transcription control elements, from or in the genome) or gain-of-function (i.e. introduction, reconstitution or (partial) activation of functional parts, such as genes transcription control elements, into or in the genome). Gene editing is thus a useful technique for studying the effect of one or more gene(s) of interest on a specific sensor.

Targeted gene editing can be achieved by delivering a site-specific (targeting) nuclease that creates a double-strand break (DSB) at a recognition site of interest. Such targeting nucleases are typically composed of a customizable sequence-specific DNA-binding domain that can be engineered to target specific recognition sites of interest, and a nuclease domain that cleaves DNA in a non-sequence-specific manner. Cleavage of the DNA by the action of the nuclease domain results in the generation of DNA double-strand breaks (DSBs) at the targeted recognition site. The DSBs are repaired by non-homologous end-joining (NHEJ) or homology-directed repair (HDR) (also referred to as "homologous recombination" or "HR") -both of which can be exploited to introduce targeted gene alterations in a wide range of organisms and cell types. NHEJ-mediated repair of a nuclease-induced DSB leads to the efficient introduction of variable length deletions that originate at the site of the break. Thus, NHEJ-mediated repair of DSBs introduced into gene coding sequences may yield frame shift mutations that can lead to knockout of gene function. Alternatively, if a double-stranded DNA "donor template" is supplied, HDR of a nuclease-induced DSB can be used to introduce precise nucleotide substitutions or insertions. The terms "donor template", "donor sequence" or "recombination substrate" refer to double.-stranded DNA molecules comprising polynucleotide sequences largely homologous to the targeted locus in the genome but carrying the specific sequence modifications. When this donor template is used for HDR, the sequence modification present in the donor template is incorporated at the target site. Such donor templates are thus envisaged to allow for a precise genetic modification by HDR. Such "donor templates" intended as HDR substrates can be provided as part of the polynucleotide of the invention or as part of a different polynucleotides (in particular a suitable DNA vector).

Four major platforms currently exist for inducing these site-specific DSBs: zinc finger nucleases (ZFNs), transcription activator-like effector (TALE)-nucleases (TALENs), meganucleases, and most recently the CRISPR/Cas system (reviewed *i.a.* in Maeder and Gersbach Mol Ther(2016); 24 (3), 430-446). Accordingly, useful effector expression products capable of acting as gene editing agents include meganucleases, ZFNs, TALENs, and components of the CRISPR-Cas system.

### Meganucleases

Meganucleases, also referred to as "homing endonucleases", are nucleases capable of specifically recognizing and targeting double-stranded DNA sequences of 12 to 40 base pairs. Meganucleases introduce DSBs in a sequence specific manner. DSBs induce the cellular repair mechanisms NHEJ or HR (if a suitable donor sequence is provided) which can be exploited for targeted gene editing as described above.

There are five families (or classes) of meganucleases. The largest class of meganucleases is the LAGLIDADG family, which includes the well-characterized and commonly used I*-Cre* I and I-*Sce* I enzymes (cf. Molina, et al. 2011. Meganucleases and Their Biomedical Applications. In: Encyclopedia of Life Sciences (ELS). John Wiley & Sons, Ltd: Chichester for review). Meganucleases such as I*-Cre* I and I*-Sce* I may be "programmed" to target genes of interest and used as effector expression products according to the present invention. In this respect, known meganucleases can be used as "scaffolds" for creating functional sequence variants capable of targeting recognition sites of interest. Routine genetic engineering methods (cf. Sambrook J et al. 2012. Molecular Cloning: A Laboratory Manual (4th Edition)) can be employed to introduce amino acid modifications (insertions, deletions, or substitutions) into known meganuclease sequences. Subsequently, functional variants can be selected by routine methods such as phage display or the yeast two-hybrid system based on their capability of recognizing the desired target sequence. Additionally, or alternatively, functional domains from other effector proteins can be associated or fused to the meganucleases in order to develop chimeric derivatives exhibiting the desired binding specificity. By way of example, fusion proteins of meganucleases, zinc finger nucleases (ZFs) and/or TALEs (which are discussed below) that take advantage of the binding affinity of ZFs and TALEs and the cleavage specificity of meganucleases are envisaged as useful derivatives in accordance with the invention.

The effector expression cassette according to the invention may therefore encode (or provide) a meganuclease or a functional derivative thereof as an effector expression product, and optionally a donor template. Alternatively, the donor template may be comprised by a different polynucleotide (in particular a DNA vector provided simultaneously with the polynucleotide of the invention).

### Zinc finger nucleases

Cys2His2 zinc fingers are DNA-binding domains that each recognize approximately three base pairs of DNA. Alteration of a small number of residues in or near an alpha-helix within this domain can lead to changes in its DNA-binding specificity. Engineered ZFs can be joined together into more extended arrays capable of recognizing longer DNA sequences of interest. Various publicly available methods for customizing ZFs have been described. Modular assembly of individual pre-selected ZFs domains provides a simple and rapid method for creating customized arrays exhibiting the desired binding specificity. Additional methods that explicitly account for context-dependent effects among ZFs in an array include: Oligomerized Pool Engineering (OPEN), Context-Dependent Assembly (CoDA), and a bacterial one-hybrid (B1H) selection-based system. Subsequently, functional variants can be selected by routine methods such as phage display or the yeast two-hybrid system based on their capability of recognizing the desired target sequence.

"Zinc finger nucleases" or "ZFNs" (cf. Chou ST et al. Drugs Future. 2012 Mar 1; 37(3): 183-196) are chimeric restriction enzymes which comprise at least one DNA binding Cys2-His2 (C2H2) zinc finger domain and at least one non-specific nuclease domain. The domains may be fused together via a suitable linker, specifically a peptide linker. Several ZF domains and nuclease domains may be combined to form an array. The term "ZFN" encompasses zinc finger nucleases comprising a nuclease domain may be derived from the Type IIS FokI restriction enzyme, including (wild-type) homodimeric or (engineered) heterodimeric FokI domains. However, any other functional nuclease domain is also envisaged.

ZNFs enable targeted gene editing by creating DNA double-strand breaks (DSBs) in DNA at in a sequence-specific manner. Said DSBs induce the cellular repair mechanisms NHEJ or HR (if a suitable donor sequence is provided) which can be exploited for targeted gene editing as described above.

The effector expression cassette according to the invention may therefore encode (or provide) a ZFN or a functional derivative thereof as an effector, and optionally a donor template. Alternatively, the donor template may be comprised by a different polynucleotide (in particular a DNA vector provided simultaneously with the polynucleotide of the invention).

### TALENs

Similar to ZNFs, Transcription activator-like effector nucleases (TALENs) (reviewed in Joung and Sander, Nat Rev Mol Cell Biol. 2013; 14(1): 49-55) are chimeric restriction endonucleases comprising a customizable DNA binding domain fused to a non-specific nuclease domain. The DNA binding domain comprises typically 7-34 highly homologous direct repeats, each typically consisting of 33-35 amino acids, that are derived from transcription activator-like effectors (TALEs), which are naturally produced by *Xanthomonas* proteobacteria. These highly conserved TALE repeats are typically 33-35 amino acid in length and bind a single base pair of DNA with specificity dictated by two hyper variable residues (repeat variable residues, RVD). By leveraging the technologies and methodologies previously developed for ZFN engineering (see above), TALE domains can be customized to specifically recognize target sites of interest. The term "Transcription activator-like effector nuclease" or "TALEN" as used herein encompasses fusion proteins comprising at least one TALE domain and at least one nuclease domain. The non-specific nuclease domain may be a Fokl nuclease domain as described in the context of ZFNs above or any other functional nuclease domain. The domains may be fused together via a suitable linker, specifically a peptide linker.

TALENs function in a similar manner as ZNFs by recognizing target sites of interest (typically 15 to 20 base-pairs in length) and creating DNA double-strand breaks (DSBs) in a sequence-specific manner. Said DSBs induce the cellular repair mechanisms NHEJ or HR (if a suitable donor template is provided) which can be exploited for targeted gene editing as described above.

The effector expression cassette according to the invention may therefore encode (or provide) a TALEN or a functional derivative thereof as an effector expression product, and optionally a donor template. Alternatively, the donor template may be comprised by a different polynucleotide (in particular a DNA vector provided simultaneously with the polynucleotide of the invention).

### CRISPR/Cas

Components of the CRISPR/Cas system -specifically, sgRNAs in combination with a suitable Cas nuclease- are particularly preferred effectors in the context of the present invention. As described in greater detail below, components of the CRISPR/Cas system or their variants or derivatives can be employed to modify (the presence or absence or sequence of) target genes -i.e. its components may qualify as "gene editing agents" as described above- or can be used to modify the target gene expression (in particular when targeting regulatory sequences driving the expression of target genes). CRISPR technologies can be employed for a variety of purposes, including functional knockout or knock-in of genes, gene editing or transcriptional activation or inhibition. All of these applications require the introduction of two molecules into each host cell -a CRISPR-associated (Cas) nuclease and a guide RNA (gRNA).

The CRISPR/Cas system confers adaptive immune protection to prokaryotes against invading DNA elements (e.g., viruses, plasmids) in a sequence-specific manner. CRISPR/Cas loci are present in both bacteria and archaea (48% and 84% respectively). Based on the conservation and composition of the Cas genes, the CRISPR/Cas systems have been classified into three major types (I, II and III).

The central mechanism of all three CRISPR/Cas types is an RNA 'guide' (gRNA) that targets the CRISPR-associated (Cas) nuclease to a specific stretch of DNA sequence by complementary binding. The Cas nuclease and the gRNA form a complex with genomic DNA, specifically targeting DNA sites complementary to an approximately 17-20-base sequence within the gRNA and neighboring a protospacer adjacent motif (PAM) which is recognized by the Cas nuclease.

The type II CRISPR/Cas9 system from *Streptococcus pyogenes* is currently the most commonly used system for targeted DNA editing in eukaryotic cells and comprises two different RNA subunits (CRISPR RNA (crRNA) and a trans-activating RNA (tracrRNA)) in complex with a single Cas9 nuclease, whereas type I and III systems comprise multiple Cas proteins in complex with a single RNA. The two RNAs of the type II system can be fused into a chimeric single guide RNA (sgRNA). This sgRNA can be engineered to target a 17-20 base pair stretch of DNA sequence preceding a PAM. The *Streptococcus pyogenes* Cas9 nuclease recognizes the optimal PAM site NGG or, to a much lesser extent, NAG. The NGG PAM sequence occurs on approximately every 8 bp in the human genome, leaving many available target sites for *Streptococcus pyogenes* Cas9.

The wild-type *Streptococcus pyogenes* Cas9 nuclease (wtCas9) has two endonuclease domains that are capable of producing double-stranded breaks (DSBs) in the targeted sites in the genomic DNA. Said DSBs are subsequently repaired through endogenous DNA repair mechanisms, either non-homologous end-joining (NHEJ) or homology-directed repair (HDR).

Repair of the cleaved DNA by non-homologous end joining (NHEJ) introduces random mutations into the cleaved site (which could potentially lead to the disruption of the codon-reading frame, resulting in erroneous transcripts and ablation of gene expression), while homology-directed repair (HDR) employs a homologous DNA template to introduce specific sequences, as in homologous recombination. Both these types of host DNA repair response mechanisms to CRISPR/Cas9 mediated DSBs offers various types of applications for CRISPR/Cas9 in mammalian gene editing. NHEJ can lead to ablation of gene mutations, and hence can be used to create loss of function effects. HDR can be used for introducing specific point mutations, or introducing DNA segments of varying length.

However, the CRISPR/Cas system can also be used to modulate target gene expression. For instance, Cas9 (either wt or Cas9 variants, fragments or derivatives as described herein) can be used in combination with transcription repression domains (such as KRAB, SID4X) to downregulate target gene transcription. Alternatively, Cas9 (either wt or Cas9 variants, fragments or derivatives as described herein) can be fused to transcription activation domains to upregulate target gene transcription (such as p65 or VP64).

### gRNAs

A functional CRISPR/Cas system requires the presence of both a gRNA (also referred to as a "guide RNA" or "targeting RNA", typically a sgRNA) and a Cas nuclease. Thus, gRNAs, and in particular sgRNAs, are preferred effectors in accordance with the present invention.

The "gRNA" preferably comprises: i) a region of complementarity that specifically hybridizes with a target polynucleotide sequence (typically in the genomic DNA) ii) a second region that interacts with a Cas nuclease; and optionally iii) a transcriptional terminator. The first region, comprising a nucleotide sequence that is complementary to a target polynucleotide sequence, is also referred to herein as a "targeting region". The term "region" refers to a section/segment of a molecule, e.g., a contiguous stretch of nucleotides in an RNA. The targeting region is typically around 17-20 nucleotides in length. The targeting region of the gRNA preferably interacts with the target polynucleotide sequence through hydrogen bonding between complementary base pairs (i.e., paired bases). A targeting RNA according to the present disclosure is preferably a single RNA molecule (single RNA polynucleotide), which can be referred to as a "single-molecule targeting RNA," "single-guide RNA," or "sgRNA." Alternatively, a targeting RNA according to the present disclosure may comprise two RNA molecules.

An exemplary two-molecule gRNA ("tracrRNA/crRNA") typically comprises a crRNA ("CRISPR RNA" or "targeter-RNA" or "crRNA" or "crRNA repeat") and a corresponding tracrRNA ("trans-acting CRISPR RNA" or "activator-RNA" or "tracrRNA") molecule. A crRNA comprises both the targeting region (single stranded) and a stretch ("duplex-forming region") of nucleotides that forms one half of the dsRNA duplex of the Cas-binding region of the gRNA. A corresponding tracrRNA comprises a stretch of nucleotides (duplex-forming region) that forms the other half of the dsRNA duplex of the Cas-binding region of the gRNA. In other words, a stretch of nucleotides of a crRNA are complementary to and hybridize with a stretch of nucleotides of a tracrRNA to form the dsRNA duplex of the Cas-binding region of the gRNA. As such, each crRNA can be said to have a corresponding tracrRNA. The crRNA additionally provides the single stranded targeting region. Thus, a crRNA and a tracrRNA (as a corresponding pair) hybridize to form a gRNA.

A single-molecule targeting RNA ("single-guide RNA", "sgRNA") typically comprises a crRNA connected at its 3' end to the 5' end of a tracrRNA through a "loop" sequence (see, e.g., U.S. Patent Application No. US 20140068797 A). Similar to crRNA, sgRNA comprises a targeting region of complementarity to a target polynucleotide sequence, typically adjacent a second region that forms base-pair hydrogen bonds that form a secondary structure, typically a stem structure. The term "sgRNA" includes truncated single-guide RNAs (tru-sgRNAs) of approximately 17-18 nt (cf. Fu, Y. et. al. Nat Biotechnol. 2014 Mar;32(3):279-84). The term also encompasses functional miniature sgRNAs with expendable features removed, but that retain an essential and conserved module termed the "nexus" located in the portion of sgRNA that corresponds to tracrRNA (not crRNA) (cf. U.S. Patent Application No. 20140315985 and Briner AE et al. Mol Cell. 2014 Oct 23;56(2):333-9). The nexus is located immediately downstream of (i.e., located in the 3' direction from) the lower stem in Type II CRISPR-Cas9 systems. The term "sgRNA" also encompasses "deadRNAs" ("dRNAs") comprising shortened targeting regions of 11-15 nucleotides. Such dRNAs can be used to recruit catalytically active (wild-type) Cas nucleases to target DNA sequences for altering gene expression without inducing DSBs (cf. Dahlman JE et al. Nat Biotechnol. 2015 Nov;33(11):1159-61). sgRNA derivatives are also comprised by the term. Such derivatives typically include further moieties or entities conferring a new or additional functionality. Particularly, MS2 aptamers added to sgRNA tetraloop and/or stem-loop structures are capable of selectively recruiting effector proteins comprising said MS2 domains to the target DNA ("sgRNA-MS2") (cf. Konermann S et al. Nature. 2015 Jan 29; 517(7536): 583-588). Further modifications are also conceivable and envisaged herein.

The effector expression cassette of the inventive polynucleotide may thus preferably provide a gRNA, in particular a sgRNA as an effector. The term "gRNA" is inclusive, referring both to two-molecule gRNAs (crRNA/tracrRNA) and to single-molecule DNA-targeting RNAs (i.e., sgRNAs). sgRNAs are particularly preferred effectors. gRNAs and in particular sgRNAs are preferably capable of interacting with or binding to (1) a target polynucleotide (DNA) sequence and (2) a Cas nuclease. Thereby, gRNAs are preferably capable of recruiting the Cas nuclease to the target polynucleotide sequence (in particular a target DNA sequence followed by a protospacer adjacent motif (PAM)).

### Cas nucleases

Functional CRISPR/Cas systems further typically require the presence of a suitable Cas nuclease.

Cas (preferably Cas9) nucleases can be delivered to the host cells by a variety of means and methods. For instance, host cells stably expressing Cas (preferably Cas9) can be established or obtained from Cas transgenic animals, and the inventive polypeptide providing the gRNA can be added later. Alternatively, a polynucleotide sequence (in particular DNA or mRNA) encoding the Cas (preferably Cas9) nuclease can be delivered simultaneously with the inventive polynucleotide. The Cas (preferably Cas9) encoding polynucleotide sequence can be introduced (1) as part of the inventive polynucleotide, or (b) as part of a Cas (preferably Cas9) expression construct that is not part of the inventive polynucleotide and that is preferably delivered by transfection or viral transduction (e.g. using adeno-associated virus (AAV), retroviral or lentiviral vectors).

Thus, the inventive polynucleotide may encode in its effector expression cassette a wild-type Cas (preferably Cas9) nuclease as defined herein or a fragment, variant or derivative thereof as defined below, or may comprise a further expression cassette encoding said wild-type Cas (preferably Cas9) nuclease or a fragment, variant or derivative thereof as defined below. Alternatively, the wild-type Cas (preferably Cas9) nuclease as defined herein or a fragment, variant or derivative thereof may be encoded by a gene located on a different polynucleotide, preferably a naked DNA molecule or a vector (including a plasmid or a viral vector optionally selected from an adeno-associated virus (AAV), retroviral or lentiviral vector) or an mRNA molecule, which is introduced into the host cells prior to, simultaneously with, or subsequently to introduction of the inventive polynucleotide. Alternatively, the wild-type Cas (preferably Cas9) nuclease or a fragment, variant or derivative thereof as defined can either be directly isolated and purified from bacteria, or synthetically or recombinantly produced and directly delivered to the host cells in proteinaceous form.

Cas9 is an exemplary Type II CRISPR Cas protein. Cas9 is an endonuclease that can recruited by a gRNA to to cleave, site-specifically, target DNA using two distinct endonuclease domains (HNH and RuvC/RNase H-like domains) (see U.S. Published Patent Application No. 2014-0068797 and Jinek M., et al. Science. 2012 Aug 17;337(6096):816-21), one for each strand of the DNA's double helix. RuvC and HNH together produce double-stranded breaks (DSBs), and separately can produce single-stranded breaks. Typically each CRISPR-Cas9 system comprises a tracrRNA and a crRNA. However, this requirement can be bypassed by using an engineered sgRNA as described above, containing a designed hairpin that mimics the tracrRNA-crRNA complex (Jinek M., et al. Science. 2012 Aug 17;337(6096):816-21). Base-pairing between the sgRNA and the target DNA sequence typically causes double-stranded breaks (DSBs) due to the endonuclease activity of Cas9. Binding specificity is determined by both sgRNA-DNA base pairing and the protospacer adjacent motif (PAM) sequence adjacent juxtaposed to the DNA complementary region (Marraffini LA and Sontheimer E J. Nat Rev Genet. 2010 Mar;11(3):181-90).

The Cas nuclease is preferably *Streptococcus pyogenes* serotype 1 Cas9 (UniProt Acc. No. Q99ZW2, sequence version #1, entry version #90 last modified 2 November 2016) encoded by the cas9 gene or an ortholog or variant thereof. In this context, the term "ortholog" refers to either of two or more homologous gene sequences or the encoded proteins found in different species related by linear descent.

A large number of Cas9 orthologs are known in the art as well as their associated tracrRNA and crRNA components (cf. Fonfara I et al. Nucleic Acids Res. 2014 Feb;42(4):2577-90, Chylinski K et al. Nucleic Acids Res. 2014 Jun;42(10):6091-105, Esvelt, KM. et al. Nat Methods. 2013 Nov;10(11):1116-21). For instance, further preferred Cas9 nucleases include *Francisella tularensis subsp. novicida (strain U112)* Cas9 (UniProt Acc. No. A0Q5Y3), *Staphylococcus aureus* Cas9 (UniProt Acc. No. J7RUA5), *Streptococcus thermophiles* Cas9 (UniProt Acc. No. G3ECR1), *Actinomyces naeslundii* (strain ATCC 12104 / DSM 43013/JCM 8349 / NCTC 10301 / Howell 279) Cas9 (UniProt Acc. No. J3F2B0), *Streptococcus thermophilus* (strain ATCC BAA-491 / LMD-9) Cas9 (UniProt Acc. No. Q03JI6), *Neisseria meningitidis* serogroup C (strain 8013) Cas9 (UniProt Acc. No. C9X1G5), *Listeria innocua* serovar 6a (strain ATCC BAA-680 / CLIP 11262) Cas9 (UniProt Acc. No. Q927P4), *Streptococcus thermophilus* (strain ATCC BAA-491 / LMD-9) Cas9 (Q03LF7), *Streptococcus mutans* serotype c (strain ATCC 700610 / UA159) Cas9 (UniProt Acc. No. Q8DTE3), *Pasteurella multocida* (strain Pm70) Cas9 (UniProt Acc. No. Q9CLT2), *Neisseria meningitidis* serogroup A / serotype 4A (strain Z2491) Cas9 (UniProt Acc. No. A1IQ68), *Corynebacterium diphtheriae* (strain ATCC 700971 / NCTC 13129 / Biotype gravis) Cas9 (UniProt Acc. No. Q6NKI3), *Campylobacter jejuni* subsp. jejuni serotype O:2 (strain ATCC 700819 / NCTC 11168) Cas9 (UniProt Acc. No. Q0P897) and *Streptococcus thermophilus* (strain ATCC BAA-491 / LMD-9) Cas9 (UniProt Acc. No. Q03LF7).

The present invention further envisages the use of fragments, variants or derivatives of Cas nucleases, preferably Cas9 nucleases as defined above.

The term "variant" or "sequence variant" refers to proteins comprising an amino acid sequence that differs in at least one amino acid residue from a reference (or "parent") amino acid sequence of a reference (or "parent") protein. The variant can be derived from, isolated from, related to, based on or homologous to the parent sequence. Variants may comprise at least one (conservative or non-conservative) substitution, insertion, and/or deletion of amino acid residues as compared to their reference sequence. The term "variant" also includes isoforms. "Isoforms" are generally to be understood as proteins or polypeptides encoded by the same gene (or an allelic variant thereof located at the same position, or genetic locus, on a chromosome) but being different e.g. in terms of chemistry, activity, localization, interaction, conformation, and/or amino acid sequence as compared to "reference" proteins. Isoforms can emerge from genetic variations in the protein-encoding gene sequences that can result in substitutions, insertions and/or deletions of amino acid residues in an amino acid sequence. It will therefore be acknowledged that the terms "isoform" and "sequence variant" may overlap to some extent. Isoforms can also result from post-translational modifications (PTM) resulting, e.g., in covalent modifications of a given protein. Common post-translational modifications include glycosylation, phosphorylation, ubiquitinylation, S-nitrosylation, methylation, N-acetylation, lipidation, disulfide bond formation, sulfation, acylation, deamination etc., alternative splicing (by exon skipping, use of alternative donor or acceptor sites or intron retention) or proteolytic cleavage (e.g. of signal peptides).

Preferred Cas9 nuclease variants include Cas9D10A (Cong L. et al. Science. 2013 Feb 15;339(6121):819-23) exhibiting nickase (but no endonuclease) activity. Cas9D10A thus cleaves only one DNA strand, and does not activate NHEJ. Instead, when provided with a homologous repair template, DNA repairs are conducted via the high-fidelity HDR pathway only. Paired Cas9D10A complexes can also be used to target genomic DNA sequences of with a high target specificity, generating adjacent DNA nicks which can then undergo homology-directed repair.

Further preferred Cas9 nuclease variants include nuclease-deficient Cas9 (dCas9). Mutations H840A in the HNH domain and D10A in the RuvC domain inactivate endonuclease activity, but do not prevent DNA binding. Therefore, dCas9 can be used to sequence-specifically target any region of the genome without cleavage. Instead, dCas9 derivatives can be used to shuttle additional effector domains to a specific genomic locus, and thus serve either as a gene silencing or activation tool.

Variants of Cas9 nucleases are known in the art. U.S. Patent Application No. 20140273226, discusses the *S. pyogenes* Cas9 gene, the Cas9 protein, Cas9 variants including host-specific codon optimized Cas9 coding sequences and Cas9 fusion proteins. U.S. Patent Application 20140315985 teaches a large number of exemplary wild-type Cas9 polypeptides (e.g., SEQ ID NO: 1-256, SEQ ID NOS: 795-1346 of US Patent Application No. 20140273226) including the sequence of Cas9 from *S. pyogenes* (SEQ ID NO: 8 of US Patent Application No. 20140273226). Modifications and variants of Cas9 proteins are also discussed.

Preferred Cas9 nuclease variants include "deadCas9 (dCas9") and "Cas9 nickases".

The term "dCas9" refers to a nuclease-deactivated Cas9, also termed "catalytically inactive", "catalytically dead Cas9" or "dead Cas9." Such nucleases lack all or a portion of endonuclease activity and can therefore be used to regulate genes in an RNA-guided manner (Jinek M et al. Science. 2012 Aug 17;337(6096):816-21). dCas9 nucleases comprise mutations that inactivate Cas9 endonuclease activity, typically in both of the two catalytic residues (D10A in the RuvC-1 domain, and H840A in the HNH domain, numbered relative to *S. pyogenes* Cas9) of the gene encoding Cas9. It is understood that mutation of other catalytic residues to reduce activity of either or both of the nuclease domains can also be carried out by one skilled in the art. In doing so, dCas9 is unable to cleave dsDNA but retains the ability to target DNA. The Cas9 double mutant with changes at amino acid positions D10A and H840A completely inactivates both the nuclease and nickase activities. Targeting specificity is determined by complementary base-pairing of a gRNA to the genomic locus and the protospacer adjacent motif (PAM).

The term "Cas9 nickase" refers to Cas9 nuclease variants that do not retain the ability to introduce double-stranded breaks in a target nucleic acid sequence, but maintains the ability to bind to and introduce a single-stranded break at a target site. Such variants will typically include a mutation in one, but not both of the Cas9 endonuclease domains (HNH and RuvC). Thus, an amino acid mutation at position D10A or H840A in Cas9, numbered relative to the *S. pyogenes* Cas9 can result in the inactivation of the nuclease catalytic activity and convert Cas9 to a nickase.

The term "fragment" refers to proteins consisting of parts or portions of a reference (or parent) sequence of a reference (or parent) protein, said fragment being N-terminally, C-terminally and/or intrasequentially truncated compared to the amino acid sequence of the reference (or parent) protein. Such truncation may thus occur either on the amino acid level or on the nucleic acid level, respectively.

The term "derivative" refers to proteins that are derived from a reference (or "parent") protein and include modifications (preferably an additional entities or moieties) conferring a new (or additional) functionality.

Particularly, interesting derivatives for interrogating gene expression are dCas9 fusion proteins with transcriptional repression (e.g., Krüppel associated box (KRAB) or Enhanced Repressor Domain for TAL Effector (SID4X)) or activation (e.g., VP64 or p65) domains, dead Cas9 (dCas9) derivatives can thus be used to ferry functional domains to the sequence-specified sites in the genome - for example, for transcriptional activation (CRISPRa) or inhibition (CRISPRi) at gene promoters (or other gene regulatory elements). The sgRNA preferably guides the dCas9 derivative to the promoter (or other regulatory) regions of the intended target gene, and the repression or activation domains modify the transcription.

Preferred Cas (preferably Cas9) nuclease derivatives in the context of the present invention thus include wtCas (preferably Cas9), or variants or fragments thereof fused to effector domains (such as KRAB, SID4X, p65, VP64 or combinations thereof). Cas nuclease derivatives may further entities or moieties, such as suitable tags (e.g. HA tag or FLAG tag).

Cas (preferably Cas9) nuclease fragments, variants or derivatives disclosed herein are preferably functional variants, fragments or derivatives, i.e. preferably retain the ability of interacting with a sequence-specific gRNA (2) specifically binding to and/or interacting with a target polynucleotide sequence in the genomic DNA. Cas (preferably Cas9) nuclease fragments, variants or derivatives are preferably also capable of introducing DSBs or nicks into said the target genomic DNA sequence and/or recruiting or delivering effector domains to said genomic DNA sequence.

### Polynucleotides for CRISPR/Cas-mediated targeting of target genes

Depending on the desired application, polynucleotides for CRISPR/Cas-mediated targeting of target genes can be designed as follows:
For polynucleotides employed for CRISPR/Cas-mediated gene editing (e.g. knock-out, knock-in or HDR-mediated mutagenesis), the inventive polynucleotide may comprise: (a) a sensor expression cassette comprising (i) a sensor transcription control element operably linked to (ii) a unique identifier sequence providing an RNA oligonucleotide; and (b) an effector expression cassette comprising (i) a sequence providing a gRNA, preferably an sgRNA as defined herein (which is preferably not a dRNA), as an effector expression product, wherein said unique identifier sequence specifically identifies said polynucleotide. Optionally, the polynucleotide may further comprise (c) a sequence ("Cas-encoding sequence") providing a Cas nuclease , preferably a Cas9 nuclease, more preferably a wild-type Cas9 nuclease or a variant, fragment or derivative thereof having endonuclease or nickase activity. Alternatively, said Cas-encoding sequence may be provided as part of a distinct polynucleotide, optionally selected from naked DNA, a plasmid, or a viral vector. In either case, the Cas-encoding sequence is preferably operably linked to suitable regulatory elements driving its expression into a functional gene product.

For polynucleotides employed for CRISPR/Cas-mediated transcriptional activation or inhibition (CRISPRa or CRISPRi), the inventive polynucleotide may comprise: (a) a sensor expression cassette comprising (i) a sensor transcription control element operably linked to (ii) a unique identifier sequence providing an RNA oligonucleotide; and (b) an effector expression cassette comprising (i) a sequence providing a dRNA, as an effector expression product, wherein said unique identifier sequence specifically identifies said polynucleotide. Optionally, the polynucleotide may further comprise (c) a sequence ("Cas-encoding sequence") providing a Cas nuclease, preferably a Cas9 nuclease optionally selected from a wild-type Cas9 nuclease or a variant, fragment or derivative. Alternatively, said Cas-encoding sequence may be provided as part of a distinct polynucleotide, optionally selected from genomic DNA (in case of a Cas stable host cell line), naked DNA, a plasmid, a viral vector, or an mRNA. In either case, the Cas-encoding sequence is preferably operably linked to suitable regulatory elements driving its expression.

Alternatively, for polynucleotides employed for CRISPR/Cas-mediated transcriptional activation or inhibition (CRISPRa or CRISPRi), the inventive polynucleotide may comprise: (a) a sensor expression cassette comprising (i) a sensor transcription control element operably linked to (ii) a unique identifier sequence providing an RNA oligonucleotide; and (b) an effector expression cassette comprising (i) a sequence providing a gRNA, preferably an sgRNA and optionally an MS2-sgRNA, as an effector expression product, wherein said unique identifier sequence specifically identifies said polynucleotide. Optionally, the polynucleotide may further comprise (c) a sequence ("Cas-encoding sequence") providing a Cas nuclease, preferably a Cas9 nuclease optionally selected from a wild-type Cas9 nuclease or a variant, fragment or derivative thereof as defined herein. Preferred Cas9 variants and derivatives include dCas9 (particularly for use in combination with MS2-sgRNAs as effectors) or dCas9 fusion proteins comprising transcriptional repressors (such as KRAB or SID4X) or transcriptional activators (such as p65 or VP64) (particularly for use in combination with sgRNAs as effectors). Alternatively, said Cas-encoding sequence may be provided as part of a distinct polynucleotide, optionally selected from genomic DNA (in case of a Cas stable host cell line), naked DNA, a plasmid, or a viral vector, or an mRNA. In either case, the Cas-encoding sequence is preferably operably linked to suitable regulatory elements driving its expression into a functional gene product. Optionally (particularly in case MS2-sgRNAs are used as effectors), the inventive polynucleotide may contain at least one additional expression cassette comprising a sequence providing (or encoding) a transcription repressor (such as KRAB or SID4X) or activator (such as p65 or VP64), said sequence preferably being operably linked to suitable regulatory elements driving its expression.

The aforementioned approaches regarding different ways of employed CRISPR/Cas systems for gene editing or for modulating gene expression can also be combined with each other.

### Regulatory elements

The sensor expression cassette and/or the effector expression cassette of the inventive polynucleotide further preferably comprises at least one regulatory element (or regulatory sequence). Said regulatory element is typically operably linked to -and thereby capable of controlling expression of- the UIS (in the sensor expression cassette) or the sequence providing the effector (in the effector expression cassette). Regulatory elements may for instance act as transcription control elements and drive transcription initiation, elongation or transcription termination. The choice of suitable regulatory elements for controlling the expression of a polynucleotide sequence of interest (e.g. UIS or effector-providing sequence) depends *inter alia* on the host cell and/or vector used for expression.

### Sensor expression cassette

In the sensor expression cassette, UIS transcription initiation is typically controlled by the sensor. The amount of transcribed UIS thus reflects the sensor activity. The sensor expression cassette may further comprise a regulatory element controlling transcription termination. Such transcription termination sequences may be selected from transcription terminators (as described below) or polyadenylation signals. "Polyadenylation" refers to the post-transcriptional addition of a poly(A) tail to a transcribed RNA sequence. The poly(A) tail consists of multiple adenosine monophosphates. Polyadenylation signals or sequences can be selected from any polyadenylation signal known in the art and may for instance comprise the consensus NN(U/T)ANA consensus sequence (e.g. AAUAAA). Examples of polyadenylation signals include the SV40 early/late polyadenylation signal, the bovine growth hormone polyadenylation signal (bGHpA), or synthetic, minimal polyadenylation signals optionally derived therefrom.

UIS-derived RNA oligonucleotides may thus preferably comprise a poly(A) tail. For cDNA synthesis of said RNA oligonucleotides during detection, an oligo(dT) primer can be used, which only hybridizes with poly(A)-tails of RNA oligonucleotides expressed by the sensor. Thus preferably avoids the detection of RNA oligonucleotide contaminants emanating from unwanted "run-through" transcription of the UIS due to inefficient transcription termination in the juxtaposed effector expression cassette.

### Effector expression cassette

The effector expression cassette typically comprises at least one transcription initiation regulatory element operably linked to the sequence providing the effector. Said regulatory sequence drives the expression of the effector under appropriate conditions. Further, the effector expression cassette may comprise a transcription termination regulatory element. Therefore, when present in a suitable host cell, the sequence providing the effector is preferably effectively transcribed and/or expressed to yield the effector (e.g. shRNA or sgRNA).

Suitable regulatory elements are preferably selected based on their capability of controlling the expression of the respective effector in a desired manner. By way of example, high expression of the effector may be desirable in order to maximize its capability of interfering with target genes, and may be achieved by using strong RNA Pol II or RNA Pol III promoters. The tools and techniques provided herein particularly lend themselves for screening eukaryotic host cells. Hence, viral and eukaryotic regulatory elements are particularly envisaged for expression of the effectors.

### Promoters

Regulatory elements acting as transcription initiation elements thus include viral promoters, such as the cytomegalovirus immediate early (CMV-IE) promoter, the simian virus 40 (SV40) promoter, the Rous sarcoma virus long terminal repeat (RSV-LTR), the Moloney murine leukaemia virus (MoMLV) LTR, and other retroviral LTR promoters, and eukaryotic promoters such as the RNA polymerase II promoters, RNA polymerase III promoters nuclear RNA U1b promoter, ubiquitin C (UBC) promoter, elongation factor 1 alpha (EF1 α) promoter, human phosphoglycerate kinase 1 (PGK1) promoter. A number of tissue-specific promoters is also available and typically mediate tissue-specific expression (are thus particularly useful in host cells of the indicated kind) but some may also work in other cells or be modified to do so. Such promoters include liver promoters such as the apolipoprotein (apo) A-I promoter, apoE promoter, α1-antitrypsin (hAAT) promoter, transthyretin promoter, liver-enriched activator promoter, albumin promoter, Phosphoenolpyruvate carboxykinase (PEPCK) promoter; vascular promoters such as the PAI-1 promoter, the ICAM-2 promoter, the endoglin promoters, the vW promoter and the tyrosine kinase-1 (flt-1) promoter; muscle promoters such as the MCK promoter, SMC α-actin promoter, Myosin heavy-chain promoter, Myosin light-chain promoter; epithelium promoters such as the Cytokeratin 18 promoter, the CFTR promoter; Neuronal promoters such as the glial fibrillary acidic protein (GFAP) promoter, neuron-specific enolase (NSE) promoter, Synapsin 1 (Syn-1) promoter, Preproenkephalin, Dopamine beta-hydroxylase (dβH) promoters, the Prolactin promoter, Myelin basic protein promoter; or erythroid promoters such as the human ankyrin (ANK-1) promoter, α-spectrin, Globin promoters, HLA-DRα promoters, CD4 promoter, Dectin-2 promoter, or cancer-cell specific promoters including the α-fetoprotein (AFP) promoter, carcinoembryonic antigen (CEA) promoter, erbB2 promoter, mucin-1 (muc1) promoter, L-plastin (LP-P) promoter, α-lactalbumin (ALA) promoter, midkine (MK) promoter, cyclooxygenase-2 (COX-2) promoter, PSA/PMSA promoter, kallikrein-2 promoter, probasin (ARR2PB) promoter, tyrosinase promoter, hypoxic response elements (HRE) promoter, hTERT promoter, prolactin (PRL) promoter, osteocalcin 2 promoter, flt-1 promoter, flk1/KDR promoter, E-selectin promoter, endoglin promoter, ICAM-2 promoter, preproendothelin 1 (PPE-1) promoter. Functional variants of the aforementioned promoters are also envisaged.

As indicated above, the specific characteristics of the effector and the host cell envisaged for expression may be vital to the choice of a suitable promoter (or other regulatory elements). By way of example, the expression of shRNAs or other effectors may be regulated by a strong RNA polymerase III promoter (e.g. a U6 or H1 promoter). However, strong RNA Polymerase II promoters may also be used. In neurons, the synapsin-1 promoter (Syn1op) or neuron-specific enolase promoter s (NSEp) may be used for expression of shRNAs or other effectors.

### Terminators

The effector expression cassette may further comprise a regulatory element mediating transcription termination operably linked to the sequence encoding the effector. The choice of suitable transcription termination sequences *inter alia* depends on the RNA Polymerase effecting the transcription of the operably linked sequence.

Regulatory elements of interest in this respect include "(transcription) terminators" or "(transcription) terminator sequences". A "transcription terminator" is a polynucleotide sequence that indicates the end of a transcribed DNA sequence and mediates transcriptional termination by triggering release of the transcribed RNA from the transcriptional complex. Suitable terminator sequences are known in the art and include, inter alia, SV40, hGH, BGH, rbGlob, T6, T7, SP6, T3 or T4 terminator sequences.

### Target gene

The "target gene" is preferably a host cell gene which is not comprised by the polynucleotide of the invention. The target gene can be native or endogenous to the host cell or it can be exogenous (foreign). The target gene may comprise mutations (i.e. nucleotide insertions, deletions, additions, substitutions). Such mutations include naturally occurring mutations or mutations that have been intentionally introduced into the gene sequence. Target genes are preferably targeted by the effector expressed from by the polynucleotide of the invention. The effector is thus envisaged to interfere with said target gene. Target genes can be selected as targets or can be targeted randomly using random effector libraries. The present invention is not limited to any specific target gene, and the following classes of possible target genes are listed for illustrative purposes: developmental genes (e.g., adhesion molecules, cyclin kinase inhibitors, Writ family members, Pax family members, Winged helix family members, Hox family members, cytokines/lymphokines and their receptors, growth/differentiation factors and their receptors, neurotransmitters and their receptors); oncogenes (e.g., ABLI, BCLI, BCL2, BCL6, CBFA2, CBL, CSFIR, ERBA, ERBB, EBRB2, ETSI, ETS1, ETV6, FGR, FOS, FYN, HCR, HRAS, JUN, KRAS, LCK, LYN, MDM2, MLL, MYB, MYC, MYCLI, MYCN, NRAS, PIM 1, PML, RET, SRC, TALI, TCL3, and YES); tumor suppressor genes (e.g., APC, BRCA 1, BRCA2, MADH4, MCC, NF 1, NF2, RB 1, TP53, and WTI); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextrinases, DNA and RNA polymerases, galactosidases, glucanases, glucose oxidases, granule-bound starch synthases, GTPases, helicases, hemicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

### Library

In a further aspect, the invention relates to libraries of polynucleotides. Such libraries are particularly useful for the screening methods described herein.

The library comprises or consist at least two distinct polynucleotides; wherein each of the polynucleotides in the library comprises a sensor expression cassette comprising a sensor transcription control element, operably linked to a unique identifier sequence encoding an RNA oligonucleotide; and (b) an effector expression cassette comprising a sequence encoding an effector expression product. Each polynucleotide provides either a unique combination of unique identifier sequence and a distinct effector (such libraries are particularly suitable for use in methods for detecting at least one or more target gene(s) capable of regulating a given sensor transcription control element), or a unique combination of unique identifier sequence and a distinct sensor (such libraries are particularly suitable for use in methods for determining the effect of a given target gene on at least one or more sensor transcription control element(s).

In some libraries, each of the polynucleotides provides a distinct and unique combination of RNA oligonucleotide and effector expression product. Each of the polynucleotides may comprise the same sensor transcription control element. Thus, each effector of said library interferes with a different target gene. Such libraries are particularly useful for detecting target genes capable of regulating a particular sensor of interest (see section captioned "Screening Methods" below). In such libraries, each unique identifier sequence specifically identifies its polynucleotide and the effector provided by said polynucleotide

In some libraries, each of the polynucleotides provides a distinct and unique combination of RNA oligonucleotide and sensor. Each of the polynucleotides may provide the same effector expression product. Thus, each effector of said library preferably interferes with the same target gene. Such libraries are particularly useful for determining the effect of a particular target gene on different sensors (see section captioned "Screening Methods" below). In such libraries, each unique identifier sequence specifically identifies its polynucleotide and the sensor comprised by said polynucleotide.

Therefore, each of the polynucleotides preferably encodes a distinct and unique combination of (a) RNA oligonucleotide and effector or (b) RNA oligonucleotide and sensor. In both cases, the provided RNA oligonucleotides can be detected and quantified without the need for a separate analysis of each single host cell. The library can thus advantageously be utilized for pooled screening methods even in post-mitotic or other host cells that cannot easily be analyzed individually (e.g. by cell sorting).

The elements of the polynucleotides forming the library of the invention (i.e., sensor expression cassette and effector expression cassette) have been described elsewhere herein and are equally applicable to the polynucleotides of the library, *mutatis mutandis.*

The library may comprise DNA and/or RNA. For example, the inventive library may comprise DNA-based expression vectors (e.g., plasmids, viruses, etc.), each comprising an inventive polynucleotide providing a different and unique combination of an effector and RNA oligonucleotide (transcribed from the UIS). Preferably, all polynucleotides of the library comprise the same sensor. The library can thus be used for screening the effects of different effectors on the same sensor response.

### Plurality of host cells or vectors

Further described is a plurality of host cells or vectors comprising the library of polynucleotides. A plurality of host cells comprising a library according to the invention may be used to screen a plurality of effector expression products, each preferably capable of specifically interfering with a distinct target gene. Thereby, target genes regulating the activity of the sensor transcription control element (and thereby expression of the UIS) can be identified.

Host cells as described, and polynucleotides, vectors and libraries according to the present invention have been described elsewhere herein. The respective definitions are equally applicable to the plurality of host cells or vectors, *mutatis mutandis.*

### Vector

In a further aspect, the present invention provides a vector comprising the polynucleotide according to the invention.

As used herein, the term "vector" refers to any element capable of serving as a vehicle for transfer, expression and/or replication of a foreign polynucleotide sequence in a host cell. A vector can be integrated into the host cell genome or exist as an independent genetic element (e.g., episome, plasmid). A vector can exist as a single polynucleotide or as two or more separate polynucleotides. Vectors according to the present invention can be single copy vectors or multicopy vectors (indicating the number of copies of the vector typically maintained in the host cell). Vectors are typically recombinant, i.e. artificial molecules which do not occur in nature. The vector can generally be a DNA or RNA vector present in linear or in circular form, depending on type of vector or type of application. Some circular nucleic acid vectors can be intentionally linearized prior to delivery into a cell.

The term "vector" includes storage vectors, cloning vectors, transfer vectors, expression vectors and the like. A "storage vector" is a vector which allows the convenient storage of a nucleic acid molecule. A "cloning vector" (also referred to as a "shuttle vector") is typically a vector that contains a cloning site containing multiple restriction endonuclease target sequences, which may be used to incorporate nucleic acid molecules into the vector. A cloning vector may be, e.g., a plasmid vector or a bacteriophage vector. A "transfer vector" may be a vector which is suitable for transferring nucleic acid molecules into cells or organisms, for example, viral vectors. An "expression vector" is a vector that is capable of effecting the expression of an expression product -such as a nucleic acid molecule or typically a protein- provided by one or more polynucleotide sequences carried by the vector when it is present in the appropriate environment. As will be readily understood, the above definitions may overlap to a certain degree, e.g. some viral transfer vectors can also function as expression vectors.

The polynucleotide sequences can be inserted into a vector "backbone" using any of the methods known in the art in order to construct vectors. These methods may include in vitro recombinant DNA and synthetic techniques and genetic recombination. The resulting vector is referred to as a "recombinant" vector because it comprises novel combinations of nucleic acid sequences from the donor genome with the vector nucleic acid sequence. Recombinant vectors comprising the polynucleotide of the invention can be identified by known techniques including (a) nucleic acid hybridization; (b) presence of reporter gene functions; and (c) expression of inserted sequences. The vector may comprise additional regulatory elements in its "backbone", e.g. an origin of replication, enhancers, restriction sites, or regulatory elements as described elsewhere herein. The vector may therefore comprise regulatory elements directing its ligation and integration into the host cell genome etc. It will be understood that the specific design of the vector may depend on such factors as the choice of the host cell and the desired expression level, etc. Expression vectors capable of driving the expression of polynucleotide sequences comprised in said vector in a suitable host cell are particularly envisaged.

### Types of vectors

The vector can be selected from a viral or a non-viral vector. Non-viral vectors include linear or circular DNA molecules, plasmids (integrating or non-integrating), plasmid mini-circles, transposons, cosmids and artificial chromosomes. Such non-viral vectors can be complexed with polymers or lipids or can be provided in the form of "naked" polynucleotide molecules.

Viral vectors include bacteriophages, retroviruses, herpes viruses, lentiviruses, adenoviruses and adeno-associated viruses. Retroviruses, lentiviruses and adeno-associated viruses integrate into host cell DNA and therefore have potential for long term expression in the host. Retroviruses may be selected from murine leukaemia virus (MLV), mouse mammary tumour virus (MMTV), Rouse sarcoma virus (RSV), Moloney murine leukaemia virus (Mo MLV), Fujinami sarcoma virus (FuSV), Moloney murine sarcoma virus (Mo-MSV), Abelson murine leukaemia virus (A-MLV) and Avian erythroblastoma virus (AEV). Lentiviruses may be selected from human immunodeficiency virus (HIV), simian immunodeficiency virus (SIV), feline immunodeficiency virus (FIV), equine infectious anaemia virus (EIAV), caprine arthritis encephalitis virus (CAEV), bovine immunodeficiency virus (BIV) and Jembrana disease virus (JDV) based vectors. Adenoviruses may be selected from adenovirus type 5 first and second generation and gutless vectors. Adeno-associated viruses may be selected from all adeno-associated serotypes.

In the context of the present invention, adeno-associated viruses (AAV) are particularly envisaged as vectors. AAV are small viruses belonging to the *Parvoviridae* family with a non-enveloped icosahedral capsid of approximately 22 nm harboring a linear single-stranded DNA genome of approximately 4.7-kilobases (kb). The genome is structurally characterized by 145-bp inverted terminal repeats (ITRs) that flank two open reading frames (ORFs). AAV are placed in the genus *Dependovirus,* because productive infection by AAV occurs only in the presence of a helper virus, either adenovirus or herpesvirus. AAV vectors have been successfully employed as viral vectors. Use of AAV in as viral vectors in accordance with the present invention has several practical and experimental advantages. AAVs require less safety precautions and are structurally more robust than lentiviruses (Bouard D et al. Br J Pharmacol. 2009 May;157(2):153-65). AAVs produced with certain capsid serotypes (e.g. serotype 1 and 2) have a natural tropism for neuronal cells and do not trigger a cellular immune response (McCown TJ Curr Gene Ther. 2005 Jun;5(3):333-8). Thus, AAV are particularly useful for vectors and methods employed for screens in neuronal cells. Within the cell the AAV genome preferentially persists extrachromosomally (Nakai H et al. J Virol. 2001 Aug;75(15):6969-76) which reduces the risk of interference of genomic regulatory elements with effector and sensor expression and eliminates the possibility that coding or regulatory regions in the genome are destroyed due to integration (Moiani et al. J Clin Invest. 2012 May;122(5):1653-66).

### Preparation of vectors

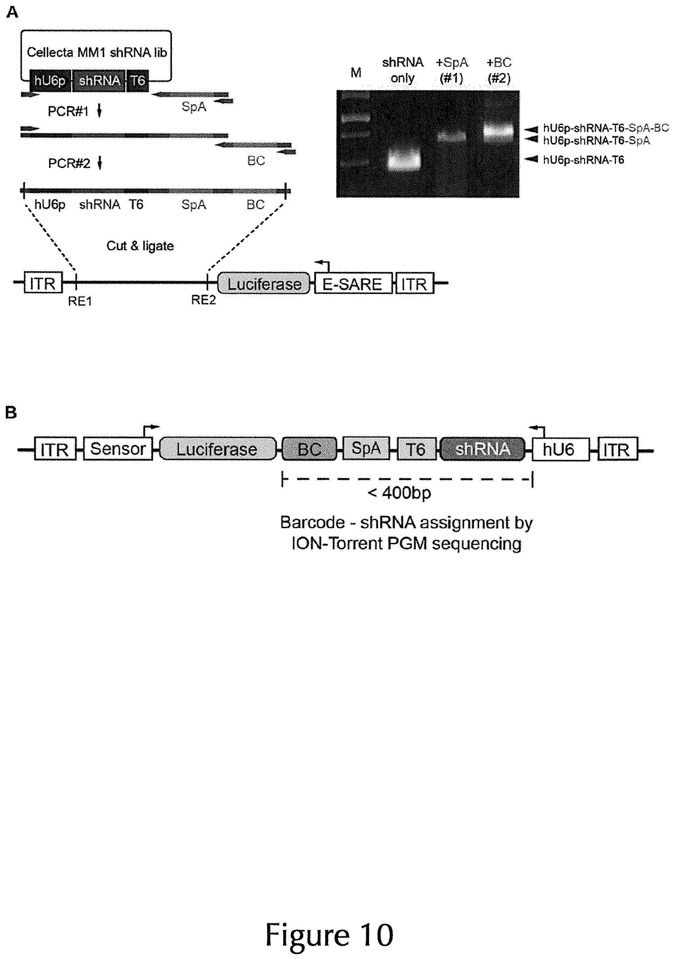

A cloning strategy for introducing a library of polynucleotides into the vectors of choice requires is depicted in Figure 10A and includes the following steps. Step (1): PCR amplification of the effector expression product (e.g. hU6p-shRNA) library cassette from the original plasmid library and simultaneous fusion of a minimal synthetic poly-adenylation signal (minimal SpA) (Levitt N et al. Genes Dev. 1989; 3, 1019-1025) that will ultimately form part of to the sensor expression cassette. Step (2): Fusion of the effector library PCR product with an oligonucleotide library containing random UIS and amplification by PCR. Step (3): Large-scale ligation of the effector-barcode library insert into a vector (e.g. AAV) backbone comprising the sensor. Step (4): Sequencing of the vector region spanning the effector and the barcode in order to assign barcode sequences to effectors/target genes (Figure 10B).

### Host cell

In a further aspect, the present invention provides a host cell comprising the vector and/or the polynucleotide according to the invention. As used herein, the term "host cell" refers to a cell into which a polynucleotide and/or a vector of the invention has been introduced and which preferably enables the expression of the polynucleotide. The term "host cell" does not only refer to the particular subject cell but to the progeny or potential progeny of such a cell. The term thus also includes cell cultures and cell lines. Specifically, the term "host cell" encompasses organotypic or acute slice culture or inducible pluripotent stem cell (iPSC) derived organoids or any other 3D culture system. A vector is considered to be present within or comprised by a host cell if it is introduced into the cell, enters the cell, or is inherited from a parental cell, regardless of whether it is subsequently modified or processed within the cell.

The skilled person will readily understand that suitable host cells include those that allow (a) the introduction of the respective polynucleotide(s) and/or vector(s) and (b) the expression of the polynucleotide sequences. Specifically, the host cells may constitutively express the effector expression cassette yielding functional effectors in sufficient quantities so as to allow for interference of said effectors with their target genes in a way that produces a detectable change of the sensor response. In this regard, a "detectable change" means an increase or decrease in the sensor response that is identifiable as a "hit" (i.e. gene implicated in sensor regulation) by RNA oligonucleotide quantification.

Host cells of interest include, without limitation primary cells, cell lines, immortalized cells, inducible pluripotent stem cells (iPSC)-derived host cells and transformed cells. The host cells can be normal (healthy) cells, or diseased host cells, including cells comprising a known genetic mutation. Mammalian cells are of particular interest and include, for example, human cells, murine cells, rodent cells, and primate cells. The host cells may be somatic cells or a germ cells. The host cell may be a post-mitotic (i.e., non-dividing) cell, or it may be capable of proliferating in vitro under suitable cell culture conditions. Specifically, host cells of the invention may be selected from a stem cell, which includes, for example, an embryonic stem cell, such as a murine embryonic stem cell. Advantageously, the polynucleotides, vectors and libraries are particularly suitable for evaluating gene function in post-mitotic (i.e. differentiated) host cells. Such host cells include, e.g., adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chrondrocytes, osteoblasts, osteoclasts, hepatocytes, and cells of the endocrine or exocrine glands.

Polynucleotides (typically comprised by vectors) can be introduced into suitable host cells using routine methods known in the art. Specifically, the polynucleotide or vector can be introduced into the host cell using any "transformation" method known in the art. The terms "transduction", "transfection" and "transformation" are used interchangeably herein to refer to the introduction of exogenous (foreign) polynucleotides or vectors into a host cell. Transformation may rely on any known method for the insertion of polynucleotides into a prokaryotic or eukaryotic host cell. The method is selected based on the type of host cell being transformed and may include, but is not limited to, viral infection, electroporation, heat shock, lipofection, calcium phosphate transfection, protoplast fusion and particle bombardment. "Transformed" cells include stably transformed cells in which the inserted nucleic acid molecule or vector is capable of replication either as an autonomously replicating episomal entity or as part of the host cell chromosome. Also included are cells that transiently express the polypeptide sequence(s) of interest. Host cells therefore comprise the polynucleotide and/or vector of the invention and/or the polynucleotide sequence(s) delivered by said nucleic acid molecule and/or vector. Further, host cells may comprise the expression products provided by the polypeptide described herein. That is, host cells may comprise one or more of the following: (1) at least one effector expression product provided by the polynucleotide present in the host cell and (2) at least one RNA oligonucleotide "barcode" provided by said polynucleotide. The host cell of the invention may further comprise at least one detectable marker as provided by said polynucleotide.

It is envisaged to choose the design of the polynucleotide of the invention based on its compatibility with the specific host cell in which it is expressed. That is, the effector expression cassette preferably comprises regulatory elements controlling the expression of effector that can be regulated by the host cell's molecule machinery. The sensor expression cassette preferably comprises a sensor that is responsive to a signaling pathway of interest known to be present within the chosen host cell.

### Screening methods

The invention further provides a variety of applications and uses of the inventive polynucleotides, host cells and libraries. Particularly, novel screening methods utilizing the inventive polynucleotides are provided, which aid in elucidating biological events and gene function. The screening methods provided herein are feasible, provide an easy readout and produce reliable results, and are thus excellently suited for high-throughput screenings. The methods provided herein may *inter alia* aid in unravelling the underlying causes of disease; detecting novel drug targets or elucidating the hitherto unknown mechanism of action of various genes or therapeutics.

### Screening methods detecting regulators of a sensor of interest

The screening methods of the present invention particularly lend themselves for pooled screenings of a variety of target genes (each specifically targeted by a different effector) in order to detect those target genes capable of controlling the sensor of interest. Thereby, regulators of the sensors of interest can be identified. As used herein, target genes capable of "regulating" or "controlling" a sensor of interest (also referred to as "regulators" of said sensor) are genes capable of affecting (i.e. maintaining, inducing, increasing, decreasing or inhibiting) the sensor response. Such regulators may affect the sensor response directly or indirectly, i.e. via interacting with the sensor or by interacting with another entity that is implicated upstream in a biological event ultimately funnelling into the sensor.

The sensor is typically chosen based on its ability of driving UIS expression in response to activation or inhibition of a pathway of interest. The event itself or the entities involved in said event do not have to be (and are typically not, or at least not fully) known. Thereby, the inventive screening methods advantageously allow for unravelling ramified biological networks funnelling into sensors of low selectivity. However, highly selective sensors that are responsive to a distinct and limited number of biological events may equally be used in the methods of the invention. Use of the same sensor for a pooled screening provides for the comparability of all sensor responses in the presence of a multitude of different effectors. The UIS operably linked to the sensor in each sensor expression cassette allows a direct quantitative readout of all sensor responses. Therefore, UIS expression is quantified by isolating and quantifying the unique RNA oligonucleotides transcribed from said UIS. Because each RNA oligonucleotide is unique and transcribed from one specific polynucleotide, each of said RNA oligonucleotides can be linked to a certain effector provided by the same polynucleotide. The present invention thus advantageously allows a direct readout of all pooled RNA oligonucleotides without the need for analysing each host cell individually. As indicated previously, preferred sensors are robust, exhibit a wide dynamic range upon pathway activation or inactivation, and a high signal-to-noise ratio. These characteristics facilitate the identification of "hits" (i.e. target genes regulating the sensor of interest). Further sensor features of interest may include cell-type specificity, responsiveness to triggers and low selectivity.

By way of example, the present inventors chose SARE enhancer as a sensor which is known to be activated upon synaptic stimulation. A library of vectors, each comprising an enhanced SARE (E-SARE) as a sensor operably linked to a UIS, and each providing a different shRNA as an effector (each shRNA targeting a different gene), was introduced into neuronal host cells. Genes targeted by shRNA effectors causing an altered sensor response (i.e. increased or decreased expression of the UIS) were identified as "hits" being involved in neuronal activation which ultimately regulates E-SARE activity. By employing the inventive screening method, the inventors were able to identify several positive and negative regulators of neuronal excitation (cf. the appended examples).

In a further aspect, the present invention thus provides a method for detecting at least one or more target gene(s) capable of regulating a given (i.e. pre-determined, pre-selected) sensor of interest comprising the following steps: (1) introducing into a plurality of host cells a library according to the invention, providing a test sample from said host cells, said test sample comprising RNA oligonucleotides, wherein each RNA oligonucleotide is transcribed from a distinct unique identifier sequence comprise by a polynucleotide of the library; (3) quantifying each RNA oligonucleotide in said test sample to determine a sensor response for each polynucleotide of the library; wherein a sensor response that deviates from the median sensor response of all polynucleotides of the library indicates that the effector expression product provided by a given polynucleotide interferes with a target gene capable of regulating the sensor transcription control element of interest. In other words, a sensor response for a given polynucleotide that deviates from the median sensor response of all polynucleotides indicates that the target gene is targeted or affected by said effector provided by said polynucleotide.

### Step (1): Introducing polynucleotides into the host cells

Step (1) of the inventive method requires introducing the inventive polynucleotides into appropriate host cells. Polynucleotides may be introduced in the form of vectors and in particular in the form of (vector) libraries. Host cells, polynucleotides, vectors, libraries and means and methods of introducing the same into the host cells are addressed elsewhere herein. The respective remarks and definitions are applicable to the inventive screening method, *mutatis mutandis.*

### Step (2): Obtaining a test sample and optionally a reference sample

Step (2) of the inventive method requires obtaining a test sample comprising RNA oligonucleotides from said host cells. Means and methods for extracting the respective RNAs are known in the art. Suitable ways of obtaining the RNA oligonucleotides from the sample are described in the appended examples.

### Trigger

The screening method may further comprise prior to step (2) a step (1a) of providing a trigger to the host cells, said trigger being capable of regulating the sensor response. In particular, a trigger may be employed to induce a desired sensor response. Triggers are typically extracellular or intracellular stimuli which induce or augment a desired sensor response (i.e. activation or inactivation). Suitable stimuli are exemplified in the section captioned "Test conditions and reference conditions" and are equally applicable to the trigger, *mutatis mutandis.* Thus, in host cells which comprise polynucleotides providing an effector which has no effect, i.e. which does not interfere with a "regulator", i.e. a target gene capable of regulating the sensor of interest, the trigger will typically induce the desired sensor response. However, in host cells which comprise polynucleotides providing an effector which has an effect, i.e. interferes with such a "regulator" of the sensor of interest, the trigger may not be able to induce the desired sensor response, because the "regulator" that would usually effect (directly or indirectly) sensor activation in response to said trigger.

In some screening methods according to the invention, the trigger is equivalent to the test condition or reference condition. In other screening methods according to the invention, the trigger is different from the test condition and reference condition and may be added to both the test population and the reference population of evaluated host cells as described below. In such cases, the trigger may be added before or after the host cells have been or are subjected to the respective conditions (typically, the trigger is added thereafter).

### Test population

It will be understood that the sensor of interest preferably produces a sensor response (quantifiably via detecting the RNA oligonucleotides expressed from the UIS) in response to a defined test condition. By way of example, the test condition can be the presence of a disease-causing mutation in said host cells, or any other test condition described below. The term "test population" thus refers to a plurality of host cells comprising a plurality of inventive polynucleotides which are (or have been) subjected to said test condition. Said host cells yield the test sample that is evaluated with regard to the sensor responses under test conditions, which are individually quantifiable via detecting and identifying the unique RNA oligonucleotides transcribed from the polynucleotides of the library. Preferably, the host cells of the test population are (or have been) subjected to a test condition prior to step (3) of the inventive method. The majority of host cells typically comprises a polynucleotide which comprises an effector which does not have an effect on the sensor response, i.e. does not target a regulator of the sensor of interest and is thus "ineffective". However, some host cells may comprise a polynucleotide providing an effector which has an effect on the sensor response, i.e. which targets a regulator of the sensor of interest. In such host cells, the test sensor response deviates from the median sensor response of all host cells encoding "ineffective" effectors.

Test conditions will be selected depending on the sensor of interest. Suitable test conditions (and reference conditions) are described below.

### Reference population

The inventive screening method may further include the provision of a reference sample. A reference sample may offer various benefits: (1) absolute abundances of individual polynucleotides may vary within the library. The sensor responses (quantifiable via UIS expression) in a reference sample may thus be used to normalize the sensor responses (quantifiable via UIS expression) in a test sample and (2) a reference sample can serve to compare the sensor responses under different test conditions.

The inventive screening method may therefore further comprise in step (2) a step of obtaining a reference sample from said host cells comprising RNA oligonucleotides provided by said plurality of polynucleotides, wherein each RNA oligonucleotide specifically identifies a polynucleotide. The term "reference population" refers to a plurality of host cells comprising a plurality of inventive polynucleotides which are evaluated using the screening method described herein and are preferably not (or have preferably not been) subjected to test conditions. Said host cells yield the reference sample that is evaluated with regard to the sensor responses under reference conditions, which are individually quantifiable via detecting and identifying the unique RNA oligonucleotides transcribed from the polynucleotides of the library.

Test population and reference population may be derived from the same pool of host cells, and be separated before the test population is subjected to test conditions.

The reference population is not subjected to test conditions but is usually treated differently as compared to the test population. The inventive screening method may thus comprise in step (2a) a step of subjecting the host cells in the reference population to a reference condition.

The reference condition will typically be selected depending on the sensor and the selected test condition. By way of example, in case the test condition is the presence of a disease-causing mutation in the host cells of the test population, the reference condition may be the absence of said mutation in the host cells of the reference sample. Or, in case the test condition is the addition of a stimulus to the host cells of the test population, the reference condition may be the absence of stimulus in the host cells of the reference sample. The inventive method thus allows comparing sensor responses under test conditions (e.g. mutated or stimulated) *versus* reference conditions (e.g. non-mutated or non-stimulated). The host cells of the test and reference population may or may not additionally be subjected to a trigger capable of inducing the sensor response. By way of example, in case the inventive screening methods compares sensor responses in mutated versus non-mutated neuronal cells, and the sensor is known to be activated upon synaptic stimulation, the trigger may be a stimulating agent that is capable of activating the sensor in cells that do not comprise the disease-causing mutation and have not been modified by introducing a polynucleotide of the invention. In this case, the inventive method allows evaluating the response of mutated neuronal host cells as compared to non-mutated neuronal host cells to said trigger in the presence of a multitude of effectors (potentially) interfering with target genes implicated in a variety of biological events. Or, in case the inventive screening method compares sensor responses in stimulated versus non-stimulated neuronal cells, an additional trigger may not be necessary.

Suitable reference conditions (and test conditions) are described below. The reference condition is different from the test condition. When the test condition is the provision of a trigger, the reference condition may be the absence of a trigger, and *vice versa.* When a test condition is the presence of a disease, the reference condition may be the absence of said disease, and *vice versa*. When a test condition is the presence of a genetic modification, the reference condition may be the absence of said genetic modification, and *vice versa*.

As indicated previously, the reference sample may serve to normalize the sensor responses obtained for the test sample. The inventive screening method may thus comprise a step (4) calculating a "sensor response ratio" for each polynucleotide by dividing the quantity of each RNA oligonucleotide in the sample by the quantity of the corresponding RNA oligonucleotide in the reference sample, wherein a "sensor response ratio" that deviates from the median "sensor response ratio" of all polynucleotides is indicative of an effect of the target gene targeted by the effector provided by said polynucleotide.

### Test conditions and reference conditions

In accordance with the above, the test conditions and/or reference conditions may be selected from an extracellular or intracellular stimulus; optionally selected from a chemical or physical stimulus or a combination thereof; an intrinsic modification optionally selected from a genome or proteome or epigenome modification; or a combination thereof.

Extracellular or intracellular stimuli are detectable changes in the internal or external environment of a host cell. Stimuli may be known or suspected to elicit a particular sensor response (and can thus serve as trigger) or can be selected irrespective of any known or suspected trigger function with regard to the sensor of interest. For instance, when evaluating responses of a sensor that is responsive to synaptic stimulation, the stimulus serving as a trigger and as a test condition may be a chemical or small organic molecule that is capable of inducing synaptic stimulation (such as Bicuculline/BIC or brain-derived neurotrophic factor, BDNF).

The term "extracellular stimulus" refers to a stimulus that is exerted on or occurs in the external environment of the host cell, e.g. by changing the chemical or physical parameters in the host cell's environment. Extracellular stimuli are those applied to the external environment of the host cells (e.g. addition of a chemical or provision of a physical stimulus to the external environment of a host cell), whereas intracellular stimuli are applied directly to the intracellular compartments (e.g. by introducing electrodes into the cytoplasm). Stimuli that can be provided in the screening methods of the invention include, without limitation, chemical stimuli including small organic molecules, drugs, drug candidates, growth factors, cytokines, chemokines, hormones, receptor agonists, receptor antagonists, antigens, nucleic acids, pathogens, and physical stimuli including temperature stimuli, electrical stimuli, mechanical stimuli (e.g. ultrasound, pressure), radiation stimuli (including light, X-ray, alpha-, beta- and gamma-radiation).

Intracellular stimuli include the introduction of compounds including nucleic acids, proteins, peptides, carbohydrates, lipids, particles, ions, small organic or inorganic molecules, intracellular pathogens, or intracellular physical stimuli (e.g. electrical stimuli) and the like into the host cell.

Intrinsic modifications include modifications to the genome or proteome that are present in the evaluated host cell but not in comparable reference (host) cells. Intrinsic modifications also encompass diseases. The modifications may be initially present in the host cell, e.g. in case of a host cell derived from a patient suffering from a disease caused by a genetic mutation, or may be introduced into the host cell, e.g. in case of mutations being introduced into the host cell genome using genetic engineering techniques. Host cells may be manipulated in order to introduce modifications using routine methods in the art (cf. Sambrook J et al. 2012. Molecular Cloning: A Laboratory Manual (4th Edition)).

By way of example, it may be desired to elucidate the function of various target genes in sensor regulation in the presence or absence of disease known to be caused by or associated with a known genetic modification. Host cells may be derived from a patient suffering from said disease (e.g. by isolating said cells for primary cell culture or by generating induced pluripotent stem cells (iPSCs) bearing said genetic modification). The presence of said genetic modification may thus be the test condition. As a reference sample, cells can be obtained from a healthy subject which does not suffer from said disease. The absence of said genetic modification may thus be the reference condition. The sensor (in-)activity in the test sample and the reference sample in response to a trigger can be quantified and analysed. Deviations between the sensor responses obtained for the test sample as compared to the reference sample provide information as to how the genetic modification affects the regulation of the sensor in response to the action of various target genes.

The inventive screening method may thus comprise a step of introducing an intrinsic modification into the host cell or selecting a host cell for the presence of an intrinsic modification. Said step is preferably accomplished before introducing the inventive polynucleotides into the host cells.

In case intrinsic modifications serve as test and/or reference conditions, the test population and the reference population may be derived from the same cell pool and separated before introducing the respective intrinsic modifications into the host cells of the test population. That is, test population and separate population are typically separated before the inventive polynucleotides are introduced into both populations. In case the intrinsic modification is a disease and the test population is a population of host cells derived from a patient suffering from said disease, the reference population may be derived from a healthy subject not suffering from said disease. In this case, test population and reference population are not derived from the same pool of host cells.

As indicated previously, intrinsic modifications encompass intrinsic modifications to the genome, proteome or metabolome of a host cell.

Genome or gene modifications encompass modifications and mutations that are naturally occurring or artificially induced. The term includes "small-scale" modifications affecting the polynucleotide sequence of a locus of interest and "large-scale" modifications affecting the chromosomal structure. Gene or genome modifications envisaged herein thus include nucleotide substitutions (transitions or transversions), point mutations (including silent mutations, missense mutations or nonsense mutations and single nucleotide polymorphisms), insertions, deletions, gene duplications, gene amplifications, frame shift mutations, repeat expansions, chromosomal deletions, chromosomal translocations, interstitial deletions and chromosomal inversions as well as allele loss. Modifications occurring in and/or affecting coding regions of the genome may lead to a loss-of-function, i.e. a decrease or loss of biological function of the affected gene product, or gain-of-function, i.e. an increase in biological function or a different (abnormal) biological function of the affected gene product.

Epigenome modifications include modifications of the nucleotides, histone proteins (histone modifications), and chromatin architecture. Nucleotide modifications envisaged herein include all chemical modifications of the DNA nucleotides, including phosphate, sugar and base modifications. Particularly envisaged herein are alterations in the pattern of DNA methylation which occurs at the 5' position of the cytosine ring within CpG dinucleotides via the addition of a methyl group to create a 5-methylcytosine (m5C). Distinctive distribution patterns of CpG methylation are believed critical for the control of gene silencing and chromosomal stability, and aberrant patterns of DNA methylation are thought to influence many aspects of disease processes. Histone modifications are post-translational modifications (PTM) are thought to be critical for regulating chromatin structure and function, which can in turn affect many DNA-related processes, such as transcription, recombination, DNA repair and replication, and chromosomal organization. Histone modifications envisaged herein include chemical modifications of the amino terminal ends of the core histone, including acetylation, methylation, phosphorylation, ubiquitinylation, sumoylation, etc. Modifications of chromatin architecture include shifting chromatin domains from an "open" to a "closed" state or *vice versa.*

Proteome modifications include posttranslational modifications such as phosphorylation, glycosylation, ubiquitinylation, S-nitrosylation, methylation, N-acetylation, lipidation, sumoylation and the like, or modification to processes affecting proteolysis, protein trafficking, folding, or secretion.

### Step (3) RNA quantification and Sensor response

In step (3) of the inventive screening method, each RNA oligonucleotide in the test sample (and optionally the reference sample) is quantified in order to determine a sensor response for each polynucleotide of the library. The sensor response can be quantified for each polynucleotide by determining the amount of RNA oligonucleotides expressed from the UIS operably linked to the sensor as described below. As used herein, the term "sensor response" refers to the activity or inhibition of previous activity of the sensor in terms of expression of the operably linked UIS, optionally induced by a trigger. In this regard, "activity" of the sensor refers to sensor active states measurable by steady or induced or increased UIS expression, whereas "inactivity" of the sensor refers to sensor inactive states measurable by decreased or abrogated UIS expression.

It is envisaged that the majority of effectors employed in the screening method have no effect, i.e. do not alter (induce, increase, decrease or abolish) the sensor response. Thus, a sensor response that deviates from the median sensor response of all polynucleotides of a library is indicative of an effect of the target gene targeted by the effector provided by said polynucleotide (i.e. indicative of an effector that is "effective" and targets a "regulator" of the sensor of interest. Such a deviating sensor response therefore indicates that the affected target gene (positively or negatively) regulates the sensor of interest. Such a target gene is classified as a "hit" or "regulator" herein.

### Readout

The UIS is placed under the control of the sensor present in the polynucleotides of the invention. Expression of the UIS is thus preferably controlled by the sensor operably linked thereto and is therefore indicative of the sensor response: an altered amount of RNA oligonucleotides expressed from the UIS indicates an altered sensor response and thus preferably interference of the effector with a regulator (i.e. target gene implicated in sensor control). As each RNA oligonucleotide is expressed from a UIS that is comprised by the a polynucleotide providing a distinct effector, altered amounts of each RNA oligonucleotide can be directly linked to the respective effectors provided by the same polynucleotide that harbors the UIS. Thus, an altered amount of a certain RNA oligonucleotide indicates that the effector provided by the same polynucleotide as the oligonucleotide targets a gene implicated in sensor control.

For quantification and identification of the expressed RNA oligonucleotides in a sample obtained from the host cells, any sequencing method known in the art can be employed. Typically, RNA will be transcribed into cDNA and subjected to Next Generation Sequencing (NGS) using appropriate protocols and commercially available devices as described in the appended examples and reviewed, inter alia, by ten Bosch and Grody. J Mol Diagn. 2008 Nov; 10(6): 484-492. Quantification of the RNA oligonucleotides in step (3) of the inventive method may comprise one or more of the following steps: (i) purification of the RNA oligonucleotides; (ii) reverse transcription of the RNA oligonucleotides into cDNA; (iii) amplification of the RNA oligonucleotides by PCR; and/or (iv) sequencing of the RNA oligonucleotides. Typically, in a first step, the total RNA is purified (or isolated) from host cell lysates, e.g. using a commercially available RNA isolation kit (e.g. Direct-zol™ RNA MiniPrep available from Zymo Research, Cat. # R2050) according to the manufacturer's instructions. Any DNA is usually removed, preferably by DNase digestion. Subsequently, the obtained RNA may be reverse transcribed into cDNA and amplified by polymerase chain reaction (PCR), e.g. as described in the appended Examples. The resulting PCR products are then subjected to next generation sequencing (NGS), e.g. using the Ion Torrent™ Next-Generation Sequencing Technology (ThermoFisher Scientific).

Next, the "sensor response ratio" is determined for each polynucleotide by dividing the amount of the RNA oligonucleotide obtained from the test sample by the amount of the corresponding RNA oligonucleotides obtained from the reference sample. For instance, an RNA oligonucleotide X is transcribed from a polynucleotide providing an effector Y. In a screening method according to the invention, the amount of said RNA oligonucleotide X in a test sample (A(X)) and the amount of said RNA oligonucleotide X in a reference sample (A(X)') are determined. The "sensor response ratio" is calculated as A(X)/A(X)'. A relative increase or decrease of the "sensor response ratio" (as determined for a given polynucleotide) as compared to the median "sensor response ratio" of all polynucleotides indicates activation or inactivation of the sensor. As each RNA oligonucleotide can be directly mapped to a distinct polynucleotide of interest (and hence to a particular effector provided by the same polynucleotide), the inventive screening methods allow for directly identifying regulatory relations between target genes and sensors of interest. In other words, target genes capable of controlling a given sensor of interest (in case a plurality of effectors is evaluated) or sensors targeted by a given gene of interest (in case a plurality of sensors is evaluated) can be directly identified via comparing the "sensor response ratio" for each polynucleotide with the median "sensor response ratio" of all polynucleotides. For instance, if the sensor response ratio A(X)/A(X)' is increased above or decreased below the median sensor response ratio determined for all polynucleotides, the gene targeted by effector Y is identified as a regulator of the sensor of interest.

### Data analysis

Once the RNA oligonucleotides have been obtained from the test sample and reference sample and have been identified and quantified, analysis of the respective data allows the identification of "hits". It is assumed that the majority of effectors do not interfere with target genes implicated in sensor control. Thus, "hits" can be identified by comparing the sensor response for each polynucleotide to the median sensor response of all polynucleotides. Sensor responses deviating from the median sensor response indicate a "hit".

By way of example, RNAi-inducing agents (such as siRNA or shRNA) or the CRISPR/Cas system may be employed as effector expression products in a polynucleotide library according to the invention. Expression of the siRNAs or shRNAs or the CRISPR/Cas system can be used to effect knockdown of various target genes. In this context, a sensor response that is decreased below the median sensor response of the polynucleotide library is envisaged to indicate that the target gene (which is identifiable via the RNA barcode expressed from the UIS) is a positive regulator of the sensor, so that knockdown of said "activating" target gene results in a decreased sensor response. A sensor response that is increased above the median sensor response of the library is envisaged to indicate that the target gene is a negative regulator of the sensor, so that knockdown of said "inhibiting" target gene results in an increased sensor response. In this regard, the expressions "activating" and "inhibiting" in all their grammatical forms do not imply that the invention is restricted to evaluating target genes capable of directly interacting with (e.g. binding to) the sensor transcription control element. The inventive means and methods aid in identifying target genes that are involved at any level of biological cascades which ultimately result in a regulation of the sensor response. Means and methods for identifying "hits" are described in the appended examples (Zhang SJ et al. PLoS Genet. 2009 Aug;5(8):e1000604 and Parnas O et al. Cell. 2015 Jul 30;162(3):675-86. Briefly, an enhanced Z-score analysis can be utilized. An advantage of the enhanced Z-score analysis is its robustness against outliers (which in this case are the hits). It enables the identification of positive and negative regulators. Another approach utilizes the Bioconductor package DESeq2 as described by Love MI et al. Genome Biol. 2014;15(12):550.

By using the above-described methods, the skilled person can easily identify positive and negative regulators of a sensor of interest. The inventive screening method described herein thus lends itself for detecting target genes capable of regulating a sensor of interest, thereby elucidating biological events funneling into said sensor.

In view of the above, the inventive screening method for detecting at least one or more target gene(s) capable of regulating a given sensor transcription control element may involve the following steps:
(1) introducing into plurality of host cells a library according to the invention;
(2a) subjecting part of host cells to a test condition;
(2a') subjecting part of host cells to a reference condition;
(2) providing a test sample from said host cells of step (2a); said test sample comprising RNA oligonucleotides, wherein each RNA oligonucleotide is transcribed from a unique identifier sequence which specifically identifies its polynucleotide;
(2') providing a reference sample from said host cells of step (2a'); said reference sample comprising RNA oligonucleotides, wherein each RNA oligonucleotide is transcribed from a unique identifier sequence which specifically identifies its polynucleotide;
(3) quantifying each RNA oligonucleotide in said test sample to determine a sensor response for each polynucleotide;
(3') quantifying each RNA oligonucleotide in said reference sample to determine a sensor response for each polynucleotide;
wherein a sensor response that deviates from the median sensor response of all polynucleotides indicates that the effector expression product provided by a given polynucleotide interferes with a target gene capable of regulating the sensor transcription control element of interest.

### Screening methods for evaluating the gene function

Polynucleotides, vectors, libraries and host cells are not only useful for detecting target genes capable of regulating a sensor of interest. Instead of using a plurality of polynucleotides providing a plurality of different effectors (each interfering with a different target gene), variability among different polynucleotides can be introduced by employing a different sensor of interest in each sensor expression cassette. The UIS (and RNA oligonucleotide transcribed therefrom) specifically identify the polynucleotide comprising the UIS -and the particular sensor present in the respective sensor expression cassette of said polynucleotide. Polynucleotide libraries employed in such screening methods therefore typically provide the same effector interfering with the same target gene, but comprise different sensors. Screening methods employing such polynucleotide libraries are therefore particularly useful for determining the effect of a certain target gene on a plurality of sensors, or, in other words, for elucidating gene function of a particular target gene of interest.

In a further aspect, the present invention thus relates to a method for determining the effect of a given target gene on at least one or more sensor transcription control element(s) comprising the following steps:
(1) introducing into a plurality of host cells a library as disclosed herein;
(1') introducing into a plurality of host cells a corresponding library as disclosed herein; wherein said effector expression product provided by the polynucleotides of said library are not capable of interfering with a target gene;
(2) providing a test sample from said host cells of step (1);
(2') providing a reference sample from said host cells of step (1')
   said test sample and said reference sample comprising RNA oligonucleotides transcribed from said polynucleotides of said library, wherein each RNA oligonucleotide is transcribed from a unique identifier sequence which specifically identifies its polynucleotide;
(3) quantifying each RNA oligonucleotide in said test sample to determine a test sample "sensor response" for each polynucleotide of the library;
(3') quantifying each RNA oligonucleotide in said reference sample to determine a reference sample "sensor response" for each polynucleotide of the library;
wherein a "sensor response" in a test sample for any given polynucleotide that deviates from the corresponding "sensor response" in the reference sample is indicative of an effect of the target gene on the sensor transcription control element comprised by said polynucleotide.

This further screening method is based on the same principle and shares many aspects with the first screening method described above. Unless denoted otherwise, the explanations and definitions provided in the context of said first screening method therefore also apply to the screening method for evaluating gene function, *mutatis mutandis.* The methods differ from one another in that in the first screening method evaluates the effect of a plurality of target genes on one sensor of interest in order to elucidate the biological event funneling into said sensor of interest; whereas the second screening method evaluates the effect of a one particular target gene on a plurality of sensors of interest in order to elucidate gene functions of said target gene.

The first screening method utilizes the same sensor for all polynucleotides, "hits" are typically identified by comparing the sensor response (ratio) for each polynucleotide to the median sensor response (ratio).

The second screening method utilizes different sensors, "hits" are typically identified by comparing the sensor response for a particular polynucleotide in a test sample to the respective sensor response in a reference sample. Specifically, in the second screening method, a "test" library of polynucleotides is introduced into a plurality of host cells that will yield the test sample. The "test" library of polynucleotides comprises polynucleotides as described herein, wherein each polynucleotide preferably provides the same effector (targeting the same target gene) and harbors a different sensor. Each polynucleotide (and thus each sensor) is "tagged" or "barcoded" with a different UIS. Further, a "reference" library of polynucleotides is introduced into a plurality of host cells that will yield the reference sample. Said "reference" library of polynucleotides corresponds to the "test" library insofar as each polynucleotide preferably provides the same effector (targeting the same target gene) and harbors a different sensor "tagged" or "barcoded" with a different UIS. The polynucleotides (and the respective sensors, UIS and unique combinations thereof) of the "test" and "reference" library are identical except that each polynucleotide of the "reference" library encodes a "functionless" effector that is not capable of interfering with any target gene. For instance, such effectors may be negative control siRNAs, shRNAs or sgRNAs with sequences that do not target any gene product.

Thus, the sensor response for a given polynucleotide from the test sample (determined as the amount of RNA oligonucleotides transcribed from the UIS harbored by said polynucleotide) can be compared with the sensor response for the corresponding polynucleotide from the reference sample (determined as the amount of RNA oligonucleotides transcribed from the UIS harbored by said polynucleotide). The sensor comprised by the polynucleotide of the reference sample is not affected by the "functionless" effector encoded by the same polynucleotide. Thus, when the sensor response of the corresponding polynucleotide of the test sample deviates from the sensor response determined for the respective polynucleotide in the reference sample (i.e. is increased or decreased as compared to said sensor response), the evaluated target gene (targeted by the "functional" effector in the test sample) is identified as a regulator of the respective sensor.

The test sample and reference sample may further be subjected to test conditions and reference conditions as described in the context of the first screening method above. Test conditions and/or reference conditions may be selected an extracellular or intracellular stimulus; optionally selected from a chemical or physical stimulus or a combination thereof; or an intrinsic modification optionally selected from a genome, proteome or epigenome modification; or a combination thereof, as described above.

The second screening method may further comprise a step of providing a trigger to the host cells, said trigger being capable of eliciting a sensor response. Suitable triggers are described above.

RNA quantification and sensor response are evaluated as described above. Particularly, step (4) of said method may comprise the steps of (i) purification of the RNA oligonucleotides; (ii) reverse transcription of the RNA oligonucleotides into cDNA; (iii) amplification of the RNA oligonucleotides by PCR; and/or (iv) sequencing of the RNA oligonucleotides.

### Effector expression products employed in the inventive methods

In the screening methods, according to the present invention, any of the effector expression products described in the context of the inventive polynucleotide can be employed.

Preferred effector expression products include, *inter alia,* RNAi-inducing agents, preferably shRNAs or siRNAs, and gRNAs.

In this context, gRNAs are particularly preferred effectors, that are used in combination with Cas nucleases. As indicated above, use of the CRISPR/Cas system requires the introduction of an sgRNA and a Cas nuclease into the host cells. The Cas (preferably Cas9) nuclease (or a fragment, variant or derivative thereof as defined herein above) can be introduced into the host cells by delivering a library of inventive polynucleotides that comprise a sequence encoding said Cas (preferably Cas9) nuclease (or a fragment, variant or derivative thereof as defined herein above) to the host cells. Alternatively, the Cas (preferably Cas9) nuclease (or a fragment, variant or derivative thereof as defined herein above) can be introduced into the host cells by introducing into said host cells a polynucleotide encoding said Cas (preferably Cas9) nuclease (or a fragment, variant or derivative thereof as defined herein above). Such polynucleotides are also referred to as "Cas-encoding polynucleotides" herein. The Cas-encoding polynucleotide is preferably a vector, in particular an expression vector capable of effecting the expression of said Cas (preferably Cas9) nuclease (or a fragment, variant or derivative thereof as defined herein above). Specifically, the expression vector may be a plasmid, or a viral vector (such as an adeno-associated virus (AAV), a lentiviral or retroviral vector). Suitable methods for delivering the inventive polynucleotides are described herein above and equally applicable to Cas-encoding polynucleotides, *mutatis mutandis.* The Cas-encoding polynucleotide may be introduced into the host cells prior to, simultaneously with or subsequently to the inventive polynucleotide. Preferably, the Cas-encoding polynucleotide is introduced simultaneously with the inventive polynucleotide. Alternatively, a host cell line stably expressing a Cas (preferably Cas9) nuclease (or a fragment, variant or derivative thereof as defined herein above) can be employed in the screening methods of the invention. Or, the Cas (preferably Cas9) nuclease (or a fragment, variant or derivative thereof as defined herein above) can be delivered in protein form the host cells.

As discussed above, the CRISPR/Cas system can be used to affect target gene(s) in a variety of ways. In order to generate gene knock-outs (i.e. render genes non-functional or remove genes from the genome), sgRNA effector expression products can be combined with wild-type Cas nucleases or fragments, variants or derivatives thereof which exhibit endonuclease activity and are thus capable of introducing DSBs into the genomic DNA. Said DSBs preferably induce NHEJ, resulting in the random insertion or deletion of short stretches of nucleotides leading to the disruption of the codon-reading frame (frame shifts), resulting in erroneous transcripts and ablation of gene expression (loss-of-function).

In order to edit the genome (i.e. introduce new or modified genes), sgRNA effector expression products can be combined with wild-type Cas nucleases or fragments, variants or derivatives thereof which exhibit nickase activity and are thus capable of introducing nicks (i.e. hydrolysis of the phosphodiester bonds of one strand of the double-stranded genomic DNA) into the genomic DNA. Such nicks preferably induce HDR, resulting in the incorporation of a DNA segment with regions having homology to the sequences flanking both sides of the DNA double strand break (said DNA segment also being referred to as a "DNA repair template" herein). Using HDR, any desired sequence can be inserted to produce, for example, loss of function, gain of function or altered (neomorphic) function or to investigate variants of unknown functional status. To utilize HDR to edit the genome, a DNA repair template with the desired sequence modification is typically introduced. Thus, the inventive screening methods may further comprise a step of introducing a DNA repair template into the host cells.

For methods that use the CRISPR/Cas system to activate or inhibit gene expression, a Cas (preferably Cas9) derivative, in particular an endonuclease-dead dCas9, is delivered to the host cells to recruit a transcriptional activating or inactivating activity to the promoter (or other regulatory) regions of genes. In general, the dCas9-sgRNA system could be used as a sequence-specific binding complex to deliver, in principle, any functional domain, reporter, etc. to sequence-specified target sites.

In the applications described herein (i.e. knock-out, knock-in, gene editing, or transcriptional activation or repression), the wild-type Cas (preferably Cas9), or a fragment, variant or derivative thereof as defined herein above, can be delivered to the host cells as described above.

### Kit

In a further aspect, the present invention provides a kit comprising one or more of the following components ("kit components"): (a) a polynucleotide, (b) a vector, (c) a host cell and/or (d) a library according to the invention. Kits of the invention may further comprise (e) means for detecting and/or quantifying the expression of the RNA oligonucleotides, (f) means or compounds (e.g. small organic molecules) to provide a stimulus to the host cells, (g) reagent(s) for introduction of the polynucleotide(s), vector(s) or libraries into suitable host cells and/or (h) means for detecting and quantifying the RNA oligonucleotides. Any useful combination of the aforementioned kit components is envisaged herein. The kit components are typically provided in suitable containers or vials. The kit can further comprise instructions for use. The kit can be employed, e.g., to practice the inventive methods described herein for elucidating pathways of interest or identifying potential drug targets, or for other purposes.

### DESCRIPTION OF THE FIGURES

In the following a brief description of the appended figures will be given. The figures are intended to illustrate the present invention in more detail. However, they are not intended to limit the subject matter of the invention in any way.

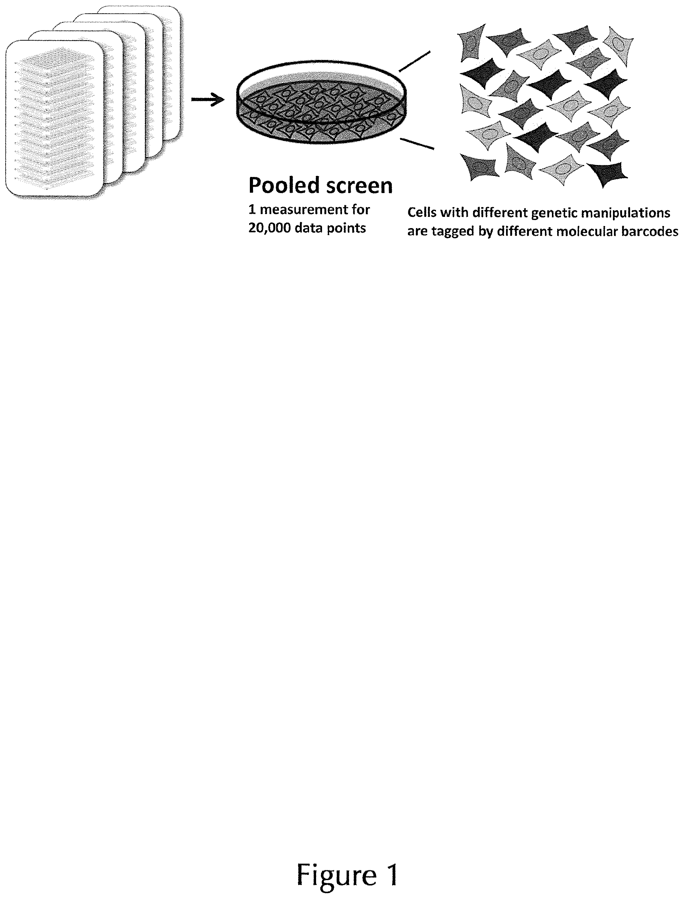

**Figure 1** schematically illustrates the screening methods and advantages of the present invention. Since each polynucleotide comprised within a host cell is tagged with a unique "barcode" UIS expressed as a unique RNA oligonucleotide, all host cells can be evaluated in one pooled screen.

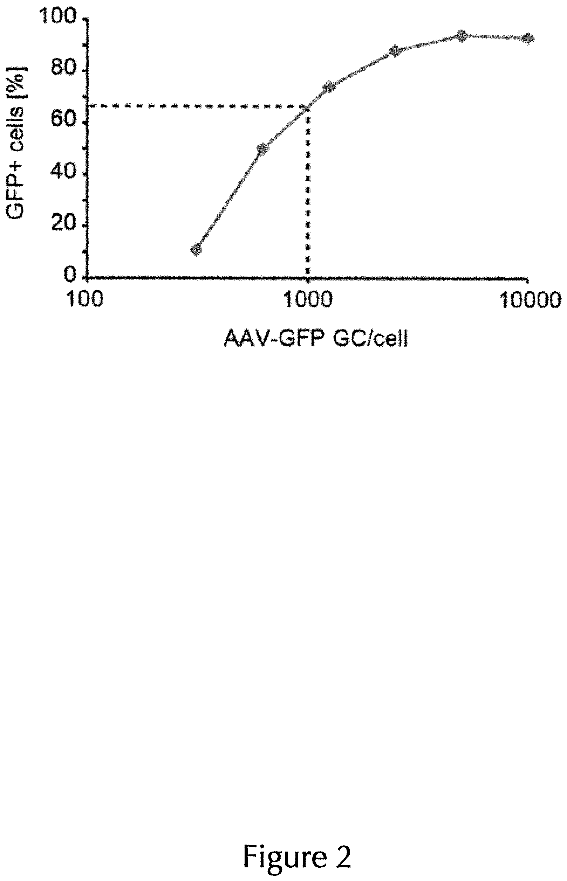

**Figure 2****: AAV infection rate.** AAV infection rate for primary neurons determined by counting GFP positive neurons after infection with a serial dilution of a GFP expressing AAV vector.

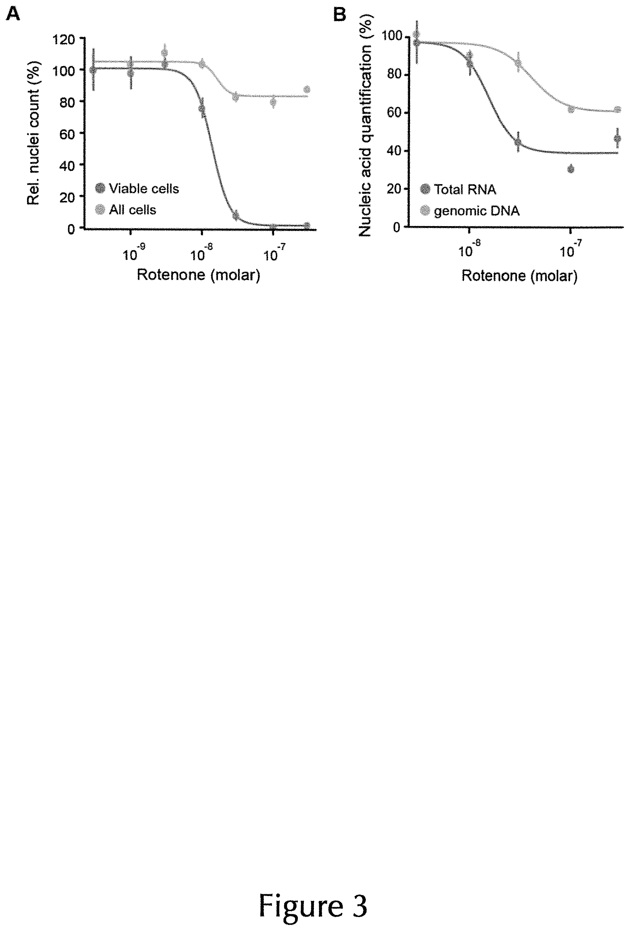

**Figure 3****: Cellular RNA content is a more sensitive indicator of neuronal viability in the dish than DNA. A:** Rotenone killing-curve. Primary neurons were treated with the indicated Rotenone concentrations from DIV7 till DIV14. Cells were counted by nuclear Hoechst stain. Either all Hoechst positive nuclei were quantified (All cells) or only non-pyknotic nuclei to discriminate viable from non-viable cells (Viable cells) (n=3 +-sem). B: Quantification of total RNA and genomic DNA content from primary neuron cultures in response to metabolic stress by Rotenone treatment (n=2 +-sem).

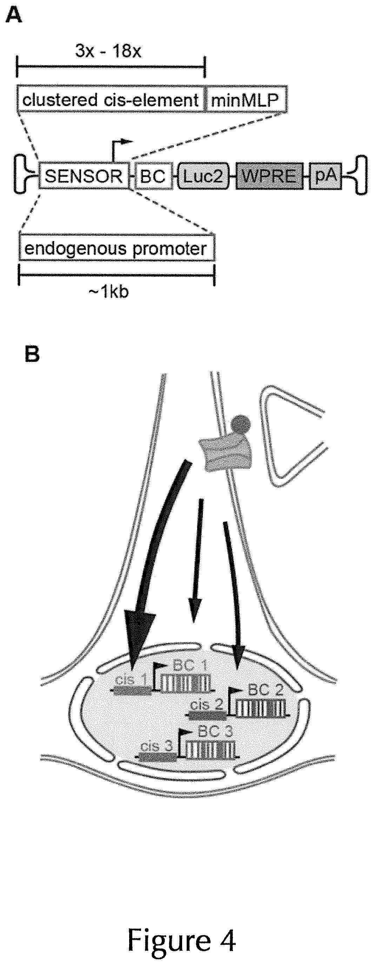

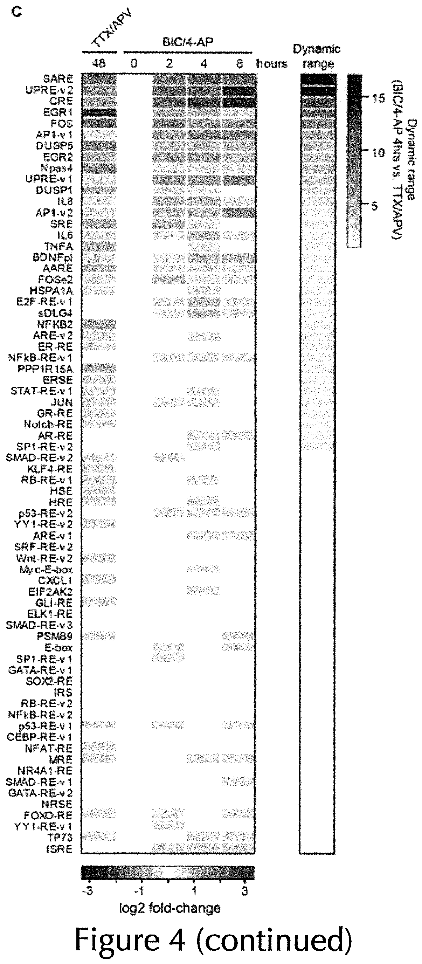

**Figure 4****: Multiplexed cis-regulatory sensor assay in response to neuronal silencing and synaptic stimulation. A:** Schematic map of the cis-regulatory sensor vector. The barcode (BC) and the firefly luciferase (luc2) are driven by clustered transcription factor binding site (cis-elements) or endogenous promoters. B: Illustration of the assay design. Neuronal cultures are infected by a pool of sensor vectors packaged into AAV particles and the sensor response is measured by NGS of the barcode pool. C: Heatmap of 70 sensor responses to TTX/APV or BIC/4-AP at the indicated hours of treatment (log2 fold changes). Sensors are ranked by dynamic range of BIC/4-AP (4hrs) vs. TTX/APV (right heatmap).

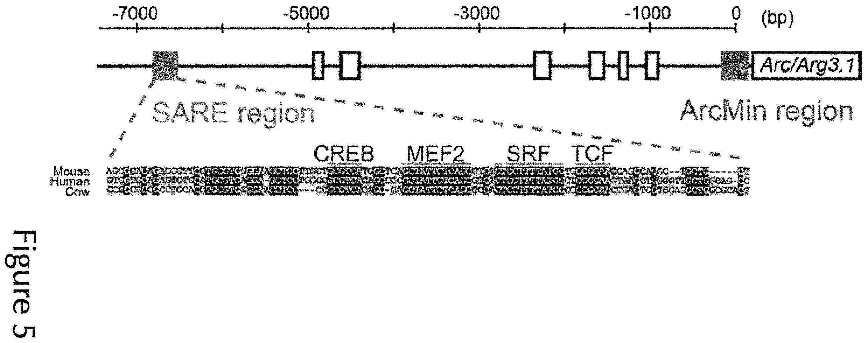

**Figure 5****: Genomic architecture of the murine Arc promoter.** Top, the SARE and ArcMin regions are indicated by blue boxes. Evolutionarily conserved genomic regions are represented by white boxes. Bottom, sequence alignment of the SARE region between mouse, human and cow. Sequences of high conservation are highlighted in black boxes. Binding sites for CREB, MEF2 and SRF/TCF are indicated. Modified from (Kawashima T et al. Nat Methods. 2013 Sep;10(9):889-95).

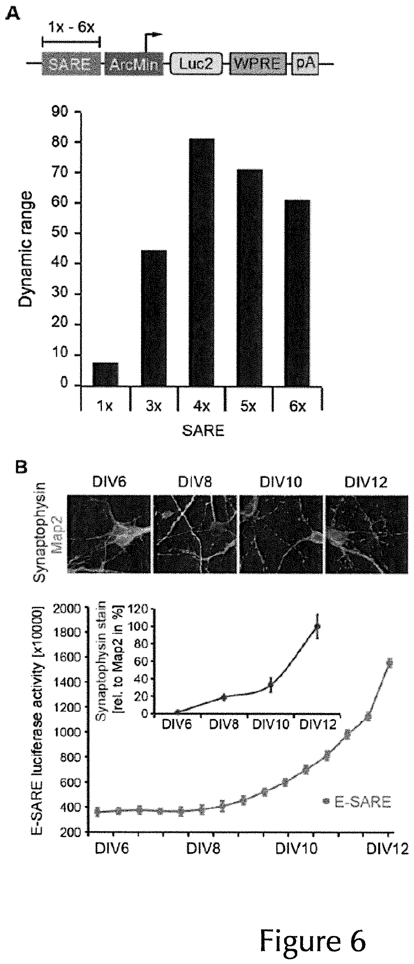

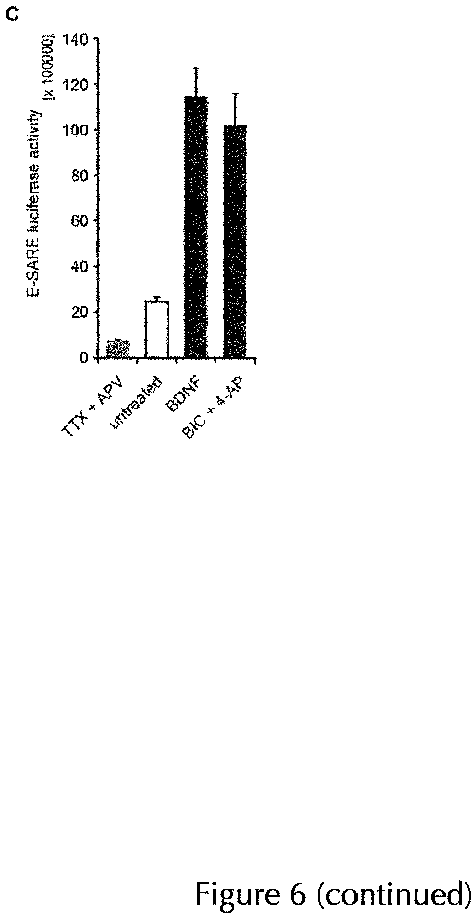

**Figure 6****: Design and characterization of the artificial E-SARE sensor. A:** Top, Schematic map of the sensor-luciferase vector with clustered SARE enhancers. Bottom, Comparison of luciferase activity of clustered SARE reporters after stimulation with PMA in SH-SY5Y cells (n=6). A cluster of four SARE is hereafter called enhanced SARE (E-SARE) sensor. B: Longitudinal measurement of basal E-SARE activity in maturing primary neurons from DIV6-12 (n=3 +-sem). Inset, Synapse quantification by staining of the presynaptic marker synaptophysin (n=3 +-sem). Top, representative images of primary neurons (DIV6-12) stained with antibodies against synaptophysin and MAP2. C: E-SARE activity in primary neurons upon silencing (TTX/APV), basal activity (untreated), and stimulation (BDNF, BIC/4-AP) at DIV14 (n=6 +-sd).

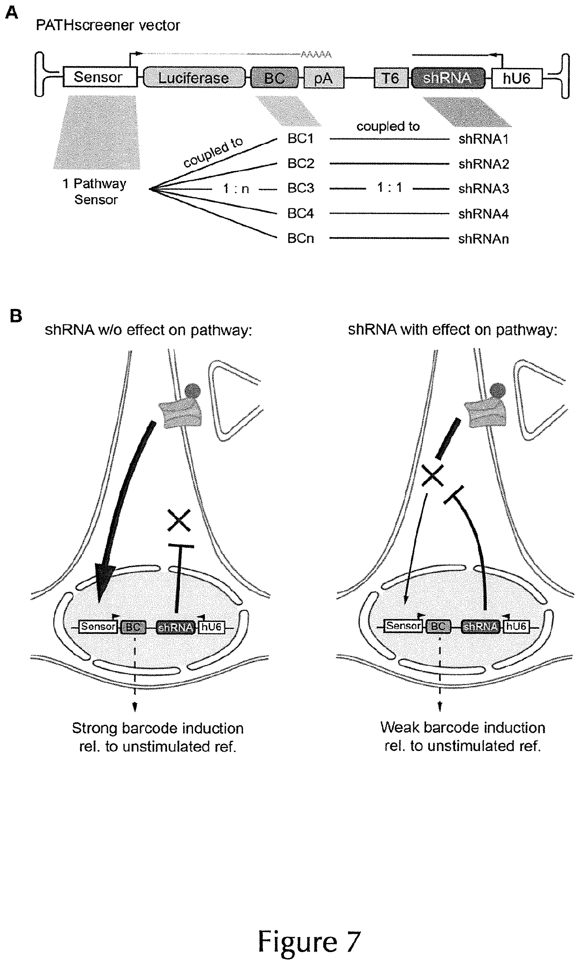

**Figure 7****: Principle of the sensor-based genetic interference screen. A:** Map of the AAV PATHscreener vector. The connection within the library between sensor, barcodes and shRNAs is indicated below. B: Graphical summary of the working hypothesis for the sensor-coupled RNAi screen. Interference independent of a given pathway addressed with a corresponding sensor does not affect the readout (left), whereas shRNAs targeting the signaling cascade alter sensor activity (right).

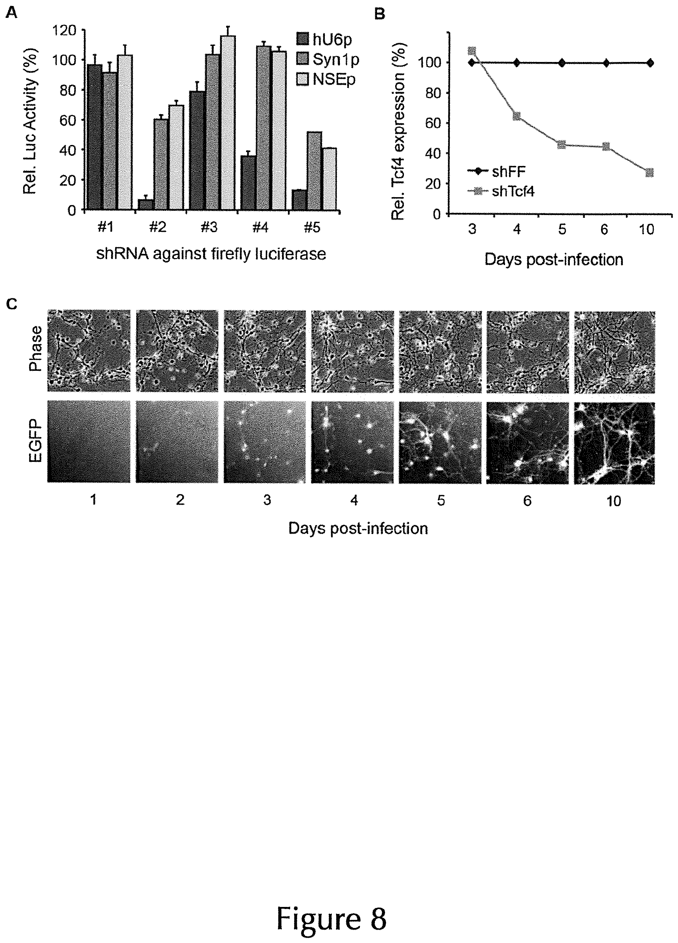

**Figure 8****: Efficient mRNA knockdown by hU6 promoter-driven shRNAs in neuronal cells. A:** Efficacy of five shRNAs, targeting firefly luciferase, driven by the hU6p, Syn1p, or NSEp promoter. shRNA expression plasmids were co-transfected with a luciferase reporter plasmid into PC12 cells (n=6, +-sd). B: Quantification of Tcf4 mRNA expression in primary neurons infected with AAVs for shRNA expression against Tcf4 or firefly luciferase as a non-targeting control on DIV1. Tcf4 mRNA was quantified at the indicated days post-infection. C: Longitudinal imaging of GFP expression in primary neurons infected on DIV1 with AAV-Syn1p-GFP (serotype 1/2).

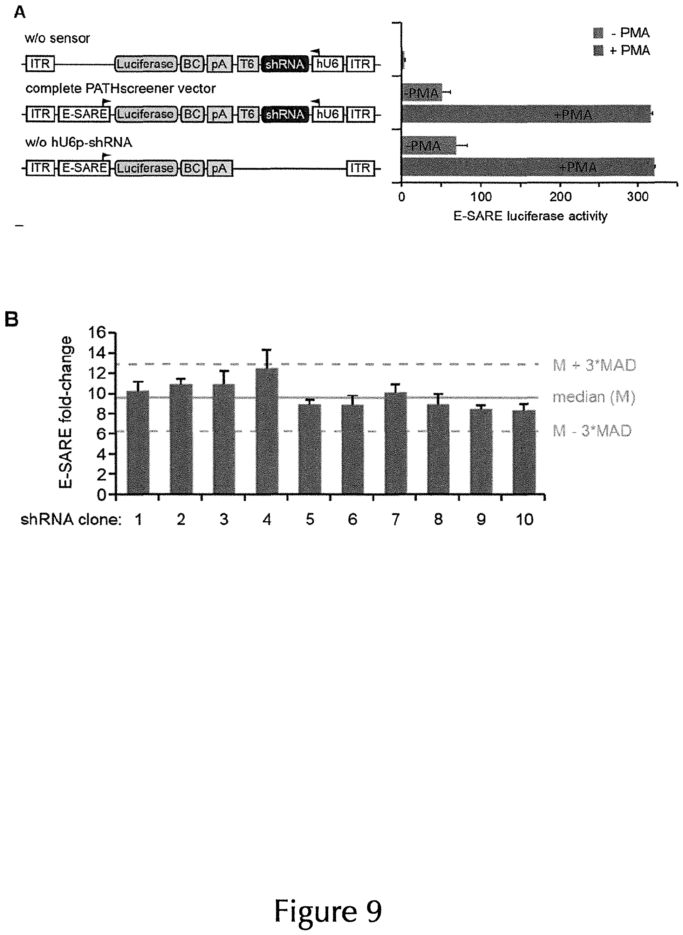

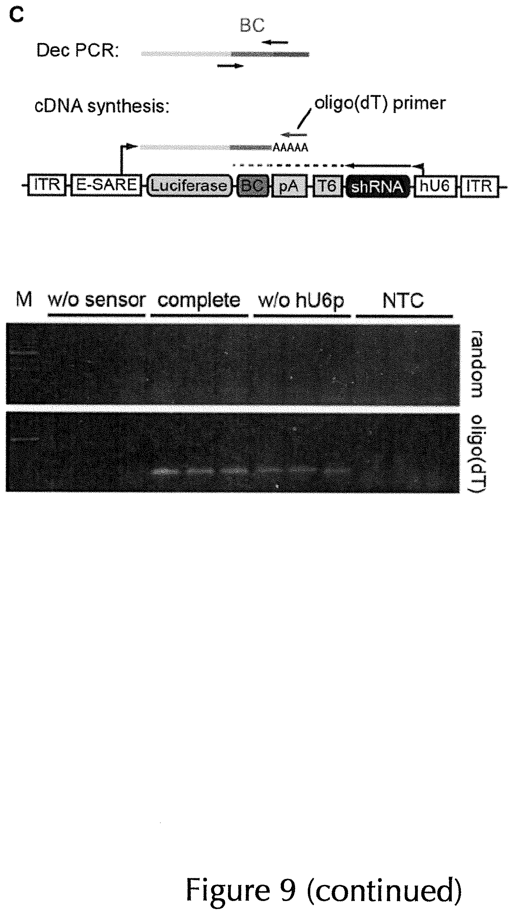

**Figure 9****: Functional validation of the PATHscreener vector. A:** Validation of an unbiased sensor response in the dual-expression PATHscreener vector. PC12 cells were transfected with the complete vector or a vector with an E-SARE sensor deletion or hU6p-shRNA deletion. Luciferase activity was measured for unstimulated and PMA-stimulated samples (n=6 +/- sd). B: PC12 cells were transfected with the PATHscreener vector expressing 10 different random shRNAs. E-SARE-luciferase fold change upon PMA stimulation does not deviate more the three median absolute deviations (MAD) from the median (n=6 +-sd). C: Left, schematic of the vector with transcripts expressed by the sensor and by the hU6 promoter. The dashed line indicates DNA polymerase III run-through transcript. The oligo(dT) primer for cDNA synthesis is indicated as well as primer binding sites for barcode amplification at the decoding (Dec) PCR. Right, PC12 cells were transfected with the vectors shown in A and purified RNA was transcribed to cDNA either with random primers or oligo(dT) primers. A prominent Dec PCR barcode product is only detectable with cDNA transcribed using oligo(dT) primers. NTC, non-template control.
**Figure 10****: Generation of the PATHscreener library. A:** Cloning workflow. Left, the shRNA expression cassette is amplified by PCR and extended by the SpA. A second PCR adds the barcode (BC) to the previous PCR product. The product of PCR#2 is finally ligated into the sensor containing AAV backbone. Right, verification of the PCR products by agarose-gel electrophoresis. B: Final cloning product. The proximity of barcode and shRNA allows the barcode - shRNA assignment by next-generation sequencing using the ION-Torrent PGM with 400bp chemistry.

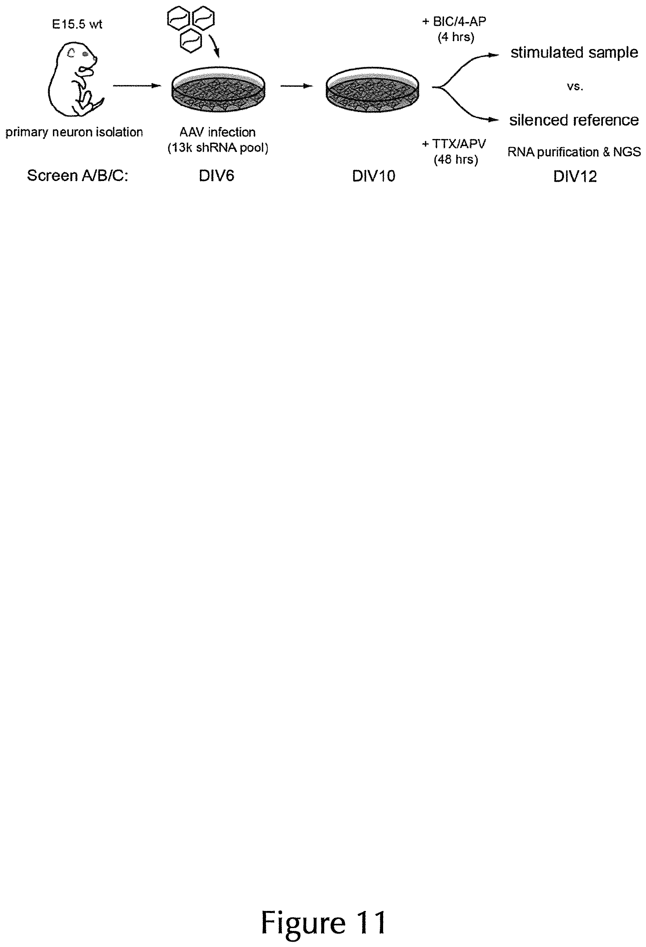

**Figure 11****: Screening design and workflow.** Primary cortical neurons were isolated from E15.5 mice and plated in 15 cm (10 mio cells; Screen A and C) or 10 cm dishes (5 mio cells; Screen B). Neurons were infected with the AAV PATHscreener library at DIV6. At DIV10 reference samples were treated with TTX/APV for the following 48 hours to reduce sensor activity. Neuronal activity is induced in the remaining cultures at DIV12 using a BIC/4-AP cocktail for 4 hours. Subsequently cultures were lysed and total RNA was purified and processed for next-generation sequencing.

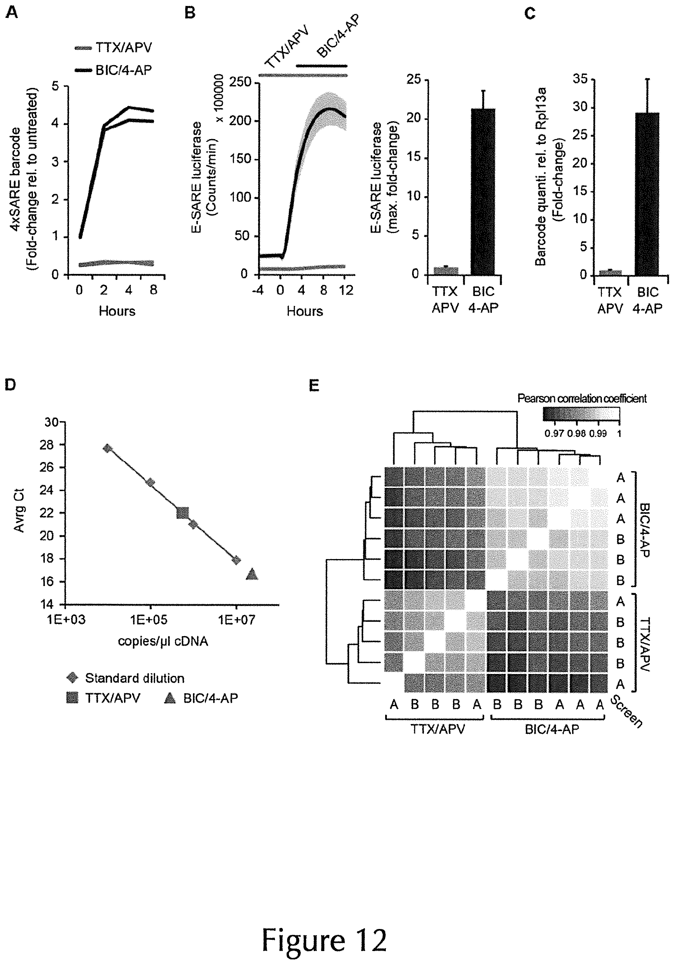

**Figure 12****: Quality control measures from the pooled RNAi screen. A:** Kinetics of the SARE-minMLP sensor response to TTX/APV and BIC/4-AP measured by barcode sequencing. The response for two individual barcodes is shown (average from two assay replicates; extracted from the multiplexed cis-regulatory sensor assay). B: Left, E-SARE sensor response to TTX/APV and BIC/4-AP determined by live cell luciferase activity measurement in sister cultures of screen A/B (n=4 +- sem). Right, maximal E-SARE luciferase induction by synaptic activity (time-point 8 hours of BIC/4-AP stimulation). C, D. Relative and absolute quantification of the barcode expression in screen A in response to TTX/APV and BIC/4-AP. E. Pair-wise correlation of normalized read counts from biological replicates of screen A and B. Clustering by unsupervised hierarchical clustering.

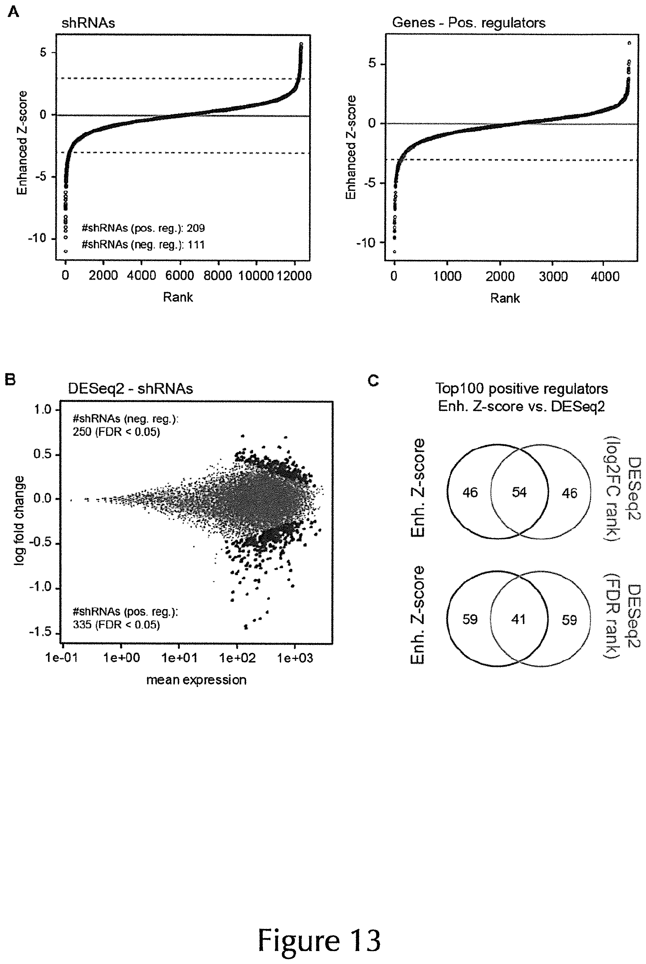

**Figure 13****: Enhanced Z-score and DESeq2 analysis of the pooled RNAi screen A. A:** Enhanced Z-score rankings. Left, For individual shRNAs from screen A. An enhanced Z-score of +3/-3 is indicated by a dashed line. Right. Collapsed to gene level by filtering for the shRNA with the most negative score per gene. The dashed line indicates a score of -3. Selected candidates for subsequent validation are highlighted in red. B: MA-plot compares for shRNAs the DESeq2 log2 fold changes (screen A) to the mean expression of the corresponding barcode. shRNAs with differentially expressed barcodes are highlighted in red (FDR < 0.05). C: Overlap between the top 100 positive regulators identified by enhanced Z-score analysis and by DESeq2 analysis (either ranked by log2 fold change or FDR). FDR was determined using the Benjamini-Hochberg method.

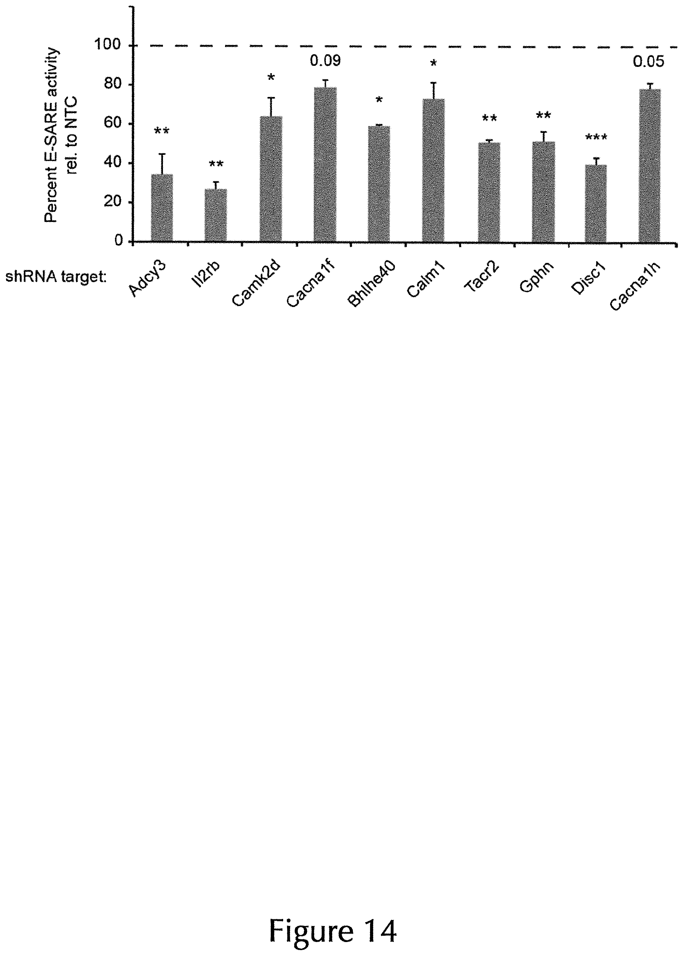

**Figure 14****: Individual validation of selected candidates.** E-SARE activity in response to knockdown of 10 candidate genes relative to a non-targeting control (NTC). Primary cortical neurons were infected with individual PATHscreener vectors for 10 different shRNAs selected from screen A and a NTC vector. In accordance to the screening conditions cultures were either silenced by TTX/APV (48 hrs) or stimulated by BIC/4-AP (4 hrs) and E-SARE activity was determined by luciferase measurement (n=3 +- sd).

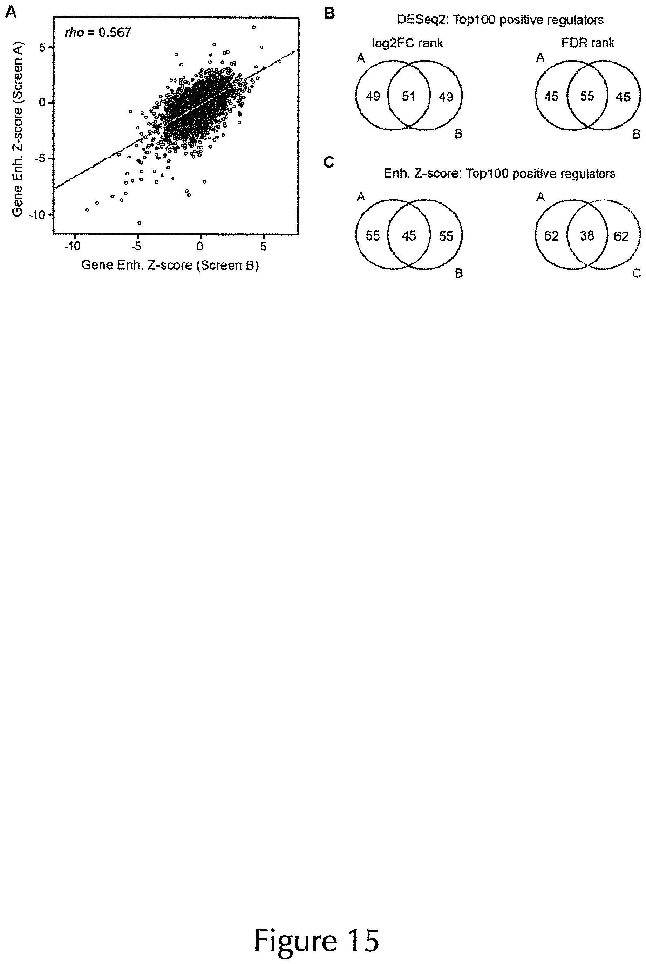

**Figure 15****: Reproducibility of the pooled RNAi screen in primary neurons. A:** Scatterplot of enhanced Z-scores from screen A and B. Data has been collapsed to gene level and filtered for positive regulators. The spearman-rank coefficient rho and linear regression are indicated. B, C. Venn diagrams for the overlap in the hit lists generated by DESeq2 analysis (B) and enhanced Z-score ranking (C).

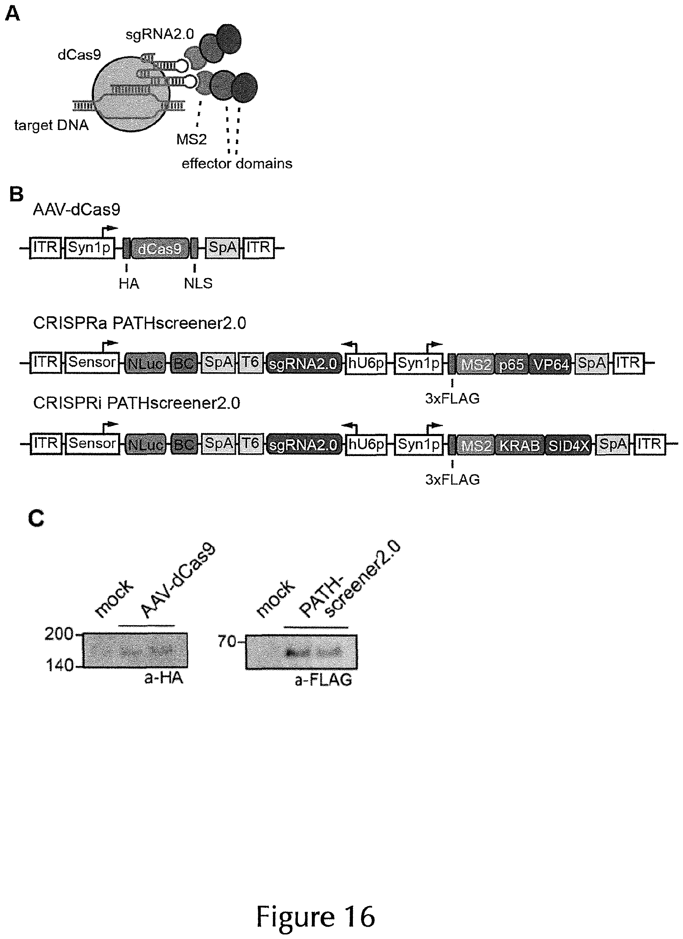

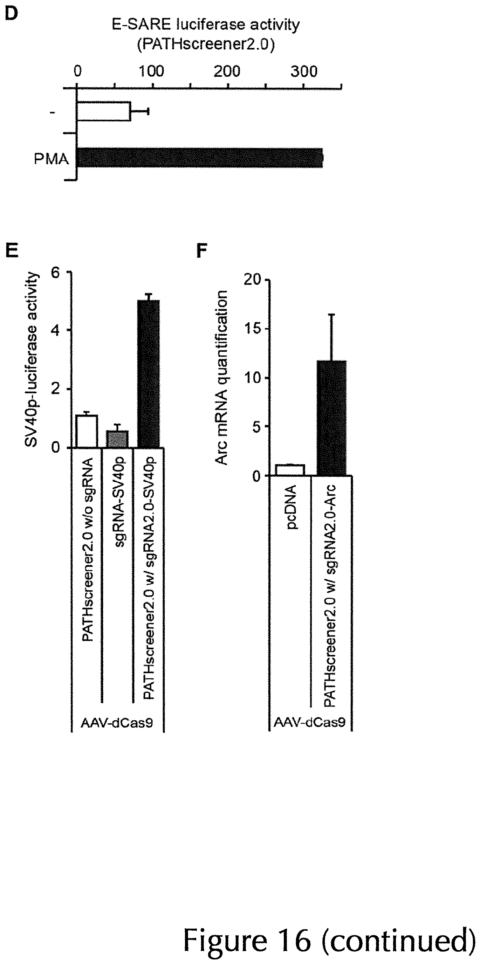

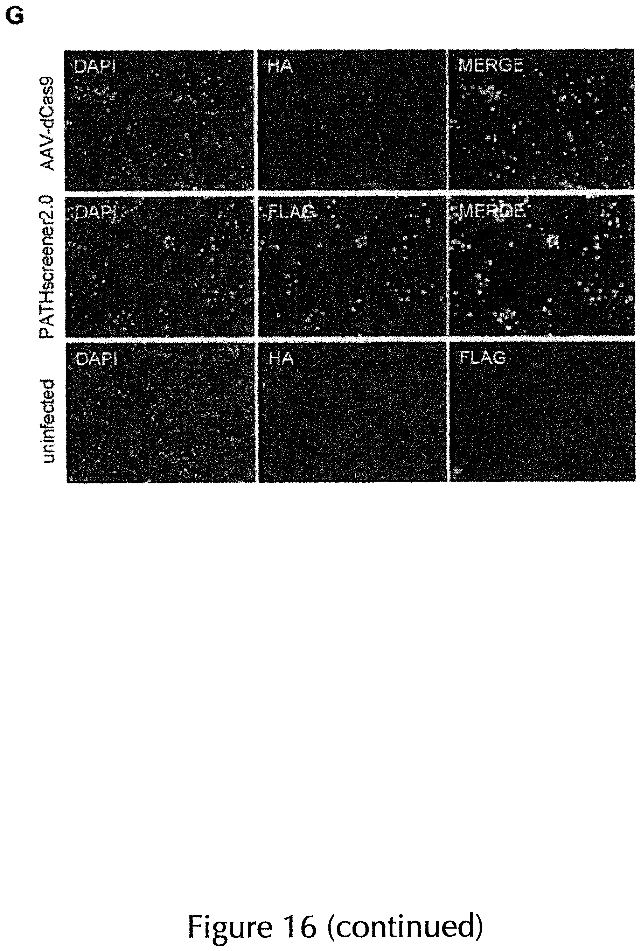

**Figure 16****: Adaptation towards an AAV CRISPR screening vector. A:** Schematic of the CRISPRa complex comprising the target DNA bound by the inactive Cas9 (dCas9) and the sgRNA2.0 harboring two MS2 loops. MS2 loops are recruiting the MS2-binding domain fused to the p65 and VP64 transcription activator domains. B: AAV maps of the dCas9 vector (top) and the CRISPRa-PATHscreener vector (bottom). C: Expression validation of dCas9 and MS2-p65-VP64 in HEK293FT cells by western blot. D: Validation of the E-SARE sensor response to PMA in the context of the CRISPRa-PATHscreener vector in HEK293 cells (n=6 +- sd). E: Transcriptional activation of a SV40p-luciferase reporter by dCas9, MS2-p65-VP64 and sgRNA2.0-SV40 (n=6 +- sd). F Transcriptional activation of Arc gene expression in N2a cells by dCas9, MS2-p65-VP64 and sgRNA2.0-Arc (n=3 +- sd). G Validation of nuclear dCas9 and MS2-p65-VP64 localization in primary mouse neurons after AAV infection of the vectors shown in B.

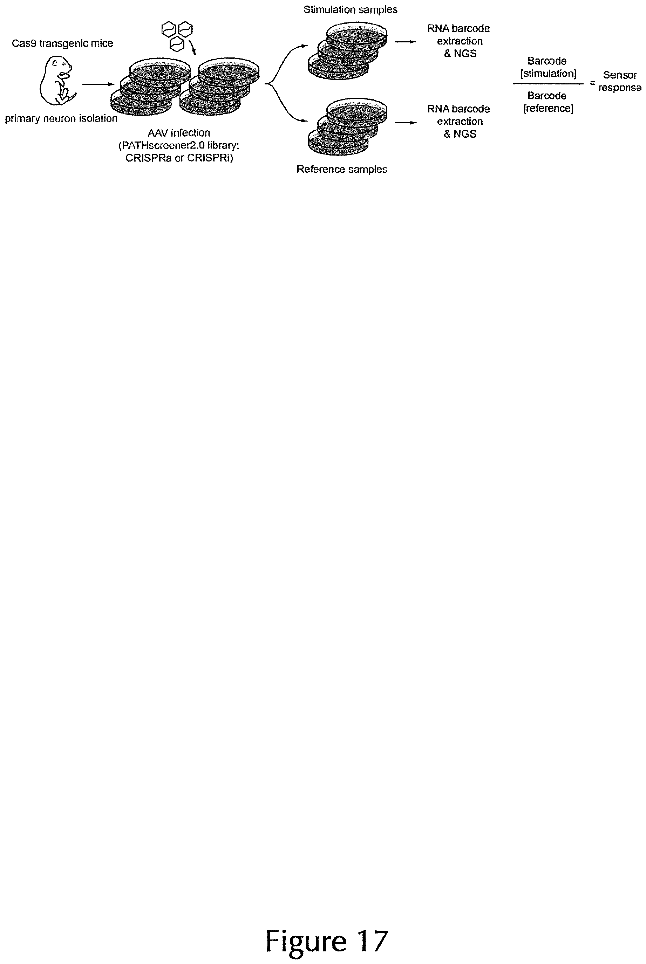

Figure 17 schematically illustrates the screening methods of the present invention using an AAV CRISPR screening vector.

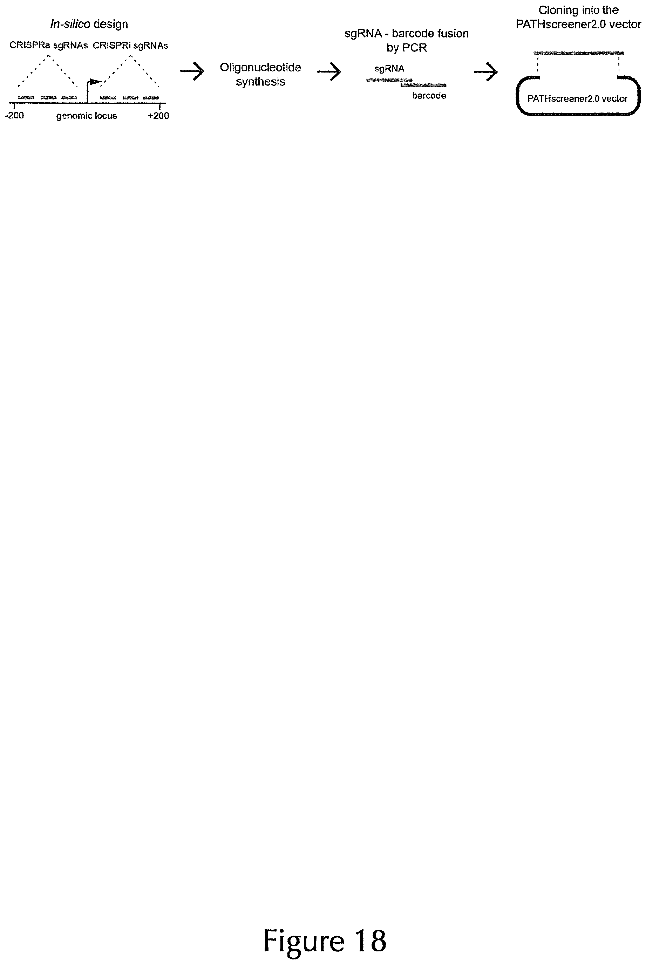

**Figure 18** schematically illustrates the generation of a CRISPRa/CRISPRi vector library.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only. Indeed, various modifications of the invention in addition to those described herein will become readily apparent to those skilled in the art from the foregoing description, accompanying figures and the examples below.

### Example 1: Materials

**Oligonucleotides:**

| **cDNA synthesis:** | | SEQ ID NO: |
|---|---|---|
| Oligo(dT) | TTTTTTTTTTTTTTTTTTTT | 3 |
| Random nonamer (N9) | NNNNNNNNN | |
| | | |

| **qRT-PCR:** | in 5' → 3' direction | |
|---|---|---|
| Tcf4 fwd (Mm) | CTGGAGCAGCAAGTTCGAG | 4 |
| Tcf4 rev (Mm) | TTCTCTTCCTCCCTTCTTTTCA | 5 |
| Arc fwd (Mm) | AGGGGCTGAGTCCTCACA | 6 |
| Arc rev (Mm) | GACTTCTCAGCAGCCTTGAGAC | 7 |
| Rpl13a fwd (Mm) | ATCCCTCCACCCTATGACAA | 8 |
| Rpl13a rev (Mm) | GCCCCAGGTAAGCAAACTT | 9 |
| WPRE fwd | ACTGTGTTTGCTGACGCAAC | 10 |
| WPRE rev | AGTCCCGGAAAGGAGCTG | 11 |
| hU6p fwd | TTTCAAGTTACGGTAAGCATATGATAGT | 12 |
| hU6p rev | CAAGGCTGTTAGAGAGATAATTGGAAT | 13 |

| **IonTorrent sequencing:** | | |
|---|---|---|
| qDec1.2 fwd | CCGAGTAGAATTAACCCTCACTAAA | 14 |
| qDec2.2. rev | CGCGTCTACTAATACGACTCAC | 15 |
| Dec fwd | AGCTAGTTGCTAAGTCTGCCGAGTAG | 16 |
| Dec rev | TCGTACATGCATTGACTCGCGTCTAC | 17 |
| PGM_A_IXcodeX_AFA_s | | 18 |
| | | |

| **shRNA library cloning:** | | |
|---|---|---|
| hU6 fwd | TCTCAGAGAGAGACAGAGACAGATCC | 19 |
| Dec rev | GTAGACGCGAGTCAATGCATGTACGA | 20 |
| BC35 rev | | 21 |
| SpA | | 22 |
| BC35 | | 23 |
| PGM_trP1_hU6_as | | 24 |
| | | |

| **shRNAs:** | | |
|---|---|---|
| Tcf4 (target sequence) | TTCTAATTACCGGATATTGAAT | 25 |
| Adcy3 | | 26 |
| Il2rb | | 27 |
| Camk2d | | 28 |
| Cacna1f | | 29 |
| Bhlhe40 | | 30 |
| Calm1 | | 31 |
| Tacr2 | | 32 |
| Gphn | | 33 |
| Disc1 | | 34 |
| Cacna1h | | 35 |
| | | |

| **sgRNAs:** | **Target sequence** | |
|---|---|---|
| SV40-promoter | GAATAGCTCAGAGGCCGAGG | 36 |
| Arc-promoter | CCTACTCGCTCCCCTCCCGT | 37 |

| **Plasmids ID:** | **AAV production** | |
|---|---|---|
| V1739 | pFdelta6 (adenoviral helper proteins) | |
| V1740 | pRV1 (serotype 2 capsid protein) | |
| V1741 | pH21 (serotype 1 capsid protein) | |

| | **cis-regulatory sensor assay:** | |
|---|---|---|
| V825 | pAAVspace_DEST_luc2_WPRE | Cloning vector |

| | **SARE sensor:** | |
|---|---|---|
| | pAAV_SARE-ArcMin-luc2_WPRE_pA | 1-6x SARE |

| | **PATHscreener:** | |
|---|---|---|
| V1337 | Cellecta Decipher Mouse Module 1 library | |
| V1338 | AAV E-SARE PATHscreener (with shRNA) | Library or single shRNA |
| V1301 | AAV E-SARE PATHscreener (shRNA stuffer) | Non-targeting control |

| | **CRISPR-Cas9:** | |
|---|---|---|
| V1785 | pAAV_Syn1 p-dCas9 | |
| V1787 | PATHscreener2.0 (sgRNA stuffer) | |
| | sgRNA-SV40 | |
| | pSV40-luc2 | |

### Example 2: Methods

### Example 2.1 Culturing of eukaryotic cell lines

The cell lines PC12, SH-SY5Y, N2a, HEK293, and HEK293FT were cultured in the appropriate growth medium until reaching 80-90% confluency and being passaged using standard protocols. For all experiments, cells were seeded into the appropriate cell culture plate one day prior to the beginning of the experiment.

### Example 2.2 Primary neuron culture

Primary mouse cortical neurons were prepared from E15.5 C57BL/6 mice embryos and cultured on poly-L-lysine (PLL, 0.1 mg/ml in dH2O)-coated culture dishes or glass coverslips in neuronal plating medium (containing 5% FBS, except for 96-well plates). Mouse cortices were dissected in cold HBSS/5 mM HEPES and dissociated by Papain treatment in Neurobasal medium and subsequently placed in neuronal plating medium. Cells were separated by pipetting and transfer through a 40 µm mesh. Subsequently, cell counts were determined and cells were plated in final culture medium volume in the cell culture dish. In all experiments a cell density of ∼500 cells/mm2 was used.

Except for experiments in 96-well plates, where serum-free medium was used from the beginning, culture medium was completely replaced by serum-free neuronal culture medium on DIV1. Feeding of the neuronal cultures was done on DIV6-7 for the first time by replacing half of the volume with neuronal culture medium. From then on, cultures were fed every 3-4 days until the end of the experiment.

### Example 2.3 AAV production

### Example 2.3.1 Transfection

AVs were produced using HEK293FT cells. For a single virus preparation, 12e+06 cells were transfected with 10 µg pFdelta6, 3.75 µg pRV1, 3.75 µg pH21 and 4 µg pAAV (i.e the custom AAV plasmid) in HEK293 medium using polyethyleneimine (PEI) as a transfection reagent. The mix of two AAV capsid expressing plasmids pH21 (serotype 1) and pRV1 (serotype 2) results in AAV particles with mixed capsid proteins form AAV serotype 1 and 2. This mix was determined to have superior infection efficiencies compared to either serotype alone (data not shown).

### Example 2.3.2 AAV harvest

Three days post-transfection, AAV particles were harvested from the culture. Therefore, cells were lysed and genomic DNA was removed. Subsequently, cell debris was removed by centrifugation and AAV-containing supernatant was ultrafiltrated to obtain the viral particles.

### Example 2.3.3 Absolute quantification of the AAV genomic copies (GC) by quantitative PCR (qPCR)

AAV GCs were quantified by qPCR as a measure to control the infection rate between experiments. Absolute quantification was done on a Qiagen Rotor-Gene cycler using the 2x RotorGene SYBRgreen PCR Master Mix and the hU6p qRT-PCR primer pair. Final AAV GC titers in the range of 1e+09- 1e+10 GC/µl were usually obtained.

### Example 2.3.4 Determination of the infectious AAV titer

In order to correlate the AAV GC titer to the infection rate of murine primary neurons, cortical cultures were infected with a serial dilution of a GFP expressing AAV and GFP positive cells were counted by a microscopic analysis (Figure 2). This correlation was used to adjust the infection rate of the AAV shRNA library to a level ensuring that the great majority of infected cells are only transduced by a single AAV particle.

### Example 2.4 Rotenone dose-response cell viability assay

The response of primary neurons to various rotenone concentrations was determined by microscopy counting of cell nucei, as well as quantification of genomic DNA (gDNA) and total RNA. Primary neurons were treated with various Rotenone concentrations ranging from 3.16e-09 M to 3.16e+12 M at DIV7. Cells were incubated under appropriate culturing conditions until DIV14.

Cell nuclei were stained on DIV14 with Hoechst dye and evaluated with a Zeiss Observer Z1 microscope. Subsequently, the neuron cultures were lysed and gDNA as well as total RNA was isolated using the Qiagen AllPrep DNA/RNA Mini Kit. The gDNA and total RNA concentrations were determined using a UV spectrophotometer.

### Example 2.5 Multiplexed cis-regulatory sensor assay

### Example 2.5.1 Cloning of the cis-regulatory sensor library

*Cis*-regulatory sensors in the AAV-based library consisted either of clustered transcription factor binding sites and response elements upstream of the minimal adenoviral major late promoter (minMLP) (termed *cis*-element-based sensors), or of 1-1.5 kb long endogenous promoter fragments which harbour the transcription start site (termed promoter-based sensors). The corresponding sequences were either synthesized by Genscript or PCR amplified from genomic DNA. Cloning was performed using the MultiSite Gateway pro plus kit (Invitrogen) according to the manufacturer's instructions. Thereby, three insert fragments were cloned into the pAAVspace_DEST_luc2_WPRE (V825) vector by recombination. For cis-element-based sensors, the three fragments consist of the (i) clustered cis-element, (ii) the minMLP, and (iii) the molecular barcode (UIS). In case of a promoter-based sensor, the three fragments are (i) a stuffer sequence, (ii) the promoter, and (iii) the molecular barcode (see Figure 4A). All constructs were verified by restriction digest and sequencing of the insert.

### Example 2.5.2 Cell culture

E15.5 wt primary cortical mouse neuron cultures were infected with the AAV *cis*-regulatory sensor library on DIV5 using 2500 AAV GCs per cell. A duplicate sample was silenced by treatment with 1 µM TTX and 100 µM APV on DIV12. On DIV14, a duplicate sample was harvested as an untreated reference sample. The remaining samples were stimulated with 50 µM BIC, 100 µM 4-AP, 100 µM glycine, and 1 µM strychnine for 2, 4, and 8 hours in duplicates. All samples were finally harvested using Qiazol reagent. RNA was purified using the Zymo Direct-zol RNA MiniPrep kit, treated with TurboDNase to digest residual AAV genomes and cleaned up by a second column purification using the Zymo Direct-zol RNA MiniPrep kit according to the manufacturer's instructions.

Subsequently, cDNA synthesis was performed using standard protocols.

Following cDNA synthesis, UIS barcodes were amplified by PCR with Dec1/2 primers (250 nM each) using HotStar Taq plus DNA polymerase (Qiagen). In addition, an external barcode mix was added to the PCR reaction, which can be used for calibration between samples.

In a second PCR, the adapter sequences for Ion Torrent sequencing were fused to the amplified UIS barcodes. The forward code primer contained the Ion-A adapter sequence required for Ion Torrent sequencing and a 12bp code sequence for multiplexing of samples in a single sequencing run. The reverse primer contained the Ion-P1 adapter sequence required for Ion Torrent sequencing. PCR was performed using HotStar Taq plus DNA polymerase (Qiagen). The PCR product was verified by agarose gel-electrophoresis. Final PCR products were pooled and purified using the NucleoSpin Gel and PCR Clean-up kit (Macherey&Nagel).

Barcode libraries were sequenced on an Ion Torrent PGM sequencer according to the manufacturer's protocols for the Ion PGM Template OT2 200 kit (for template preparations and enrichments) or the Ion PGM Sequencing 200 v2 kit (for sequencing). Processing of the raw data was performed using custom shell and R scripts. First, raw reads were split into individual samples using the 12 bp code and subsequently mapped to a reference barcode library using a local BLAST. Thereby, reads were counted. Next, read counts were normalized to total read numbers per sample. Normalized read counts were standardized to read counts of a sensor which contains only the minMLP, but no *cis*-regulatory element. Finally, data was presented as a heatmap of log2-transformed fold changes relative to the untreated reference sample.

### Example 2.6 Luciferase Assays

Firefly luciferase reporter gene assays were performed during the development and validation of the PATHscreener and PATHscreener2.0 vector and for the validation of individual candidates from the screen.

### Example 2.6.1 Multiplate luciferase assays

For multiplate assays, cells were transfected (for cell lines) using Lipofectamine2000 (Invitrogen) according to the manufacturer's instructions or infected (for primary neurons) by an E-SARE-luciferase containing AAV with 500-1000 AAV GCs per cell.

The assay compares an unstimulated response with a stimulated response.

Validation of individual shRNAs was accomplished with primary neurons infected with AAV PATHscreener vectors either expressing an shRNA or a non-targeting control RNA. Cultures were treated the same way as the screening samples (BIC/4-AP vs. TTX/APV). At the end of the assay, cells were lysed and luciferase activity was measured by a Mithras LB 940 Microplate Reader (Berthold Technologies) and the software MicroWin2000.

### Example 2.7 Protein detection by immunocytochemistry

Immunocytochemistry was used to quantify synapses in maturing primary neuron cultures and to verify the expression of CRISPR-Cas9 components.

Therefore, primary neurons were grown on glass coverslips and treated with primary and secondary antibodies and DAPI.

### Synapse stainings

Primary antibodies: mouse anti-MAP2 (1:200); rabbit anti-synaptophysin (1:250)

Secondary antibodies: Alexa488 anti-mouse (1:500); Alexa647 anti-rabbit (1:500)

### CRISPR-Cas9 stainings

Primary antibodies: mouse anti-FLAG-M2 (1:100); rat anti-HA (1:100)
Secondary antibodies: Alexa488 anti-mouse; Cy3 anti-rat (both 1:500)

Images were acquired using a Zeiss Observer Z1 microscope in combination with the Zeiss Zen 2012 software. For the synapse quantification, images were analyzed with Fiji ImageJ using the 'Find Maxima' function. The synapse count was normalized to the mean intensity of the MAP2 staining.

### Example 2.8 Protein detection by western blot

Western blotting was used to verify the expression of the CRISPR-Cas9 components dCas9 (HA-tagged) and MS2-p65-VP64 (FLAG-tagged) in HEK293 cells.

Therefore, HEK293 cells transfected either with V1785 or V1787 using Lipofectamine2000 (Invitrogen) according to the manufacturer's protocol. Western Blotting was performed according to standard protocols using the following primary and secondary antibodies:.

### Primary antibodies:

rat anti-HA (1:1000), mouse anti-FLAG-M2 (1:5000)
mouse anti-GAPDH (loading control, 1:1000)
Secondary antibodies: anti-rat-HRP (1:5000), anti-mouse-HRP (1:5000)

### Exampe 2.9 RNA detection by reverse transcription and qRT-PCR

Relative mRNA quantification was used to determine the knockdown of an shRNA or the activation of gene expression by CRISPRa. In both cases, total RNA was isolated using the Zymo Direct-zol RNA MiniPrep kit according to the manufacturer's instructions, including an on-column DNA digest. The first-strand cDNA synthesis was done using SuperScript III reverse transcriptase kit (Invitrogen). qRT-PCR assay primers were designed using the Roche Universal Probe Library assay design center. qRT-PCRs were performed on a Qiagen Rotor-Gene cylcer using the 2x Rotogene SYBR green Master Mix. Rpl13a was used as a reference gene. The relative quantification was done with the Qiagen Rotogene software using the ΔΔCt-method (Schmittgen TD and Livak KJ. Nat Protoc. 2008;3(6):1101-8.).

### Example 2.10 Design and cloning of the PATHscreener library

### Insert preparation

The Decipher Mouse Modul 1 shRNA library by Cellecta was used as the shRNA library template for the insert. The insert was prepared by two consecutive PCRs in order to add the synthetic polyA signal (SpA) and the random UIS barcode (BC35).

**PCR#1**

| | |
|---|---|
| Decipher shRNA library (10 ng/µl) | 1 µl |
| SpA oligo (2 nM) | 1 µl |
| Dec rev primer (10 µM)* | 0.5 µl |
| hU6 fwd primer (10 µM)* | 0.5 µl |
| dH2O | 7 µl |
| 2x PWO Master Mix | 10 µl |

| | |
|---|---|
| *add after first 5 cycles PCR parameters: 95°C 2 min, 95°C 20 sec, 59°C 20 sec, 72°C 30 sec (5 cylces), 95°C 20 sec, 55°C 20 sec, 72°C 30 sec (20 cycles). | |

**PCR#2**

| | |
|---|---|
| Product PCR#1 (2e+09 molecules/µl) | 1 µl |
| BC35 oligo (4e+09 molecules/µl) | 1 µl |
| BC35 rev primer (10 µM) | 0.5 µl |
| hU6 fwd primer (10 µM) | 0.5 µl |
| dH2O | 7 µl |
| 2x PWO Master Mix | 10 µl |

| | |
|---|---|
| PCR parameters: 95°C 2 min, 95°C 20 sec, 55°C 20 sec, 72°C 30 sec (10 cylces) | |

After each PCT, the PCR product was purified using the NucleoSpin Gel and PCR Clean-up kit (Macherey&Nagel) and validated by agarose gel-electrophoresis.

The purified product of PCR#2 was digested with BamHI and Clal and subsequently purified using the NucleoSpin Gel and PCR Clean-up kit (Macherey&Nagel). The final insert concentration was determined using the Picodrop spectrophotometer.

### Vector preparation

The empty PATHscreener vector (V1301, 10 µg) was digested with BamHI and Clal and purified by agarose gel-electrophoresis, the NucleoSpin Gel and PCR Clean-up kit (Macherey&Nagel). Linearized vectors were purified by phenol/chloroform/ethanol extraction, precipitated and pelleted before being reconstituted in TE buffer.

### Ligation

Inserts were ligated into empty linearized vectors at a vector:insert ratio of 1:3 overnight using T4 DNA ligase (NEB).

### Transformation

Transformation of MegaX Dh10b E.coli was accomplished using electroporation. Immediately after electroporation, E.coli bacteria were recovered in Recovery medium and incubated at 37°C for 1 hour before being plated on LB-agar (ampicillin) plates for determining colony numbers. Twelve mini cultures were inoculated with individual colonies in order to verify the cloning.

The volume for ∼10,000 colonies was spread onto a 15cm LB-agar (ampicillin) plate. In total 3 plates were inoculated in order to get a maximum number of ∼30,000 different UIS barcodes within the library. The next day, colonies from all 3 plates were collected and plasmid DNA was purified using the NucleoBond PC100 Midiprep Kit (Macherey-Nagel).

### Example 2.11 Sequencing of shRNA and barcode

In order to assign the UIS barcode sequence to the shRNA, the region encoding the UIS barcode and the shRNA were amplified by PCR using primers with Ion Torrent sequencing adapters (PGM_A_IXcode3_AFA_s fwd primer / PGM_trP1_hU6_as rev primer).

The PCR product has a size of 345 bp and was sequenced on an Ion Torrent PGM sequencer. All template preparations and enrichments were performed according to the manufacturer's protocols for the Ion PGM Template OT2 400 Kit. Sequencing was performed according to the manufacturer's protocols for Ion PGM Hi-Q Sequencing Kit.

The analysis was done using a custom R script which included the following steps: Raw reads were filtered by length (>231 bp) in order to cover the barcode sequence and the shRNA antisense strand. Using the Biostrings function matchLRPatterns() filtered raw reads were scanned for the UIS barcode (left pattern) and the 8 bp adjacent to the shRNA antisense strand (right pattern). 5 mismatches were allowed in the left pattern without indels and 3 mismatches in the right pattern with indels. Thereby, barcode sequences and shRNA antisense strand sequences were extracted. Next, shRNA antisense strand sequences were aligned by BLAST to the Cellecta shRNA library reference list. At this stage, a matrix with a barcode ID, the barcode sequence, the shRNA sequence, and the shRNA ID, and the Refseq ID of the shRNA target was created. This matrix was finally filtered for barcodes that were coupled to the same shRNA in more than 80% of the cases. The final barcode library was aligned to itself using a local megablast with varying parameters to determine optimal megablast stringency for barcode discrimination during the screen. These were an e-value threshold of 1e-10 and a word-size of 12.

### Example 2.12 Pooled RNAi screen in primary neurons

### Cell culture

Primary cortical neuron cultures were prepared from E15.5 wt mice. Neurons from 16-24 embryos were pooled per screen. Cells were seeded onto PLL-coated tissue culture dishes in Neurobasal medium supplemented with 5% FBS, 2% B27, and 1% GlutaMax. 2-4 replicate cultures were prepared per treatment condition. On DIV1, the medium was replaced by Neurobasal medium supplemented with 2% B27 and 1 % GlutaMax (NeuroCulture medium). On DIV6, cultures were infected with the AAV-PATHscreener library at an AAV particle to cell ratio of 1000:1. Simultaneously, cultures were fed by adding NeuroCulture. On DIV10, all cultures were fed with NeuroCulture and half of the cultures were additionally treated with 1 µM TTX and 100 µM APV to silence neuronal activity. Samples for real-time luciferase recordings were additionally supplemented with the firefly luciferase substrate luciferin before evaluation with the Lumicycler. On DIV12, non-silenced cultures were stimulated with a cocktail containing 50 µM BIC, 100 µM 4-AP, 100 µM glycine, 1 µM strychnine for 4 hours.

### Harvest

Cells of silenced and stimulated cultures were harvested and lysed using standard protocols for RNA isolation.

### Total RNA isolation

Lysates were thawed at room temperature and cell debris was pelleted by centrifugation. Total RNA was isolated from supernatants using the Zymo Direct-zol RNA MiniPrep kit according to the manufactures instructions with the following modifications. The lysate from the sample obtained from 10 million cells was split onto 2 RNA purification columns in order to not exceed the RNA binding capacity of a column. Elution was accomplished in 50 µl RNase-free H2O per column and the two eluates from 10 million cells were pooled afterwards Total RNA was quantified using a Picodrop spectrophotometer. ∼60 µg or ∼30 µg total RNA was obtained from 10 million or 5 million cells, respectively.

In order to digest traces of co-isolated AAV genomes, total RNA was treated with TurboDNase for 30 minutes at 37°C (6 µl TurboDNAse/∼60 µg total RNA). With less total RNA input the reaction was scaled down accordingly. The DNase-digested total RNA was subsequently purified by adding 1 vol. of 100% ethanol and using the Zymo Direct-zol RNA MiniPrep Kit for purification. One column was used per sample. Total RNA was again quantified using a Picodrop spectrophotometer.

### cDNA synthesis

The first-strand cDNA synthesis was performed using the Invitrogen SuperScript III reverse transcriptase. The entire total RNA was reverse transcribed in multiple 20 µl reactions containing 5 µg total RNA each and using oligo(dT) primer. The reaction protocol was as followed:

| | |
|---|---|
| Total RNA | 5 µg |
| Oligo(dT) primer (50 µM) | 1 µl |
| dNTPs (10 mM each) | 1 µl |
| H2O | Up to 13 µl |

5 minutes at 65°C, followed by 1 minute on ice. Then add per reaction:

| | |
|---|---|
| 5x First-strand reaction buffer | 4 µl |
| DTT (0.1 M) | 1 µl |
| H2O | 1 µl |
| SuperScript III RT | 1 µl |

Incubate first at 50°C for 30 minutes, followed by 15 minutes at 70°C.

### Barcode quantification by qRT-PCR

In order to validate the sensor induction during the screen, UIS barcode expression was quantified relative to Rpl13a expression or absolute using a plasmid standard with 1e+02 copies - 1e+05 copies/µl. Primer pairs were qDec1.2/qDec2.2 for the barcode and the plasmid standard and qRT-PCR primer for Rpl13a. Analysis was done using the Qiagen Rotor-Gene Software with the ΔΔCt-method for relative quantification.

| | |
|---|---|
| 2x RotorGene SYBRgreen PCR | 5 µl |
| Master Mix | 1 µl |
| Fwd primer (10 µM) | 1 µl |
| Rev primer (10 µM) | 3 µl |
| cDNA (pre-diluted 1:100) | |

Default qRT-PCR cycling parameters.

### Dec PCR

The Dec PCR amplifies the barcode from the cDNA sample. Prior to the 'Dec PCR', the entire cDNA was purified using the Macherey&Nagel PCR clean-up kit. For each sample, 100 µl reactions were prepared, split into 2x 50 µl reactions for PCR and pooled again afterwards.

| | |
|---|---|
| cDNA (purified) | 10 µl |
| qDec1.2 fwd primer (10 µM) | 1.25 µl |
| qDec2.2 rev primer (10 µM) | 1.25 µl |
| H2O | 37.5 µl |
| NEBNext 2x PCR MasterMix | 50 µl |

The PCR product was confirmed by agarose gel-electrophoresis.

### Code PCR

The "'Code PCR" fuses sample specific 12bp code sequences to the "Dec PCR" product in order to pool samples for next-generation sequencing. The forward code primer contains the Ion-A adapter sequence required for Ion Torrent sequencing and the 12bp code sequence. The reverse primer contains the Ion-P1 adapter sequence required for Ion Torrent sequencing. Code PCR reaction per screen sample:

| | |
|---|---|
| Dec PCR product | 5 µl |
| (pre-diluted 1:10) | |
| Code fwd primer (10 µM) | 0.625 µl |
| Code rev primer (10 µM) | 0.625 µl |
| H2O | 18.75 µl |
| NEBNext 2x PCR MasterMix | 25 µl |

PCR parameters: 98 °C 30 sec, 98 °C 10 sec, 58 °C 30 sec, 72 C 30 sec (10 cycles).

The PCR product was confirmed by agarose gel-electrophoresis.

20-40 µl per sample were pooled subsequently and purified using the NucleoSpin Gel and PCR Clean-up kit (Macherey&Nagel).

### Next-generation sequencing of barcodes

UIS barcode libraries were sequenced on an Ion Torrent Proton sequencer using the PI chip according to the manufacturer's protocols for the Ion PI Template OT2 200 v3 kit (template preparation and enrichment) and for the Ion PI Sequencing 200 v3 kit (sequencing). One PI chip delivered on average 100 million raw reads.

Processing of the raw data was performed using custom shell and R scripts. First, raw reads were split into individual samples using the 12 bp code and subsequently mapped to a reference barcode library using a local BLAST. Thereby, reads were counted and assigned to shRNAs and gene targets. Next, read counts were normalized to total read numbers per sample. If multiple barcodes are assigned to the same shRNA, corresponding read counts were summed. To control the correlation between replicates, similarities between all samples were estimated using pair-wise Pearson correlation coefficient and plotted as a heatmap with hierarchical clustering. Analysis was then continued by enhanced Z-score ranking or using the DESeq2 R package (Love MI et al. Genome Biol. 2014;15(12):550).

For the enhanced Z-score analysis, normalized read counts of replicates were collapsed to mean count values and log2 transformed. Log2 ratios were calculated between stimulated and silenced samples and normalized to enhanced Z-scores. In order to collapse to gene level, the barcode/shRNA with the strongest effect towards the positive- (for negative regulators) or negative direction (for positive regulators) was selected to represent a certain gene.

The DESeq2 package allows testing for differential expression of a gene or in this case of a barcode. Therefore, normalized read count data with all replicates for the stimulated and silenced conditions was first processed using the DESeqDataSetFromMatrix() function. Next, data was analyzed using the DESeq() function which includes the Wald test for differential expression and correction by multiple testing using the Benjamini-Hochberg method (Benjamini and Hochberg, 1995).

### Example 2.13 Cloning individual shRNAs and sgRNAs

In order to validate screen results, selected shRNAs from the library were individually cloned into the AAV E-SARE PATHscreener vector (V1301).

Pairs of oligonucleotides were synthesized by Eurofins with Agel and EcoRI-compatible overhangs and annealed in 10x T4 DNA ligase buffer (NEB). The annealed oligonucleotides were diluted to 5 ng/µl and ligated into the linearized AAV E-SARE PATHscreener vector.

shRNA oligonucleotide pairs were designed as followed:
Forward: 5'-CCGG-shRNA forward sequence-3'
Reverse: 5'-AATT-shRNA reverse sequence-3'

Cloning of sgRNAs into the linearized PATHscreener2.0 vector was perfomred using the same protocol as for shRNAs. For linearization, the PATHscreener2.0 vector was digested with Bbsl, creating overhangs for the ligation.

sgRNA oligonucleotide pairs were designed as followed:
Forward: 5'-ACCG-sgRNA target sequence-3'
Reverse: 5'-AAAC-sgRNA reverse target sequence-3'

### Example 3: Results and Discussion

Until now the majority of pooled functional genetic screens (RNAi or CRISPR-Cas9) have been performed in oncology (Diehl P et al. Drug Discov Today Technol. 2014 Mar;11:11-8 and Shalem O et al. Nat Rev Genet. 2015 May;16(5):299-311). This has an obvious impact on existing protocols and plasmid libraries. In oncology, the general goal is to screen for modifiers of cell proliferation/survival. To achieve the desired segregation of "hits" from "non-hits" in terms of cell numbers within the total cell population, long cultivation periods over multiple passages are required. This implies the use of lentiviral libraries (shRNA or sgRNA) to generate stable integrations in the genome of the mitotic cell line of interest (Rubinson DA et al. Nat Genet. 2003 Mar;33(3):401-6 and Stewart SA et al. RNA. 2003 Apr;9(4):493-501). The long selection procedure in order to obtain a strong phenotype finally allows isolating the genomic DNA and counting the number of integrations per shRNA by next-generation sequencing. An alternative approach involves the use of reporter cell lines and flow cytometry as a strategy to segregate different phenotypes at the endpoint of the screen and to focus the readout to cellular functions other than proliferation/survival (Means and methods for identifying "hits").

Existing protocols cannot readily be applied to post-mitotic cell types such as primary neurons. Standard cell culture protocols for primary mouse neurons allow cultivation for not more than a few weeks and viability usually declines after two weeks in culture. Thus, the time window to produce a strong cell survival phenotype is relatively short. Nevertheless, it would be of great interest to screen for modifiers of cell survival neurodegenerative diseases.

The present inventors aimed to combine a pooled shRNA screen with a sensitive and robust genetic sensor. This sensor should consist of an optimized synthetic promoter which regulates the expression of a RNA barcode upon pathway activation. The use of a "barcoded" genetic sensor has two major advantages over currently used readout options for pooled interference screens (RNAi or CRISPR-Cas9, Figure 1 and Figure 17): (1) It allows to measure cellular phenotypes other than proliferation/survival which is the case if only the bare shRNA pool complexity is analyzed. (2) It is independent of cell sorting based on a fluorescent reporter and therefore a direct quantitative readout. In order to achieve the combined approach of a pooled RNAi screen with a pathway activity readout, two libraries need to become one, the shRNA library and the sensor-coupled molecular barcode library. Each barcode within this library is controlled by the same genetic sensor and codes for an shRNA that is expressed from the same AAV vector (Figure 7A). The corresponding vector is hereafter named PATHscreener vector.

The screen itself is based on the simplified hypothesis that a stimulus (e.g. receptor agonist) triggers a signaling cascade which induces the "barcoded" sensor and that interference (by shRNAs) within this pathway leads to an altered sensor induction compared to shRNAs that are not targeting the pathway (Figure 7B). Sensor activities are finally measured by counting of the corresponding RNA barcodes using NGS. The screen is based on the assumption that each cell is only infected once in order to avoid cross-contamination of multiple shRNAs and barcodes.

The present inventors first evaluated changes in the abundance of genomic DNA and total RNA in response to cytotoxic stress within a primary neuron population. Therefore, neuronal cell cultures where treated with the electron transport chain inhibitor rotenone at different concentrations for 7 days and cell number, genomic DNA and total RNA were quantified.

Surprisingly, the total nuclei count was only modestly reduced even at high rotenone concentrations (>100nM), although quantification of viable cells, by filtering out all pyknotic nuclei, revealed the toxicity of rotenone with an IC50 of 14 nM (Figure 3A). This already indicated that an analysis based on a DNA barcode readout might lack the required sensitivity in a cell viability paradigm.

In accordance to the cell count, the present inventors observed a reduction of genomic DNA content by ∼40% at rotenone concentrations above 100nM (IC50 40 nM). In contrast, changes in total RNA content with an IC50 (15 nM) were more sensitive and exhibited a higher dynamic range (Figure 3B). Taken together, this indicated that without long-term culturing including cell passaging, a segregation of viable and non-viable cell pools based on a DNA reporter is not feasible.

Rather, RNA-based barcode readouts seem to be preferable in many (post-mitotic) cell types, such as primary neurons.

A second modification to current protocols was the viral system employed for transduction of primary neurons for a pooled RNAi screen. Post-mitotic cells such as primary neurons do not require integration of the shRNA library for stable long-term transgene expression. Since the readout does not require stable barcode integration as well, Adeno-associated virus (AAV) were used for transduction. This has several practical and experimental advantages. AAVs require less safety precautions and are structurally more robust than lentiviruses (Bouard D et al. Br J Pharmacol. 2009 May;157(2):153-65). More importantly, however, is that AAVs produced with certain capsid serotypes (e.g. serotype 1 and 2) have a natural tropism for neuronal cells and do not trigger a cellular immune response (McCown TJ Curr Gene Ther. 2005 Jun;5(3):333-8). Within the cell the AAV genome preferentially persists extrachromosomally (Nakai H et al. J Virol. 2001 Aug;75(15):6969-76). This might reduce the risk of integration locus effects on shRNA and UIS barcode expression and eliminates the possibility that coding or regulatory regions in the genome are destroyed due to integration (Moiani et al. J Clin Invest. 2012 May;122(5):1653-66).

### Example 3.1: Identification of a genetic sensor

In order to identify a genetic sensor with a wide dynamic range upon synaptic stimulation, a multiplexed *cis*-regulatory sensor assay was performed. Each sensor within this assay either consists of a clustered transcription factor binding sites (*cis*-element) coupled to a minimal adenoviral major late promoter (minMLP) or a ∼1 kb promoter fragment (Figure 4A). During the assay, sensors are driving the expression of unique molecular RNA barcodes (UIS) in response to biological events. UIS barcode transcripts are finally isolated and decoded by NGS in order to measure sensor activities (Figure 4B).

For identification of a synaptic activity sensor, primary cortical neurons were infected with the AAV pool of 70 sensor vectors and the sensor activities were measured at DIV14 under neuronal silencing conditions, basal activity (i.e. untreated), and synaptic stimulation.

Silencing of neuronal activity was achieved by treating cells with the voltage-gated sodium channel inhibitor tetrodotoxin (TTX) in combination with the NMDAR antagonist D-(-)-2-Amino-5-phosphonopentanoic acid (APV) (McLennan H. Eur J Pharmacol. 1981 Jul 17;73(1):97-9 and Narahashi T et al. Science. 1966 Aug 12;153(3737):765-7). Neuronal activity of the culture was triggered by blocking inhibitory synaptic transmission using the GABAA receptor antagonist bicuculline (BIC). In all experiments, BIC has been supplemented with the potassium channel blocker 4-Aminopyridine (4-AP), the NMDAR co-agonist glycine, and the glycine receptor antagonist strychnine (Curtis DR et al. Nature. 1970 Jun 27;226(5252):1222-4. and Meves H and Pichon Y.J Physiol. 1975 Sep;251(1):60P-62P). This stimulation cocktail is referred to as BIC/4-AP.

Multiple sensors qualified as potential synaptic activity reporters as they exhibited reduced activity in silenced neurons and increased UIS barcode expression after synaptic stimulation (Figure 4C). The largest dynamic range between synaptic silencing and stimulation was measured for a sensor consisting of an enhancer from the murine *Arc* promoter, called synaptic activity-responsive element (SARE) (Kawashima T et al. Proc Natl Acad Sci USA. 2009 Jan 6;106(1):316-21). The SARE sensor outperformed classical neuronal activity reporters such as the endogenous promoters of *Fos* and *Egr1* (Figure 4C).

The SARE enhancer is a ∼100 bp sequence localized >6 kb upstream of the Arc gene and contains binding sites for three activity-dependent transcription factors (CREB, MEF2 and SRF) (Figure 5). The Arc gene is expressed in response to neuronal activity and ARC protein is enriched at the post-synaptic density of dendritic spines and exerts functions during synaptic plasticity via regulation of AMPA receptor endocytosis (Chowdhury S et al. Neuron. 2006 Nov 9;52(3):445-59; Plath N et al. Neuron. 2006 Nov 9;52(3):437-44 and Shepherd JD et al. Neuron. 2006 Nov 9;52(3):475-84). Kawashima and colleagues identified and characterized the SARE enhancer and evaluated its sensitivity to synaptic stimulation (Kawashima T el al. Proc Natl Acad Sci U S A. 2009 Jan 6;106(1):316-21).

A genome-wide bioinformatic search for SARE-like sequences revealed that this arrangement of transcription factor binding sites is not unique for the Arc promoter, but is found in promoter regions of many other neuronal activity-dependent genes (Rodríguez-Tornos FM et al. PLoS One. 2013;8(1):e53848). During the development of the sensor pool for the *cis-*regulatory assay, the SARE sensor was optimized for highest signal-to-noise ratio by clustering of multiple SARE repeats in front of a 420bp minimal Arc promoter (ArcMin), similar to work by Kawashima and colleagues (Kawashima T et al. Nat Methods. 2013 Sep;10(9):889-95). Sensors with one, three, four, five, and six SARE repetitions were tested for a maximal dynamic range in the neuronal cell line SH-SY5Y upon stimulation with PMA (phorbol-12-myristat-13-acetat). A cluster of four SARE repetitions gave highest fold inductions and was therefore used in all subsequent experiments (Figure 6A).

This composite promoter is hereafter named enhanced SARE (E-SARE) sensor, in line with the nomenclature used by Kawashima and colleagues, although their construct contains five SARE repetitions. Next, the E-SARE sensor was further characterized in primary cortical neurons. Longitudinal recordings of E-SARE-driven luciferase activity from untreated, maturing primary neurons revealed that the baseline E-SARE activity recapitulates the course of increasing synaptogenesis, as determined by stainings of the presynatic marker synaptophysin in maturing cultures (Figure 6B). The kinetics also match the staging established by Baj and colleagues (Baj G et al. Front Cell Neurosci. 2014 Feb 5;8:18).

Finally, the E-SARE sensor not only responds to neuronal silencing (TTX/APV) and synaptic stimulation (BIC/4-AP), but can also be induced by brain-derived neurotrophic factor (BDNF) which is a well-established stimulus with functions in neuronal differentiation and plasticity (Figure 6C) (Park H and Poo M. Nat Rev Neurosci. 2013 Jan;14(1):7-23). The optimized and validated E-SARE sensor has been subsequently used for developing the pooled RNAi screen in primary neurons.

### Example 3.2 Vector design for sensor-based RNAi screenings

### shRNA expression cassette

In order to compare the impact of different promoters on RNAi efficiency, knockdown of a luciferase reporter by five different shRNAs driven by the hU6 promoter (hU6p) or the DNA polymerase II promoters of synapsin-1 (Syn1p) or neuron-specific enolase (NSEp) was evaluated (Figure 8A).

Overall, the hU6p-driven shRNAs exhibited superior knockdown efficiencies. In particular when shRNA efficiency is suboptimal, the hU6 promoter demonstrates its impressive strength to boost the knockdown (Figure 8A, shRNA#4).

Moreover, only the hU6 promoter-mediated RNAi achieved knockdown efficiencies above 90% in this test. Hence, the hU6 promoter was selected to drive the expression of the shRNA library for screening.

In order to transduce primary neurons with the sensor-coupled shRNA library, adenovirus-associated vectors (AAV) were employed. AAVs have the advantage of exhibiting a natural tropism for neurons and does not evoke any cellular immune response. Stability of expression of shRNAs and transgenes in primary neurons over time was determined by infecting the cells with AAVs expressing GFP and a shRNA against Tcf4 and analyzing GFP fluorescence, and Tcf4 mRNA abundance at various time points (Figure 8B and C). Knockdown of the Tcf4 mRNA was first detectable at four days post-infection (DIV4), which corresponds to the time point of maximal AAV (serotype 1/2) replication. The knockdown was increasing and remained stable until DIV14. In accordance, GFP expression was first visible at two days post-infection (DIV2) and increases until it remains stable over the entire time course (Figure 8C). This indicates that the AAV system is well-suited for screening.

### Combining sensor and shRNA expression

In order to determine whether the UIS-barcoded E-SARE sensor and the shRNA expression cassette sensor function in parallel and in close proximity if cloned into the same AAV backbone, the E-SARE induction upon PMA stimulation was determined in a luciferase assay with the complete AAV PATHscreener vector (containing E-SARE sensor and hU6p) or vectors where either the E-SARE sensor (w/o sensor) or the hU6p-shRNA cassette (w/o hU6p-shRNA) were deleted.

Comparison of the complete PATHscreener vector with the hU6p-shRNA deletion vector showed that the sensor is not compromised by the hU6 promoter (Figure 9A). Both vectors generated similar luciferase activities at baseline as well as upon stimulation by PMA.

Uniform sensor inductions across different constructs with variable barcode and shRNA sequences are an essential requirement for the pooled RNAi screen. To this end, 10 constructs with different UIS barcodes and shRNA sequences were cloned. E-SARE induction for each clone was tested by PMA stimulation in rat PC12 cells to minimize the risk of a true shRNA effect on the sensor activity. Overall, the inductions across all clones were similar (Figure 9B). None of the clones deviated more than three median absolute deviations (MAD) from the median, which is a frequently used hit criterion in high-throughput screens (Birmingham A et al. Nat Methods. 2009 Aug;6(8):569-75.).

### Library cloning strategy

A high quality shRNA library is the basis for successful pooled RNAi screenings. Libraries can be custom-made by high-throughput synthesis of shRNA oligonucleotides or purchased from various suppliers. Libraries can be either genome-wide or focused on specific groups of genes. Parameters affecting the choice of a genome-wide or focused approach include the biological event to be elucidated and the cell number required for robust results. Generally, a cell number to shRNA complexity ratio of 200-1000:1 is favorable for robust screenings. For primary cells, cell numbers are often limited -which suggests the use of a focused library format. The present inventors aimed to screen for regulators of neuronal excitation and synapse-to-nucleus signalling. Hence the employed library was chosen with a focus on signaling pathway genes in general. Such a library is available from Cellecta (as part of the Decipher project (http://www.decipherproject.net/)). The Decipher Mouse Modul 1 (MM1) shRNA library covers 4625 genes that were selected based on expert-curated pathway databases like KEGG and Reactome, the CSHL Cancer 1000 List, the Cancer Genome Atlas, FDA drug targets and MeSH. With nearly 5000 gene targets, the library is well-suited for pooled RNAi screenings in primary neurons. The applied cloning strategy for introducing the shRNA library into the vectors of choice requires the following steps (Figure 10A): (1) PCR amplification of the hU6p-shRNA library cassette from the original shRNA plasmid library and simultaneous fusion of a minimal SpA (Levitt N et al. Genes Dev. 1989; 3, 1019-1025). The SpA will finally be part of to the sensor expression cassette, (2) Fusion of the shRNA library PCR product with an oligonucleotide library containing random UIS barcode sequences and amplification by PCR, (3) Large-scale ligation of the shRNA-barcode library insert into the sensor containing AAV backbone, (4) Sequencing of the vector region spanning the shRNA and the barcode in order to assign barcode sequences to shRNAs/gene targets (Figure 10B). The Ion Torrent PGM employed for deep-sequencing of the final library can be used to sequence fragments up to ∼400bp. Thus, the shRNA and UIS barcode must be in close proximity -and as a consequence both expression cassettes are facing towards each other- and the SpA was selected as one of the smallest available poly-adenylation signals (Levitt N et al. Genes Dev. 1989; 3, 1019-1025). During library cloning, 12 individual clones were isolated and analyzed by restriction digest and Sanger sequencing. All clones passed the restriction digest, only one clone contained a mutated insert and each clone had a unique shRNA and UIS barcode sequence. This confirmed that the protocol is delivering reliable cloning products. The cloned library contains ∼25,000 unique barcodes which code for ∼13,000 different shRNAs covering ∼4500 genes. Hence, after library repurposing ∼97% of target genes are covered. An shRNA complexity of ∼13,000 allows to perform a screen in a cell culture dish with 10 million cells. With an infection rate of 60%, ∼500 cells would be infected per shRNA on average. The barcode to shRNA ratio of ∼2:1 results from a 2:1 ratio between UIS barcode oligo and hU6p-shRNA-SpA fragment during PCR#2. This suggests that the cloning strategy by PCR is well customizable -a feature which renders the present cloning strategy superior to commonly applied library cloning protocols. Such protocols typically require a barcoded backbone vector library into which an enhancer library is cloned. This usually results in higher numbers of different UIS barcodes per enhancer. The generated E-SARE-shRNA library was successfully packaged into AAV particles (serotype 1/2) and subsequently used for screenings in primary neuron cultures.

### Sensor-based pooled RNAi screen in primary neurons

In order to broaden the studies cell types and biological events or processes towards relevant psychiatric risk pathways in primary neurons, a new barcoded genetic sensor readout was developed. Using an AAV library that contains the E-SARE sensor and a focused shRNA library of ∼4500 signaling pathway genes, the present inventors performed the first pooled RNAi screen in primary neurons. The screen successfully used to identify genes involved in neuronal excitation and synpase-to-nucleus signaling.

Two screens (A and B) were performed applying the same protocol, using 10 million cells (A) and 5 million cells (B) per sample, respectively. Each condition had 2-3 biological replicates. Cortical neurons were isolated from E15.5 wild-type mice and infected on DIV6 with the AAV pool at an AAV particle to cell ratio of 1000:1 (∼60% infection rate). On DIV10 half of the samples were treated with TTX and APV to silence the spontaneous neuronal network activity and thereby reduce the E-SARE activity to baseline. The remaining samples were stimulated with a cocktail containing bicuculline, 4-AP, glycine and strychnine (BIC/4-AP) on DIV12 to boost synaptic activity (Figure 11). The RNA harvest time point for a maximal dynamic range was at 4 hours after BIC/4-AP application (Figure 12A). The kinetics of the RNA-based reporter is thus faster compared to the luciferase reporter which peaks at 8 hours after BIC/4-AP application (Figure 12B). All samples, silenced and stimulated, were subsequently lysed for total RNA purification. The sensor response was controlled by qRT-PCR using barcode flanking primers (qDec primers). Relative and absolute quantification of the barcode cDNA using qDec primers verified the E-SARE stimulation upon BIC/4-AP during the screen (Figure 12C and D). UIS barcode libraries were prepared for deep-sequencing on an Ion Torrent Proton sequencer. It was hypothesized that the induction of a barcode, which codes for a "hit" shRNA, would be reduced or increased compared to the median induction of the total library. For "hit" nomination, two analysis methods were applied: (1) Ranking by enhanced Z-scores of normalized fold changes between stimulated and silenced conditions and (2) a protocol analogous to differential gene expression analysis in RNA-seq using the DESeq2 package in R (Dai Z et al. F1000Res. 2014 Apr 24;3:95 and Parnas O et al. Cell. 2015 Jul 30;162(3):675-86 and Love MI et al. Genome Biol. 2014;15(12):550).

Data analysis and ranking of shRNAs and gene targets has been done by two independent methods. The knockdown effects on the E-SARE sensor activity were analyzed by an enhanced Z-score ranking and using the Bioconductor package DESeq2 (Love MI et al. Genome Biol. 2014;15(12):550). Since absolute abundances of individual vectors vary within the library pool, UIS barcode counts in the stimulated samples are typically normalized to the barcode counts in the unstimulated reference samples (Figure 11). Both methods are based on the assumption that the majority of shRNAs within the library do not alter the induction of the E-SARE sensor in response to synaptic stimulation by BIC/4-AP. This is particularly the case for shRNA libraries of high complexity, as in this case, as supported by the analysis of 10 random shRNA constructs (Figure 10B).

The enhanced Z-score method analyzes the effect of a knockdown by reporting the deviation of the corresponding sensor activity from the median sensor activity of the entire pool. The advantage of the enhanced Z-score is its robustness against outliers which in this case are the hits. A negative enhanced Z-score of a shRNA means that the target gene is a positive regulator of the measured phenotype, whereas a positive enhanced Z-score identifies genes that act as negative regulators. Sensor activity in the screen can be shifted in general towards both directions, hence, positive and negative regulators can be identified. However, more shRNAs have a negative than a positive enhanced Z-score above the thresholds of 3/-3 (209 shRNAs at enhanced Z-score < -3; 111 shRNAs at enhanced Z-score > 3) and the overall amplitude is stronger at the negative scale (Figure 13A, left). Thus, the screen appears to be partiuclarly sensitive for positive regulators, which is likely due to the strong stimulation applied during the screen. In order to collapse the hit list to the gene level, the shRNA with the strongest effect was selected to represent the corresponding gene target. At this level, 151 genes have an enhanced Z-score of less than -3 (Figure 13A, right) and can be considered as primary hits for positive regulators.

A second powerful analysis strategy uses the Bioconductor R package DESeq2 (Love MI et al. Genome Biol. 2014;15(12):550). This package was originally developed for the identification of differentially expressed genes in RNA-seq experiments. Nevertheless, it has been shown that the DESeq2 package as well as the similar edgeR package are also powerful tools for hit nomination, as genetic screening data and RNA-seq data are very similar and have a negative binominal distribution (Dai Z et al. F1000Res. 2014 Apr 24;3:95 and Parnas O et al. Cell. 2015 Jul 30;162(3):675-86). In this study, DESeq2 was used for read count normalization between samples and identification of differentially expressed barcodes. Significance of differential expression was tested using the Wald test and corrected by multiple-testing using the Benjamini-Hochberg method (Benjamini Y and Hochberg Y J. R. Stat. Soc. Ser. B Methodol. 1995; 57: 289-300). In accordance with the results from the enhanced Z-score analysis, the DESeq2 analysis identified more shRNAs where the sensor induction is significantly down-regulated (335 at a false discovery rate (FDR) < 0.05) compared to shRNAs with an enhanced sensor induction (250 at FDR < 0.05) (Figure 13B). Hit lists generated by both methods show a substantial overlap (Enh. Z-score vs. DESeq2 log2FC 54/100; vs. DESeq2 FDR 41/100) (Figure 13C) and the use of both strategies in parallel can increase the confidence in "hit" nomination. The following steps of analysis are based on the enhanced Z-score ranking unless otherwise stated.

Next, 10 candidates for positive regulators were selected from screen A, covering an enhanced Z-score range from -2 to -10 as indicated in figure 13A (right, red dots). These candidates are components of cAMP signaling (Adcy3), cytokine signaling (Il2rb), calcium signaling (Calm1, Camk2d, Cacnalf, Cacna1h, Tacr2), the circadian clock (Bhlhe40), the postsynaptic density (Gphn), and a schizophrenia risk gene (Disc1). They were selected to test the first step of validation by performing individual knockdown experiments. For each gene the shRNA with the strongest effect in the screen was cloned into the PATHscreener vector and tested individually for its interference in BIC-induced synaptic signaling (Figure 14). All tested shRNAs reduced the E-SARE induction compared to a non-targeting control (NTC) vector and 8 out of 10 shRNAs showed a significant effect (student's t-test, p < 0.05). While this is the first validation step, additional orthogonal validation tools (e.g. synapse/dendrite stainings, electrophysiology) need to be implemented into the protocol and high-content analysis of hit candidates might be a powerful strategy.

### Example 3.3 Adaptation towards CRISPR-Cas9-based screening in primary neurons

Based on the acquired expertise from the proof-of-concept pooled RNAi screen in primary neurons, the adaptation towards a CRISPR-Cas9-based screening tool has been developed. Transcriptional regulation by CRISPR-Cas9 relies on the recruitment of effector domains (e.g. p65, VP64 activator domains) into proximity of the transcriptional start site of the gene-of-interest. It was shown that recruitment of multiple domains to the same locus enhances activation of gene expression (Konermann S et al.Nature. 2015 Jan 29;517(7536):583-8 and Tanenbaum ME et al. Cell. 2014 Oct 23;159(3):635-46). The present inventors therefore decided to use the CRISPR-Cas9-SAM (Synergistic Activation Mediator), that recruits multiple effector domains using a fusion of the sgRNA with two MS2 aptamer sequences and a second fusion of the MS2-binding domain with the effector domains (e.g. p65 and VP64) (Figure 16A). This increases the number of effector domains at the locus compared to a direct fusion of the effector domain to dCas9. The screen can be performed either by co-infection of a Cas9 vector and the sgRNA library or by the infection of the sgRNA library into cells that stably express Cas9 (e.g. primary neurons from Cas9 transgenic mice) (Figure 17). AAV vectors were cloned for dCas9 (*Streptococcus pyogenes*) expression and two vectors represent the PATHscreener2.0 vectors for CRISPRa and CRISPRi with the following modifications to the RNAi-based PATHscreener vector: (1) the firefly luciferase was substituted by the shorter NanoLuc luciferase, (2) the shRNA was substituted by the sgRNA2.0 and (3) a third expression cassette was introduced for expression of MS2-p65-VP64 or MS2-KRAB-SID4X for CRISPRa and CRISPRi, respectively (Figure 16B, PATHscreener2.0).

Multiple experiments were performed to validate the CRISPRa PATHscreener2.0 vector. Expression of dCas9 and MS2-p65-VP64 from the AAV backbone was verified by western blot (Figure 16C). Stimulation of the E-SARE sensor within the PATHscreener2.0 backbone by PMA in HEK293 cells showed comparable inductions as with the RNAi-based PATHscreener vector (Figures 9A and 16D). The system is able to induce expression of a luciferase reporter as well as endogenous Arc mRNA (Figure 16E and F). Primary neurons express both dCas9 and MS2-p65-VP64 after AAV infection, even though AAV-dCas9 genome slightly exceeds the optimal AAV packaging capacity of 4.8 kb. Importantly, both proteins are localized in the nucleus (Figure 16G).

### Example 3.3.1: CRISPRa/i PATHscreener2.0 library cloning strategy

Cas9 protein in complex with sgRNAs with a 15 bp instead of 20 bp target sequence binds the DNA without DNA cleavage activity (Dahlman JE et al. Nat Biotechnol. 2015 Nov;33(11):1159-61). Such sgRNAs are referred to as dead sgRNAs. Thus, wtCas9 protein (in combination with dead sgRNAs) can be employed for CRISPRa and CRISPRi. The PATHscreener2.0 libraries were designed with dead sgRNAs targeting a region of 200 bp upstream or downstream of the transcription start site (TSS) for CRISPRa and CRISPRi, respectively (Figure 18). Each gene-of-interest is targeted by multiple sgRNAs. For library construction, sgRNA oligonucleotides were synthesized and fused to a random barcode library by PCR similar to the RNAi library cloning strategy (Example 2.10). The insert pool consisting of the dead sgRNAs and the UIS barcodes were subsequently cloned into the PATHscreener2.0 vector which contains the E-SARE sensor and sgRNA - barcode assignment has been performed by sequencing as described previously for shRNAs (Example 2.12)

### Example 3.3.2: Sensor-based pooled CRISPR-Cas9 screen in primary neurons

For a proof-of-concept, primary cortical neurons were isolated from Cas9 transgenic mice (Platt RJ et al. Cell. 2014 Oct 9;159(2):440-55 and Minichiello L et al. Neuron. 1999 Oct;24(2):401-14) and infected either with the CRISPRa or CRISPRi AAV PATHscreener2.0 library pool. The cultures were further incubated to allow the Cas9/sgRNAs/MS2-effector domain complexes to exert their gene regulatory function on the target genes. In order to identify genes involved in neuronal excitability and synapse-to-nucleus signaling, cultures were treated with TTX and APV to silence the spontaneous neuronal network activity and thereby reduce the E-SARE activity to baseline, or with the BIC/4-AP cocktail to stimulate synaptic activity (Figure 17). At the peak of E-SARE induction, total RNA was harvested from the cultures and the barcode libraries were amplified and prepared for deep sequencing. For each sensor-coupled barcode the ratio between stimulated test sample and the silenced reference sample was calculated to measure the effect of the corresponding sgRNA. Activation or inhibition of gene expression of a target gene involved in neuronal excitability and synapse-to-nucleus signalling via CRISPRa or CRISPRi thus leads to a change in E-SARE sensor induction. Furthermore, it is expected that the opposing regulation of a "hit" gene by CRISPRa and CRISPRi might also be reflected by an opposing sensor response.

## Claims

1. A polynucleotide comprising:
(a) a sensor expression cassette comprising
(i) a sensor transcription control element operably linked to
(ii) a unique identifier sequence providing an RNA oligonucleotide;
and
(b) an effector expression cassette comprising
(i) a sequence providing an effector expression product;
wherein
said unique identifier sequence specifically identifies said polynucleotide.

2. The polynucleotide according to claim 1, wherein said effector expression product is capable of interfering with a target gene and/or wherein said sensor transcription control element is capable of being regulated by a target gene.

3. The polynucleotide according to claim 1 or 2, wherein said effector expression product is selected from a protein, a peptide, an aptamer, or a nucleic acid.

4. The polynucleotide according to any one of the preceding claims, wherein said effector expression product is an RNAi-inducing agent or a precursor thereof.

5. The polynucleotide according to any one of claims 1 to 3, wherein said effector expression product is a gene editing agent, preferably a gene editing agent capable of specifically inserting, deleting or replacing said target gene from or into the genome of said host cell.

6. The polynucleotide according to any one of claims 1 to 3 or 5, wherein said effector expression product is a guide (sg) RNA, selected from the group of a single-guide (sg) RNA, crRNA/tracrRNA or dead(d)RNA, and wherein said polynucleotide further optionally encodes a CRISPR-associated endonuclease (Cas), preferably selected from Cas9 or a fragment, variant or derivative thereof.

7. A vector comprising the polynucleotide according to any one of claims 1 to 6.

8. A host cell comprising the polynucleotide according to any one of claims 1 to 6 and/or the vector according to claim 7.

9. A library of polynucleotides according to any one of claims 1 to 6, said library comprising at least two distinct polynucleotides, wherein each of the polynucleotides in the library comprises:
(a) a sensor expression cassette comprising
(i) a sensor transcription control element, operably linked to
(ii) a unique identifier sequence providing an RNA oligonucleotide;
and
(b) an effector expression cassette comprising
(i) a sequence providing an effector expression product;
wherein
each polynucleotide comprises either a unique combination of a unique identifier sequence and a distinct effector expression product, or a unique combination of a unique identifier sequence and a distinct sensor transcription control element.

10. A method for detecting at least one or more target gene(s) capable of regulating a given sensor transcription control element comprising the following steps:
(1) introducing into a plurality of host cells a library according to claim 9;
(2) providing a test sample from said host cells, said test sample comprising RNA oligonucleotides, wherein each RNA oligonucleotide is transcribed from a distinct unique identifier sequence comprised by a polynucleotide of the library;
(3) quantifying each RNA oligonucleotide in said test sample to determine a "sensor response" for each polynucleotide of the library;
wherein a "sensor response" that deviates from the median "sensor response" of all polynucleotides indicates that the effector expression product encoded by a given polynucleotide interferes with a target gene capable of regulating the sensor transcription control element of interest.

11. The method according to claim 10, further comprising prior to step (3) a step (2a) of subjecting part of the host cells to a test condition, said host cells yielding the test sample, and/or prior to step (3) a step (2a') of subjecting part of the host cells to a reference condition, said host cells yielding a reference sample.

12. The method according to claim 10 or 11, further comprising a step (3') quantifying each RNA oligonucleotide in said reference sample to determine a sensor response for each polynucleotide.

13. The method according to claim 12, further comprising a step (4) of calculating a "sensor response ratio" for any given polynucleotide by dividing the quantity of each RNA oligonucleotide in the test sample by the quantity of the corresponding RNA oligonucleotide in the reference sample, wherein a "sensor response ratio" that deviates from the median "sensor response ratio" of all polynucleotides is indicative of an effect of the target gene targeted by the effector expression product encoded by said polynucleotide.

14. The method according to any one of claims 10 to 13, wherein said test conditions and/or said reference conditions are selected from an extracellular or intracellular stimulus; optionally selected from a chemical or physical stimulus or a combination thereof; an intrinsic modification optionally selected from a genome or proteome or epigenome modification; or a combination thereof.

15. A method for determining the effect of a given target gene on at least one or more sensor transcription control element(s) comprising the following steps:
(1) introducing into a plurality of host cells a library according to claim 9;
(1') introducing into a plurality of host cells a corresponding library according to claim 11; wherein said effector expression product provided by the polynucleotides of said library are not capable of interfering with a target gene;
(2) providing a test sample from said host cells of step (1);
(2') providing a reference sample from said host cells of step (1');
said test sample and said reference sample comprising RNA oligonucleotides transcribed from said polynucleotides of each library, wherein each RNA oligonucleotide is transcribed from a unique identifier sequence which specifically identifies its polynucleotide;
(3) quantifying each RNA oligonucleotide in said test sample to determine a test sample "sensor response" for each polynucleotide of the library;
(3') quantifying each RNA oligonucleotide in said reference sample to determine a reference sample "sensor response" for each polynucleotide of the library;
wherein a "sensor response" in a test sample for any given polynucleotide that deviates from the corresponding "sensor response" in the reference sample is indicative of an effect of the target gene on the sensor transcription control element comprised by said polynucleotide.

16. A kit comprising a polynucleotide according to any one of claims 1 to 6, a vector according to claim 7, or a host cell according to claim 8 and means for detecting and/or quantifying the expression of RNA oligonucleotides.

## Patentansprüche

1. Ein Polynukleotid, umfassend:
(a) eine Sensorexpressionskassette, umfassend
(i) ein Sensor-Transkriptionssteuerungselement, das operabel verbunden ist mit
(ii) eine(r) eindeutige(n) Identifizierungssequenz, die ein RNA-Oligonukleotid bereitstellt;
und
(b) eine Effektorexpressionskassette, umfassend
(i) eine Sequenz, die ein Effektorexpressionsprodukt bereitstellt;
wobei
die eindeutige Identifizierungssequenz das Polynukleotid spezifisch identifiziert.

2. Polynukleotid nach Anspruch 1, wobei das Effektorexpressionsprodukt in der Lage ist, mit einem Zielgen zu interferieren und/oder wobei das Sensor-Transkriptionssteuerungselement in der Lage ist, durch ein Zielgen reguliert zu werden.

3. Polynukleotid nach Anspruch 1 oder 2, wobei das Effektorexpressionsprodukt ausgewählt ist aus einem Protein, einem Peptid, einem Aptamer oder einer Nukleinsäure.

4. Polynukleotid nach einem der vorhergehenden Ansprüche, wobei das Effektorexpressionsprodukt ein RNAi-induzierendes Mittel oder ein Vorläufer davon ist.

5. Polynukleotid nach einem der Ansprüche 1 bis 3, wobei das Effektorexpressionsprodukt ein Gen-Editierungsagens ist, vorzugsweise ein Gen-Editierungsagens, das in der Lage ist, das Zielgen aus oder in das Genom der Wirtszelle spezifisch einzubringen, zu deletieren oder zu ersetzen.

6. Polynukleotid nach einem der Ansprüche 1 bis 3 oder 5, wobei das Effektorexpressionsprodukt eine Führungs(sg)-RNA ("guide RNA") ist, ausgewählt aus der Gruppe einer Single-Guide(sg)-RNA, crRNA/tracrRNA oder dead(d)RNA, und wobei das Polynukleotid ferner optional für eine CRISPR-assoziierte Endonuklease (Cas) kodiert, vorzugsweise ausgewählt aus Cas9 oder einem Fragment, einer Variante oder einem Derivat davon.

7. Ein Vektor, umfassend das Polynukleotid nach einem der Ansprüche 1 bis 6.

8. Eine Wirtszelle, umfassend das Polynukleotid nach einem der Ansprüche 1 bis 6 und/oder den Vektor nach Anspruch 7.

9. Eine Bibliothek von Polynukleotiden nach einem der Ansprüche 1 bis 6, wobei die Bibliothek mindestens zwei verschiedene Polynukleotide umfasst, wobei jedes der Polynukleotide in der Bibliothek umfasst:
(a) eine Sensorexpressionskassette, umfassend
(i) ein Sensor-Transkriptionssteuerungselement, das operabel verbunden ist mit
(ii) eine(r) eindeutige(n) Identifizierungssequenz, die ein RNA-Oligonukleotid bereitstellt;
und
(b) eine Effektorexpressionskassette, umfassend
(i) eine Sequenz, die ein Effektorexpressionsprodukt bereitstellt;
wobei
jedes Polynukleotid entweder eine einzigartige Kombination aus einer eindeutigen Identifizierungssequenz und einem distinkten Effektorexpressionsprodukt oder eine einzigartige Kombination aus einer eindeutigen Identifizierungssequenz und einem distinkten Sensor-Transkriptionssteuerungselement umfasst.

10. Ein Verfahren zum Nachweis mindestens eines oder mehrerer Zielgens/e, das/die in der Lage ist/sind, ein bestimmtes Sensor-Transkriptionssteuerungselement zu regulieren, umfassend die folgenden Schritte:
(1) Einführen einer Bibliothek nach Anspruch 9 in eine Vielzahl von Wirtszellen;
(2) Bereitstellen einer Testprobe aus den Wirtszellen, wobei die Testprobe RNA-Oligonukleotide umfasst, wobei jedes RNA-Oligonukleotid von einer distinkten eindeutigen Identifizierungssequenz transkribiert wird, die von einem Polynukleotid der Bibliothek umfasst ist;
(3) Quantifizieren jedes RNA-Oligonukleotids in der Testprobe, um eine "Sensorantwort" für jedes Polynukleotid der Bibliothek zu bestimmen;
wobei
eine "Sensorantwort", die von der Median-"Sensorantwort" aller Polynukleotide abweicht, anzeigt, dass das von einem gegebenen Polynukleotid kodierte Effektorexpressionsprodukt mit einem Zielgen interferiert, das in der Lage ist, das interessierende Sensor-Transkriptionssteuerungselement zu regulieren.

11. Verfahren nach Anspruch 10, ferner umfassend vor Schritt (3) einen Schritt (2a), bei dem ein Teil der Wirtszellen einer Testbedingung unterworfen wird, wobei die Wirtszellen die Testprobe ergeben, und/oder umfassend vor Schritt (3) einen Schritt (2a'), bei dem ein Teil der Wirtszellen einem Referenzzustand unterworfen wird, wobei die Wirtszellen eine Referenzprobe ergeben.

12. Verfahren nach Anspruch 10 oder 11, ferner umfassend einen Schritt (3') des Quantifizierens jedes RNA-Oligonukleotids in der Referenzprobe, um eine Sensorantwort für jedes Polynukleotid zu bestimmen.

13. Verfahren nach Anspruch 12, ferner umfassend einen Schritt (4) des Berechnens eines "Sensor-Antwort-Verhältnisses" für ein bestimmtes Polynukleotid durch Division der Menge jedes RNA-Oligonukleotids in der Testprobe durch die Menge des entsprechenden RNA-Oligonukleotids in der Referenzprobe, wobei ein "Sensor-Antwort-Verhältnis", das vom Median-"Sensor-Antwort-Verhältnis" aller Polynukleotide abweicht, auf eine Wirkung des Zielgens hinweist, das durch das Effektorexpressionsprodukt, das durch das Polynukleotid kodiert wird, adressiert wird.

14. Verfahren nach einem der Ansprüche 10 bis 13, wobei die Testbedingungen und/oder die Referenzbedingungen ausgewählt sind aus einem extrazellulären oder intrazellulären Stimulus; optional ausgewählt aus einem chemischen oder physikalischen Stimulus oder einer Kombination davon; einer intrinsischen Modifikation, optional ausgewählt aus einer Genom- oder Proteom- oder einer Epigenom-Modifikation; oder einer Kombination davon.

15. Ein Verfahren zum Bestimmen der Wirkung eines gegebenen Zielgens auf mindestens ein oder mehrere Sensor-Transkriptionssteuerungselemente, umfassend die folgenden Schritte:
(1) Einführen einer Bibliothek nach Anspruch 9 in eine Vielzahl von Wirtszellen;
(1') Einführen einer entsprechenden Bibliothek nach Anspruch 11 in eine Vielzahl von Wirtszellen; wobei das Effektorexpressionsprodukt, das von den Polynukleotiden der Bibliothek bereitgestellt wird, nicht in der Lage ist, mit einem Zielgen zu interferieren;
(2) Bereitstellen einer Testprobe aus den Wirtszellen von Schritt (1);
(2') Bereitstellen einer Referenzprobe aus den Wirtszellen von Schritt (1');
wobei die Testprobe und die Referenzprobe RNA-Oligonukleotide umfassen, die aus den Polynukleotiden jeder Bibliothek transkribiert wurden, wobei jedes RNA-Oligonukleotid von einer eindeutigen Identifizierungssequenz transkribiert wird, die deren Polynukleotid spezifisch identifiziert;
(3) Quantifizieren jedes RNA-Oligonukleotids in der Testprobe, um eine "Sensor-Antwort" einer Testprobe für jedes Polynukleotid der Bibliothek zu bestimmen;
(3') Quantifizieren jedes RNA-Oligonukleotids in der Referenzprobe, um eine Referenzprobe "Sensor-Antwort" für jedes Polynukleotid der Bibliothek zu bestimmen;
wobei eine "Sensor-Antwort" in einer Testprobe für ein gegebenes Polynukleotid, die von der entsprechenden "Sensor-Antwort" in der Referenzprobe abweicht, auf eine Wirkung des Zielgens auf das Sensor-Transkriptionssteuerungselement hinweist, das von dem Polynukleotid umfasst ist.

16. Ein Kit, umfassend ein Polynukleotid nach einem der Ansprüche 1 bis 6, einen Vektor nach Anspruch 7 oder eine Wirtszelle nach Anspruch 8 und Mittel zum Nachweis und/oder zur Quantifizierung der Expression von RNA-Oligonukleotiden.

## Revendications

1. Polynucléotide comprenant :
(a) une cassette d'expression de capteur comprenant
(i) un élément de régulation de transcription de capteur lié de manière fonctionnelle à
(ii) une séquence d'identifiant unique fournissant un oligonucléotide d'ARN ;
et
(b) une cassette d'expression d'effecteur comprenant
(i) une séquence fournissant un produit d'expression effecteur ;
dans lequel
ladite séquence d'identifiant unique identifie spécifiquement ledit polynucléotide.

2. Polynucléotide selon la revendication 1, dans lequel ledit produit d'expression effecteur est apte à interférer avec un gène cible et/ou dans lequel ledit élément de régulation de transcription de capteur est apte à être régulé par un gène cible.

3. Polynucléotide selon la revendication 1 ou 2, dans lequel ledit produit d'expression effecteur est choisi parmi une protéine, un peptide, un aptamère ou un acide nucléique.

4. Polynucléotide selon l'une quelconque des revendications précédentes, dans lequel ledit produit d'expression effecteur est un agent inducteur d'ARNi ou un précurseur de celui-ci.

5. Polynucléotide selon l'une quelconque des revendications 1 à 3, dans lequel ledit produit d'expression effecteur est un agent d'édition de gène, de préférence un agent d'édition de gène apte à insérer, à supprimer ou à remplacer spécifiquement ledit gène cible à partir du génome ou dans le génome de ladite cellule hôte.

6. Polynucléotide selon l'une quelconque des revendications 1 à 3 ou 5, dans lequel ledit produit d'expression effecteur est un ARN guide (sg), choisi dans le groupe d'un ARN guide unique (sg), d'un ARNcr/ARNtracr ou d'un ARNmort(d), et dans lequel ledit polynucléotide code en outre éventuellement pour une endonucléase associée à CRISPR (Cas), de préférence choisie parmi Cas9 ou un fragment, un variant ou un dérivé de celle-ci.

7. Vecteur comprenant le polynucléotide selon l'une quelconque des revendications 1 à 6.

8. Cellule hôte comprenant le polynucléotide selon l'une quelconque des revendications 1 à 6 et/ou le vecteur selon la revendication 7.

9. Banque de polynucléotides selon l'une quelconque des revendications 1 à 6, ladite banque comprenant au moins deux polynucléotides distincts, dans laquelle chacun des polynucléotides de la banque comprend :
(a) une cassette d'expression de capteur comprenant
(i) un élément de régulation de transcription de capteur, lié de manière fonctionnelle à
(ii) une séquence d'identifiant unique fournissant un oligonucléotide d'ARN ;
et
(b) une cassette d'expression d'effecteur comprenant
(i) une séquence fournissant un produit d'expression effecteur ;
dans lequel
chaque polynucléotide comprend soit une combinaison unique d'une séquence d'identifiant unique et d'un produit d'expression effecteur distinct, soit une combinaison unique d'une séquence d'identifiant unique et d'un élément de régulation de transcription de capteur distinct.

10. Procédé de détection d'au moins un ou plusieurs gène(s) cible(s) apte(s) à réguler un élément de régulation de transcription de capteur donné, comprenant les étapes suivantes :
(1) l'introduction dans une pluralité de cellules hôtes d'une banque selon la revendication 9 ;
(2) la fourniture d'un échantillon pour essai à partir desdites cellules hôtes, ledit échantillon pour essai comprenant des oligonucléotides d'ARN, dans lequel chaque oligonucléotide d'ARN est transcrit à partir d'une séquence d'identifiant unique distincte constituée d'un polynucléotide de la banque ;
(3) la quantification de chaque oligonucléotide d'ARN dans ledit échantillon pour essai afin de déterminer une « réponse de capteur » pour chaque polynucléotide de la banque ;
dans lequel une « réponse de capteur » qui s'écarte de la « réponse de capteur » médiane de tous les polynucléotides indique que le produit d'expression effecteur codé par un polynucléotide donné interfère avec un gène cible apte à réguler l'élément d'intérêt de régulation de transcription de capteur.

11. Procédé selon la revendication 10, comprenant en outre, avant l'étape (3), une étape (2a) consistant à soumettre une partie des cellules hôtes à une condition d'essai, lesdites cellules hôtes produisant l'échantillon pour essai, et/ou avant l'étape (3) une étape (2a') consistant à soumettre une partie des cellules hôtes à une condition de référence, lesdites cellules hôtes produisant un échantillon de référence.

12. Procédé selon la revendication 10 ou 11, comprenant en outre une étape (3') de quantification de chaque oligonucléotide d'ARN dans ledit échantillon de référence afin de déterminer une réponse de capteur pour chaque polynucléotide.

13. Procédé selon la revendication 12, comprenant en outre une étape (4) consistant à calculer un « taux de réponse de capteur » pour un polynucléotide donné quelconque en divisant la quantité de chaque oligonucléotide d'ARN dans l'échantillon pour essai par la quantité de l'oligonucléotide d'ARN correspondant dans l'échantillon de référence, dans lequel un « taux de réponse de capteur » qui s'écarte du « taux de réponse de capteur » médian de tous les polynucléotides indique un effet du gène cible ciblé par le produit d'expression effecteur codé par ledit polynucléotide.

14. Procédé selon l'une quelconque des revendications 10 à 13, dans lequel lesdites conditions d'essai et/ou lesdites conditions de référence sont choisies parmi un stimulus extracellulaire ou intracellulaire ; éventuellement choisies parmi un stimulus chimique ou physique ou une combinaison de ceux-ci ; une modification intrinsèque éventuellement choisie parmi une modification du génome ou du protéome ou de l'épigénome ; ou une combinaison de celles-ci.

15. Procédé de détermination de l'effet d'un gène cible donné sur au moins un ou plusieurs élément(s) de régulation de transcription de capteur, comprenant les étapes suivantes :
(1) l'introduction dans une pluralité de cellules hôtes d'une banque selon la revendication 9 ;
(1') l'introduction dans une pluralité de cellules hôtes d'une banque correspondante selon la revendication 11 ; dans laquelle ledit produit d'expression effecteur fourni par les polynucléotides de ladite banque n'est pas apte à interférer avec un gène cible ;
(2) la fourniture d'un échantillon d'essai à partir desdites cellules hôtes de l'étape (1) ;
(2') la fourniture d'un échantillon de référence à partir desdites cellules hôtes de l'étape (1') ;
ledit échantillon pour essai et ledit échantillon de référence comprenant des oligonucléotides d'ARN transcrits à partir desdits polynucléotides de chaque banque, dans lequel chaque oligonucléotide d'ARN est transcrit à partir d'une séquence d'identifiant unique qui identifie spécifiquement son polynucléotide ;
(3) la quantification de chaque oligonucléotide d'ARN dans ledit échantillon pour essai afin de déterminer une « réponse de capteur » de l'échantillon pour essai pour chaque polynucléotide de la banque ;
(3') la quantification de chaque oligonucléotide d'ARN dans ledit échantillon de référence afin de déterminer une « réponse de capteur » de l'échantillon de référence pour chaque polynucléotide de la banque ;
dans lequel une « réponse de capteur » dans un échantillon pour essai pour un polynucléotide donné quelconque qui s'écarte de la « réponse de capteur » correspondante dans l'échantillon de référence indique un effet du gène cible sur l'élément de régulation de transcription de capteur constitué par ledit polynucléotide.

16. Kit comprenant un polynucléotide selon l'une quelconque des revendications 1 à 6, un vecteur selon la revendication 7, ou une cellule hôte selon la revendication 8 et des moyens pour détecter et/ou quantifier l'expression d'oligonucléotides d'ARN.
